# EUROPEAN PATENT SPECIFICATION

(11) **EP 4 195 928 B1**
(45) Date of publication and mention of the grant of the patent: **18.06.2025**
(21) Application number: 21758073.7
(22) Date of filing: 02.08.2021
(51) Int. Cl.: A01N 43/40, A01N 43/42, A01N 43/50, A01N 43/54, A01N 43/56, A01N 43/58, A01N 43/60, A01N 43/78, A01N 43/80, A01P 3/00

(54) **USE OF STROBILURIN TYPE COMPOUNDS FOR COMBATING PHYTOPATHOGENIC FUNGI CONTAINING AN AMINO ACID SUBSTITUTION F129L IN THE MITOCHONDRIAL CYTOCHROME B PROTEIN CONFERRING RESISTANCE TO QO INHIBITORS VIII**
VERWENDUNG VON VERBINDUNGEN VOM STROBILURINTYP ZUR BEKÄMPFUNG VON PHYTOPATHOGENEN PILZEN, DIE EINE AMINOSÄURESUBSTITUTION F129L IN DEM MITOCHONDRIALEN CYTOCHROM-B-PROTEIN ENTHALTEN, DAS RESISTENZ GEGENÜBER QO-INHIBITOREN VIII VERLEIHT
UTILISATION DE COMPOSÉS DE TYPE STROBILURINE POUR LUTTER CONTRE LES CHAMPIGNONS PHYTOPATHOGÈNES CONTENANT UNE SUBSTITUTION D'ACIDE AMINÉ F129L DANS LA PROTÉINE MITOCHONDRIALE DU CYTOCHROME B CONFÉRANT UNE RÉSISTANCE AUX INHIBITEURS QO VIII

(30) Priority: 11.08.2020 IN 202021034471; 21.09.2020 EP 20197096; 25.06.2021 EP 21181804
(43) Date of publication of application: 21.06.2023
(73) Proprietor: BASF SE, 67056 Ludwigshafen am Rhein (DE)
(72) Inventor: KOCH, Andreas, 67117 Limburgerhof (DE); FEHR, Marcus, 67117 Limburgerhof (DE); TEGGE, Vanessa, 67117 Limburgerhof (DE); DEY, Chandan, Mumbai 400705 (IN); POONOTH, Manojkumar, 67056 Ludwigshafen (DE); KULKARNI, Sarang, Pune, 412210 (IN); LE VEZOUET, Ronan, 67056 Ludwigshafen (DE); WINTER, Christian Harald, 67056 Ludwigshafen (DE); RUDOLF, Georg Christoph, 67056 Ludwigshafen (DE); RATH, Rakesh, Mumbai 400705 (IN); KHANNA, Smriti, Mumbai 400705 (IN); CRAIG, Ian Robert, 67056 Ludwigshafen (DE); GRAMMENOS, Wassilios, 67056 Ludwigshafen (DE); GROTE, Thomas, 67157 Wachenheim (DE); STAMMLER, Gerd, 67117 Limburgerhof (DE); MENTZEL, Tobias, 67117 Limburgerhof (DE); HADEN, Egon, 67346 Speyer (DE); RHEINHEIMER, Joachim, 67063 Ludwigshafen (DE)
(74) Representative: BASF IP Association
(86) International application number: PCT/EP2021/071520
(87) International publication number: WO 2022/033906

(56) References cited:
- WO-A1-2013/092224
- WO-A1-2019/219464
- CN-A- 106 946 770

## Description

The present invention relates the use of strobilurin type compounds of formula I and the N-oxides and the salts thereof for combating phytopathogenic fungi containing an amino acid substitution F129L in the mitochondrial cytochrome b protein (also referred to as F129L mutation in the mitochondrial cytochrome b gene) conferring resistance to Qo inhibitors (Qol), and to methods for combating such fungi. The invention also relates to novel compounds, processes for preparing these compounds, to compositions comprising at least one such compound, to plant health applications, and to seeds coated with at least one such compound. The present invention also relates to a method for controlling soybean rust fungi *(Phakopsora pachyrhizi*) with the amino acid substitution F129L in the mitochondrial cytochrome b protein.

"Qo inhibitor," as used herein, includes any substance that is capable of diminishing and/or inhibiting respiration by binding to a ubihydroquinone oxidation center of a cytochrome bc, complex in mitochondria. The oxidation center is typically located on the outer side of the inner mitochondrial membrane. Many of these compounds are also known as strobilurin-type or strobilurin analogue compounds.

The mutation F129L in the mitochondrial cytochrome b (CYTB) gene shall mean any substitution of nucleotides of codon 129 encoding "F" (phenylalanine; e.g. TTT or TTC) that leads to a codon encoding "L" (leucine; e.g. TTA, TTG, TTG, CTT, CTC, CTA or CTG), for example the substitution of the first nucleotide of codon 129 'T' to 'C' (TTT to CTT), in the CYTB (cytochrome b) gene resulting in a single amino acid substitution in the position 129 from F to L in the cytochrome b protein. Such F129L mutation is known to confer resistance to Qo inhibitors.

Qol fungicides, often referred to as strobilurin-type fungicides, are conventionally used to control a number of fungal pathogens in crops. Qo inhibitors typically work by inhibiting respiration by binding to a ubihydroquinone oxidation center of a cytochrome bc, complex (electron transport complex III) in mitochondria. Said oxidation center is located on the outer side of the inner mitochondrial membrane. A prime example of the use of Qols includes the use of, for example, strobilurins on wheat for the control of *Septoria tritici* (also known as *Mycosphaerella graminicola),* which is the cause of wheat leaf blotch. Unfortunately, widespread use of such Qols has resulted in the selection of mutant pathogens which are resistant to such Qols. Resistance to Qols has been detected in several phytopathogenic fungi such as *Blumeria graminis, Mycosphaerella fijiensis, Pseudoperonspora cubensis* or *Venturia inaequalis.* The major part of resistance to Qols in agricultural uses has been attributed to pathogens containing a single amino acid residue substitution G143A in the cytochrome b gene for their cytochrome bc, complex, the target protein of Qols which have been found to be controlled by specific Qols (WO 2013/092224). Despite several commercial Qol fungicides have also been widely used in soybean rust control, the single amino acid residue substitution G143A in the cytochrome b protein conferring resistance to Qol fungicides was not observed.

Instead soybean rust acquired a different genetic mutation in the cytochrome b gene causing a single amino acid substitution F129L which also confers resistance against Qol fungicides. The efficacy of Qol fungicides used against soybean rust conventionally, i.e. pyraclostrobin, azoxystrobin, picoxystrobin, orysastrobin, dimoxystrobin and metominostrobin, has decreased to a level with practical problems for agricultural practice.

Although it seems that trifloxystrobin was less affected by the F129L mutation to the same degree as other Qol fungicides such as azoxystrobin and pyraclostrobin, trifloxystrobin was never as efficacious on a fungal population bearing the F129L Qol resistance mutation as on a sensitive population (Crop Protection 27, (2008) 427-435).

Thus, new methods are desirable for controlling pathogen induced diseases in crops comprising plants subjected to pathogens containing a F129L amino acid substitution in the mitochondrial cytochrome b protein conferring resistance to Qo inhibitors. Furthermore, in many cases, in particular at low application rates, the fungicidal activity of the known fungicidal strobilurin compounds is unsatisfactory, especially in case that a high proportion of the fungal pathogens contain a mutation in the mitochondrial cytochrome b gene conferring resistance to Qo inhibitors. Besides there is an ongoing need for new fungicidally active compounds which are more effective, less toxic and/or environmentally safer. Based on this, it was also an object of the present invention to provide compounds having improved activity and/or a broader activity spectrum against phytopathogenic fungi and/or even further reduced toxicity against non target organisms such as vertebrates and invertebrates.

The strobilurin-analogue compounds used to combat phytopathogenic fungi containing a F129L amino acid substitution in the mitochondrial cytochrome b protein conferring resistance to Qo inhibitors according to the present invention differ from trifloxystrobin inter alia containing a specific group attached to the central phenyl ring in ortho position to the methyl oxime side chain defined herein as R³ as well as by the heteroaryl attached to the methyl oxime side chain.

The methods and uses according to the present invention do not extend to medical uses nor to methods of therapy.

Accordingly, the present invention relates to the use of compounds of formula I 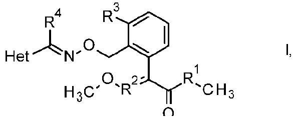 wherein
- R¹: is selected from O and NH;
- R²: is selected from CH and N;
- R³: is selected from halogen, CN, C₁-C₄-alkyl, C₂-C₄-alkenyl, C₂-C₄-alkynyl, C₁-C₄-haloalkyl, C₂-C₄-haloalkenyl, C₂-C₄-haloalkynyl, C₃-C₆-cycloalkyl, -O-C₁-C₄-alkyl, -O-C₁-C₄-haloalkyl, -O-C₃-C₆-cycloalkyl, -C₁-C₂-alkyl-C₃-C₆-cycloalkyl, phenyl, 3- to 6-membered heterocycloalkyl and 5- or 6-membered heteroaryl,
wherein said heterocycloalkyl and heteroaryl besides carbon atoms contain 1, 2 or 3 heteroatoms selected from N, O and S, provided that such heterocycloalkyl and heteroaryl cannot contain 2 contiguous atoms selected from O and S;
wherein said phenyl, heterocycloalkyl and heteroaryl are bound directly or via an oxygen atom or via a C₁-C₂-alkylene linker, and wherein said phenyl and heteroaryl are unsubstituted or substituted by 1, 2 or 3 identical or different substituents selected from halogen, CN, NH₂, NO₂, C₁-C₄-alkyl, C₁-C₄-haloalkyl, -O-C₁-C₄-alkyl and -O-C₁-C₄-haloalkyl;
- R⁴: is selected from C₁-C₆-alkyl, C₂-C₄-alkenyl, C₂-C₄-alkynyl, C₁-C₆-haloalkyl, C₂-C₄-haloalkenyl, C₂-C₄-haloalkynyl, O-C₁-C₄-alkyl, -C(=O)-C₁-C₄-alkyl, -(C₁-C₂-alkyl)-O-(C₁-C₂-alkyl), -(C₁-C₂-alkyl)-O-(C₁-C₂-haloalkyl), C₃-C₆-cycloalkyl, C₃-C₆-halocycloalkyl and -C₁-C₄-alkyl-C₃-C₆-cycloalkyl;
- Het: is 5- or 6-membered heteroaryl, wherein said heteroaryl besides carbon atoms contains 1, 2 or 3 heteroatoms selected from N, O and S, provided that such heteroaryl cannot contain 2 contiguous atoms selected from O and S;
wherein said heteroaryl is unsubstituted or carries 1, 2, 3 or up to the maximum number of identical or different groups R^{a}:
R^{a} is selected from halogen, CN, -NR⁵R⁶, C₁-C₄-alkyl, C₂-C₄-alkenyl, C₂-C₄-alkynyl, -O-C₁-C₄-alkyl, -C(=N-O-C₁-C₄-alkyl)-C₁-C₄-alkyl, -C(=O)-C₁-C₄-alkyl, -O-CH₂-C(=N-O-C₁-C₄-alkyl)-C₁-C₄-alkyl, C₃-C₆-cycloalkyl, C₃-C₆-cycloalkenyl, -C₁-C₂-alkyl-C₃-C₆-cycloalkyl, -O-C₃-C₆-cycloalkyl, phenyl, 3- to 6-membered heterocycloalkyl, 3- to 6-membered heterocycloalkenyl and 5- or 6-membered heteroaryl,
wherein said heterocycloalkyl, heterocycloalkenyl and heteroaryl besides carbon atoms contain 1, 2 or 3 heteroatoms selected from N, O and S, provided that such heterocycloalkyl, heterocycloalkenyl and heteroaryl cannot contain 2 contiguous atoms selected from O and S;
   and/or
2 R^{a} substituents bound to neighboring carbon ring atoms, together with the two interjacent carbon ring atoms, form a partially unsaturated or aromatic 5- to 6-membered fused carbo- or heterocycle,
wherein the heterocycle includes beside carbon atoms 1 or 2 heteroatoms independently selected from N, O and S as ring member atoms, provided that such heterocycle cannot contain 2 contiguous atoms selected from O and S;
and wherein the aliphatic and cyclic moieties of R^{a} and the abovementioned fused carbo- or heterocycle are unsubstituted or carry 1, 2, 3, 4 or up to the maximum number of identical or different groups R^{b}:
   R^{b} is selected from halogen, CN, NH₂, NO₂, C₁-C₄-alkyl, C₁-C₄-haloalkyl, -O-C₁-C₄-alkyl, -O-C₁-C₄-haloalkyl and C₃-C₆-cycloalkyl;
   R⁵, R⁶ are independently of each other selected from the group consisting of H, C₁-C₆-alkyl, C₁-C₆-haloalkyl and C₂-C₄-alkynyl;
and in form of stereoisomers and tautomers thereof, and the N-oxides and the agriculturally acceptable salts thereof, for combating phytopathogenic fungi containing an amino acid substitution F129L in the mitochondrial cytochrome b protein conferring resistance to Qo inhibitors.

The mutation F129L in the cytochrome b (cytb, also referred to as cob) gene shall mean any substitution of nucleotides of codon 129 encoding "F" (phenylalanine; e.g. TTT or TTC) that leads to a codon encoding "L" (leucine; e.g. TTA, TTG, TTG, CTT, CTC, CTA or CTG), for example the substitution of the first nucleotide of codon 129 'T' to 'C' (TTT to CTT), in the cytochrome b gene resulting in a single amino acid substitution in the position 129 from F (phenylalanine) to L (leucine) (F129L) in the cytochrome b protein (Cytb). In the present invention, the mutation F129L in the cytochrome b gene shall be understood to be a single amino acid substitution in the position 129 from F (phenylalanine) to L (leucine) (F129L) in the cytochrome b protein.

Many other phytopathogenic fungi acquired the F129L mutation in the cytochrome b gene conferring resistance to Qo inhibitors, such as rusts, in particular soybean rust *(Phakopsora pachyrhizi* and *Phakopsora meibromiae)* as well as fungi from the genera Alternaria, Pyrenophora and Rhizoctonia.

Preferred fungal species are *Alternaria solani, Phakopsora pachyrhizi, Phakopsora meibromiae, Pyrenophora teres, Pyrenophora tritici-repentis* and *Rhizoctonia solani;* in particular *Phakopsora pachyrhizi.*

In one aspect, the present invention relates to the method of protecting plants susceptible to and/or under attack by phytopathogenic fungi containing an amino acid substitution F129L in the mitochondrial cytochrome b protein conferring resistance to Qo inhibitors, which method comprises applying to said plants, treating plant propagation material of said plants with, and/or applying to said phytopathogenic fungi, at least one compound of formula I or a composition comprising at least one compound of formula I.

According to another embodiment, the method for combating phytopathogenic fungi, comprises: a) identifying the phytopathogenic fungi containing an amino acid substitution F129L in the mitochondrial cytochrome b protein conferring resistance to Qo inhibitors, or the materials, plants, the soil or seeds that are at risk of being diseased from phytopathogenic fungi as defined herein, and b) treating said fungi or the materials, plants, the soil or plant propagation material with an effective amount of at least one compound of formula I, or a composition comprising it thereof.

The term "phytopathogenic fungi an amino acid substitution F129L in the mitochondrial cytochrome b protein conferring resistance to Qo inhibitors" is to be understood that at least 10% of the fungal isolates to be controlled contain a such F129L substitution in the mitochondrial cytochrome b protein conferring resistance to Qo inhibitors, preferably at least 30%, more preferably at least 50%, even more preferably at at least 75% of the fungi, most preferably between 90 and 100%; in particular between 95 and 100%.

Although the present invention will be described with respect to particular embodiments, this description is not to be construed in a limiting sense.

Before describing in detail exemplary embodiments of the present invention, definitions important for understanding the present invention are given. As used in this specification and in the appended claims, the singular forms of "a" and "an" also include the respective plurals unless the context clearly dictates otherwise. In the context of the present invention, the terms "about" and "approximately" denote an interval of accuracy that a person skilled in the art will understand to still ensure the technical effect of the feature in question. The term typically indicates a deviation from the indicated numerical value of ±20 %, preferably ±15 %, more preferably ±10 %, and even more preferably ±5 %. It is to be understood that the term "comprising" is not limiting. For the purposes of the present invention the term "consisting of" is considered to be a preferred embodiment of the term "comprising of".

Unless otherwise indicated, the following definitions are set forth to illustrate and define the meaning and scope of the various terms used to describe the invention herein and the appended claims. These definitions should not be interpreted in the literal sense as they are not intended to be general definitions and are relevant only for this application.

The term "compounds I" refers to compounds of formula I. Likewise, this terminology applies to all sub-formulae, e. g. "compounds I.2" refers to compounds of formula I.2 or "compounds V" refers to compounds of formula V, etc..

The term "independently" when used in the context of selection of substituents for a variable, it means that where more than one substituent is selected from a number of possible substituents, those substituents may be the same or different.

The organic moieties or groups mentioned in the above definitions of the variables are collective terms for individual listings of the individual group members. The term "Cᵥ-C_{w}" indicates the number of carbon atom possible in each case.

The term "halogen" refers to fluorine, chlorine, bromine and iodine.

The term "C₁-C₄-alkyl" refers to a straight-chained or branched saturated hydrocarbon group having 1 to 4 carbon atoms, for example, methyl (CH₃), ethyl (C₂H₅), propyl, 1-methylethyl (isopropyl), butyl, 1-methylpropyl, 2-methylpropyl, 1,1-dimethylethyl.

The term "C₂-C₄-alkenyl" refers to a straight-chain or branched unsaturated hydrocarbon radical having 2 to 4 carbon atoms and a double bond in any position such as ethenyl, 1-propenyl, 2-propenyl, 1-methylethenyl, 1-butenyl, 2-butenyl, 3-butenyl, 1-methyl-1-propenyl, 2-methyl-1-propenyl, 1-methyl-2-propenyl, 2-methyl-2-propenyl.

The term "C₂-C₄-alkynyl" refers to a straight-chain or branched unsaturated hydrocarbon radical having 2 to 4 carbon atoms and containing at least one triple bond such as ethynyl, prop-1-ynyl, prop-2-ynyl, but-1-ynyl, but-2-ynyl, but-3-ynyl, 1-methyl-prop-2-ynyl.

The term "C₁-C₄-haloalkyl" refers to a straight-chained or branched alkyl group having 1 to 4 carbon atoms wherein some or all of the hydrogen atoms in these groups may be replaced by halogen atoms as mentioned above, for example chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl and pentafluoroethyl, 2-fluoropropyl, 3-fluoropropyl, 2,2-difluoropropyl, 2,3-difluoropropyl, 2-chloropropyl, 3-chloropropyl, 2,3-dichloropropyl, 2-bromopropyl, 3-bromopropyl, 3,3,3-trifluoropropyl, 3,3,3-trichloropropyl, CH₂-C₂F₅, CF₂-C₂F₅, CF(CF₃)₂, 1-(fluoromethyl)-2-fluoroethyl, 1-(chloromethyl)-2-chloroethyl, 1-(bromomethyl)-2-bromoethyl, 4-fluorobutyl, 4-chlorobutyl, 4-bromobutyl or nonafluorobutyl.

The term "-O-C₁-C₄-alkyl" refers to a straight-chain or branched alkyl group having 1 to 4 carbon atoms which is bonded via an oxygen, at any position in the alkyl group, e.g. OCH₃, OCH₂CH₃, O(CH₂)₂CH₃, 1-methylethoxy, O(CH₂)₃CH₃, 1-methyl-propoxy, 2-methylpropoxy or 1,1-dimethylethoxy.

The term "C₃-C₆-cycloalkyl" refers to monocyclic saturated hydrocarbon radicals having 3 to 6 carbon ring members, such as cyclopropyl (C₃H₅), cyclobutyl, cyclopentyl or cyclohexyl. The term "C₃-C₆-cycloalkenyl " refers to monocyclic saturated hydrocarbon radicals having 3 to 6 carbon ring members and one or more double bonds.

The term "3- to 6-membered heterocycloalkyl" refers to 3- to 6-membered monocyclic saturated ring system having besides carbon atoms one or more heteroatoms, such as O, N, S as ring members. The term "C₃-C₆-membered heterocycloalkenyl" refers to 3- to 6-membered monocyclic ring system having besides carbon atoms one or more heteroatoms, such as O, N and S as ring members, and one or more double bonds.

The term "-C₁-C₄-alkyl-C₃-C₆-cycloalkyl" refers to alkyl having 1 to 4 carbon atoms (as defined above), wherein one hydrogen atom of the alkyl radical is replaced by a cycloalkyl radical having 3 to 6 carbon atoms.

The term "phenyl" refers to C₆H₅.

The term "5- or 6-membered heteroaryl" which contains 1, 2, 3 or 4 heteroatoms from the group consisting of O, N and S, is to be understood as meaning aromatic heterocycles having 5 or 6 ring atoms. Examples include:
- 5-membered heteroaryl which in addition to carbon atoms, e.g. contain 1, 2 or 3 N atoms and/or one sulfur and/or one oxygen atom: for example 2-thienyl, 3-thienyl, 3-pyrazolyl, 4-pyrazolyl, 5-pyrazolyl, 2-oxazolyl, 4-oxazolyl, 5-oxazolyl, 2-thiazolyl, 4-thiazolyl, 5-thiazolyl, 2-imidazolyl, 4-imidazolyl and 1,3,4-triazol-2-yl;
- 6-membered heteroaryl which, in addition to carbon atoms, e.g. contain 1, 2, 3 or 4 N atoms as ring members, e.g. 2-pyridinyl, 3-pyridinyl, 4-pyridinyl, 3-pyridazinyl, 4-pyridazinyl, 2-pyrimidinyl, 4-pyrimidinyl, 5-pyrimidinyl and 2-pyrazinyl.

The term "C₁-C₂-alkylene linker" means a divalent alkyl group such as -CH₂- or -CH₂-CH₂-that is bound at one end to the core structure of formula I and at the other end to the particular substituent.

As used herein, the "compounds", in particular "compounds I" include all the stereoisomeric and tautomeric forms and mixtures thereof in all ratios, prodrugs, isotopic forms, their agriculturally acceptable salts, N-oxides and S-oxides thereof.

The term "stereoisomer" is a general term used for all isomers of individual compounds that differ only in the orientation of their atoms in space. The term stereoisomer includes mirror image isomers (enantiomers), mixtures of mirror image isomers (racemates, racemic mixtures), geometric (cis/trans or E/Z) isomers, and isomers of compounds with more than one chiral center that are not mirror images of one another (diastereoisomers). The term "tautomer" refers to the coexistence of two (or more) compounds that differ from each other only in the position of one (or more) mobile atoms and in electron distribution, for example, keto-enol tautomers. The term "agriculturally acceptable salts" as used herein, includes salts of the active compounds which are prepared with acids or bases, depending on the particular substituents found on the compounds described herein. "N-oxide" refers to the oxide of the nitrogen atom of a nitrogen-containing heteroaryl or heterocycle. N-oxide can be formed in the presence of an oxidizing agent for example peroxide such as m-chloro-perbenzoic acid or hydrogen peroxide. N-oxide refers to an amine oxide, also known as amine-N-oxide, and is a chemical compound that contains N→O bond.

In respect of the variables, the embodiments of the intermediates correspond to the embodiments of the compounds I.

Preference is given to those compounds I and where applicable also to compounds of all sub-formulae provided herein, e. g. formulae I.1 and I.2, and to the intermediates such as compounds II, III, IV and V, wherein the substituents and variables (such as n, R¹, R², R³, R⁴, R⁵, R⁶, R^{a}, and R^{b}) have independently of each other or more preferably in combination (any possible combination of 2 or more substituents as defined herein) the following meanings:
Preference is also given to the uses, methods, mixtures and compositions, wherein the definitions (such as phytopathogenic fungi, treatments, crops, compounds II, further active ingredients, solvents, solid carriers) have independently of each other or more preferably in combination the following meanings and even more preferably in combination (any possible combination of 2 or more definitions as provided herein) with the preferred meanings of compounds I herein:
One embodiment of the invention relates to the abovementioned use and or method of application (herein collectively referred to as "use") of compounds I, wherein R¹ is selected from O and NH; and R² is selected from CH and N, provided that R² is N in case R¹ is NH. More preferably R¹ is NH. In particular, R¹ is NH and R² is N.

According to another embodiment, R³ is selected from CN, C₁-C₄-alkyl, C₂-C₄-alkenyl, C₁-C₄-haloalkyl, C₂-C₄-haloalkenyl, C₃-C₆-cycloalkyl, -O-C₁-C₄-alkyl, -O-C₁-C₄-haloalkyl, -C₁-C₂-alkyl-C₃-C₆-cycloalkyl and 3- to 6-membered heterocycloalkyl; more preferably from is selected from CN, C₁-C₄-alkyl, C₂-C₄-alkenyl, C₂-C₄-alkynyl, C₁-C₄-haloalkyl, C₃-C₄-cycloalkyl, -O-C₁-C₄-alkyl, -O-C₁-C₄-haloalkyl and 3- to 4-membered heterocycloalkyl, wherein said heterocycloalkyl besides carbon atoms contain 1 or 2 heteroatoms selected from N, O and S, and wherein said heterocycloalkyl is bound directly or via an oxygen atom or via a C₁-C₂-alkylene linker; even more preferably from C₁-C₂-alkyl, C₂-alkenyl, C₁-C₂-haloalkyl, -O-C₁-C₂-alkyl, -O-C₁-C₂-haloalkyl, C₃-C₄-cycloalkyl, -C₁-C₂-alkyl-C₃-C₄-cycloalkyl, and 3- to 4-membered heterocycloalkyl; further more preferably form C₁-C₂-alkyl, C₁-C₂-haloalkyl, C₃-C₄-cycloalkyl, -O-C₁-C₂-alkyl and -O-C₁-C₂-haloalkyl; particularly preferred from methyl and C₁-C₂-haloalkyl, in particular methyl.

According to one embodiment, R⁴ is selected from is selected from C₁-C₆-alkyl, C₂-C₄-alkenyl, -C(=O)-C₁-C₂-alkyl, C₁-C₆-haloalkyl, C₂-C₄-haloalkenyl, -(C₁-C₂-alkyl)-O-(C₁-C₂-alkyl) and -CH₂-cyclopropyl; more preferably from C₁-C₄-alkyl, C₂-C₄-alkenyl, -C(=O)-C₁-C₂-alkyl, C₁-C₄-haloalkyl, C₂-C₄-haloalkenyl, -(C₁-C₂-alkyl)-O-(C₁-C₂-alkyl), cyclopropyl and -CH₂-cyclopropyl; even more preferably from C₁-C₄-alkyl, C₁-C₄-haloalkyl and cyclopropyl, particularly preferably from methyl and C₁-haloalkyl; in particular methyl.

According to a further embodiment, Het is 5-membered heteroaryl, wherein said heteroaryl besides carbon atoms contains 1, 2 or 3 heteroatoms selected from N, O and S, provided that such heteroaryl cannot contain 2 contiguous atoms selected from O and S; preferably said 5-membered heteroaryl besides carbon atoms contains 1 nitrogen atom and 0 or 1 further heteroatom selected from N, O and S; more preferably said 5-membered heteroaryl is selected from 2-thienyl, 3-thienyl, 3-pyrazolyl, 4-pyrazolyl, 5-pyrazolyl, 2-oxazolyl, 4-oxazolyl, 5-oxazolyl, 3-isoxazolyl, 2-thiazolyl, 4-thiazolyl, 5-thiazolyl, 3-isothiiazolyl, 2-imidazolyl, 4-imidazolyl, 5-imidazolyl and 1,3,4-triazol-2-yl; even more selected from 4-pyrazolyl, 5-pyrazolyl, 3-isoxazolyl, 2-thiazolyl, 4-thiazolyl, 3-isothiazolyl and 5-imidazolyl, in particular 3-isothazolyl, 2-thiazolyl or 4-thazolyl.

According to a further embodiment, Het is 6-membered heteroaryl, wherein said heteroaryl besides carbon atoms contains 1, 2 or 3 heteroatoms selected from N, O and S, provided that such heteroaryl cannot contain 2 contiguous atoms selected from O and S; preferably said 6-membered heteroaryl besides carbon atoms contains 1 or 2 nitrogen atoms; more preferably said heteroaryl is selected from 2-pyridinyl, 3-pyridinyl, 4-pyridinyl, 3-pyridazinyl, 4-pyridazinyl, 2-pyrimidinyl, 4-pyrimidinyl, 5-pyrimidinyl and 2-pyrazinyl; even more preferably selected from 2-pyridinyl, 3-pyridinyl, 4-pyridinyl, 2-pyrimidinyl, 4-pyrimidinyl, 3-pyridazinyl, 2-pyrazinyl; in particular 2-pyridinyl.

According to a further embodiment, Het is pyridyl or thiazolyl.

According to a further embodiment, Het caries 1, 2, 3, 4 or 5 R^{a} substituents; more preferably 1, 2 or 3 R^{a} substituents, even more preferably 1 or 2 R^{a} substituents; in particular 1 R^{a} substituent.

According to a further embodiment, Het is unsubstituted or carries 1, 2 or 3 R^{a} substituents, more preferably Het is unsubstituted or carries 1 or 2 R² substituents, in particular Het is unsubstituted.

According to a further embodiment, R^{a} is selected from CN, C₁-C₄-alkyl, C₂-C₄-alkenyl, C₂-C₄-alkynyl, -O-C₁-C₄-alkyl, -C(=O)-C₁-C₄-alkyl,-C(=N-O-C₁-C₄-alkyl)-C₁-C₄-alkyl, -O-CH₂-(=N-O-C₁-C₄-alkyl)-C₁-C₄-alkyl, -C(=N-O-C₁-C₄-alkyl)-C(=O-NH-C₁-C₄-alkyl), C₃-C₆-cycloalkyl, C₃-C₆-cycloalkenyl, -C₁-C₂-alkyl-C₃-C₆-cycloalkyl, -O-C₃-C₆-cycloalkyl, phenyl, 3- to 5-membered heterocycloalkyl, 3- to 5-membered heterocycloalkenyl and 5- or 6-membered heteroaryl, wherein said heterocycloalkyl, hetercycloalkenyl and heteroaryl besides carbon atoms contain 1, 2 or 3 heteroatoms selected from N, O and S, and wherein the aliphatic and cyclic moieties of R^{a} are unsubstituted or carry 1, 2, or 3 of identical or different groups R^{b} which independently of one another are selected from halogen, CN, NH₂, NO₂, C₁-C₂-alkyl and C₁-C₂-haloalkyl.

More preferably, R^{a} is selected from CN, C₁-C₄-alkyl, C₂-C₄-alkenyl, C₂-C₄-alkynyl, -O-C₁-C₄-alkyl, -C(=O)-C₁-C₂-alkyl,-C(=N-O-C₁-C₂-alkyl)-C₁-C₂-alkyl, -O-CH₂-C(=N-O-C₁-C₂-alkyl)-C₁-C₂-alkyl, -C(=N-O-C₁-C₂-alkyl)-C(=O-NH-C₁-C₂-alkyl), C₃-C₄-cycloalkyl, C₃-C₄-cycloalkenyl, -C₁-C₂-alkyl-C₃-C₄-cycloalkyl, -O-C₃-C₄-cycloalkyl, phenyl, 3- to 5-membered heterocycloalkyl and 5- or 6-membered heteroaryl, wherein said heterocycloalkyl and heterocycloalkyl and heteroaryl besides carbon atoms contain 1 or 2 heteroatoms selected from N, O and S, and wherein the aliphatic or cyclic moieties of R^{a} are unsubstituted or carry 1, 2, or 3 of identical or different groups R^{b} which independently of one another are selected from halogen, CN, C₁-C₂-alkyl and C₁-C₂-haloalkyl.

Even more preferably R^{a} is selected from C₁-C₃-alkyl, C₂-C₃-alkenyl, C₂-C₃-alkynyl, -O-C₁-C₃-alkyl, -C(=O)-C₁-C₂-alkyl,-C(=N-O-C₁-C₂-alkyl)-C₁-C₂-alkyl, C₃-C₄-cycloalkyl, -C₁-C₂-alkyl-C₃-C₄-cycloalkyl, -O-C₃-C₄-cycloalkyl, phenyl, 3- to 5-membered heterocycloalkyl and 5- or 6-membered heteroaryl, wherein said heterocycloalkyl and heteroaryl besides carbon atoms contain 1 or 2 heteroatoms selected from N, O and S, and wherein the aliphatic and cyclic moieties of R^{a} are unsubstituted or carry 1, 2 or 3 of identical or different groups R^{b} which independently of one another are selected from halogen, CN, methyl and C₁-haloalkyl.

Particularly preferred R^{a} are selected from halogen, C₁-C₄-alkyl, C₂-C₃-alkenyl, C₂-C₃-alkynyl, -O-C₁-C₄-alkyl, -C(=N-O-C₁-C₂-alkyl)-C₁-C₂-alkyl and phenyl, wherein the aliphatic or cyclic moieties of R^{a} are unsubstituted or carry 1, 2 or 3 of identical or different groups R^{b} which independently of one another are selected from halogen, CN, methyl and C₁-haloalkyl.

According to a further embodiment, R⁵, R⁶ are independently of each other preferably selected from the group consisting of H, C₁-C₄-alkyl, C₁-C₄-haloalkyl and C₂-C₄-alkynyl, more preferably from H and C₁-C₄-alkyl.

According to a further preferred embodiment, the present invention relates to the use of compounds of formula I wherein:
- R¹: is selected from O and NH; and
- R²: is selected from CH and N, provided that R² is N in case R¹ is NH;
- R³: is selected from halogen, CN, C₁-C₄-alkyl, C₂-C₄-alkenyl, C₁-C₄-haloalkyl and C₃-C₄-cycloalkyl;
- R⁴: is selected from C₁-C₆-alkyl, C₂-C₄-alkenyl, C₂-C₄-alkynyl, C₁-C₆-haloalkyl, C₂-C₄-haloalkenyl, -C(=O)-C₁-C₄-alkyl, -(C₁-C₂-alkyl)-O-(C₁-C₂-alkyl), -(C₁-C₂-alkyl)-O-(C₁-C₂-haloalkyl), C₃-C₆-cycloalkyl, C₃-C₆-halocycloalkyl and -C₁-C₄-alkyl-C₃-C₆-cycloalkyl;
- Het: is a 5- or 6-membered heteroaryl, wherein said heteroaryl besides carbon atoms contain 1 or 2 heteroatoms selected from N, O and S, provided that such heteroaryl cannot contain 2 contiguous atoms selected from O and S;
wherein said heteroaryl is unsubstituted or carries 1, 2 or 3 identical or different groups R^{a}:
R^{a} is selected from halogen, CN, -NR⁵R⁶, C₁-C₄-alkyl, C₂-C₄-alkenyl, C₂-C₄-alkynyl, -O-C₁-C₄-alkyl, -C(=N-O-C₁-C₄-alkyl)-C₁-C₄-alkyl, -C(=O)-C₁-C₄-alkyl, -O-CH₂-C(=N-O-C₁-C₄-alkyl)-C₁-C₄-alkyl, C₃-C₆-cycloalkyl, C₃-C₆-cycloalkenyl, -C₁-C₂-alkyl-C₃-C₆-cycloalkyl, -O-C₃-C₆-cycloalkyl, phenyl, 3- to 6-membered heterocycloalkyl, 3- to 6-membered heterocycloalkenyl and 5- or 6-membered heteroaryl,
wherein said heterocycloalkyl, heterocycloalkenyl and heteroaryl besides carbon atoms contain 1 or 2 heteroatoms selected from N, O and S,
   and/or
2 R^{a} substituents bound to neighboring carbon ring atoms, together with the two interjacent carbon ring atoms, form a fused phenyl ring,
and wherein the aliphatic and cyclic moieties of R^{a} and the abovementioned fused phenyl ring are unsubstituted or carry 1, 2, 3, 4 or up to the maximum number of identical or different groups R^{b}:
   R^{b} is selected from halogen, CN, NH₂, NO₂, C₁-C₄-alkyl, C₁-C₄-haloalkyl, -O-C₁-C₄-alkyl, -O-C₁-C₄-haloalkyl and C₃-C₆-cycloalkyl;
   R⁵, R⁶ are independently of each other selected from the group consisting of H, C₁-C₆-alkyl and C₂-C₄-alkynyl;
and in form of stereoisomers and tautomers thereof, and the N-oxides and the agriculturally acceptable salts thereof, for combating phytopathogenic fungi containing an amino acid substitution F129L in the mitochondrial cytochrome b protein conferring resistance to Qo inhibitors.

Certain strobilurin type compounds bearing a terminal pyridyl ring have been described in WO 1998/23156. Further strobilurin type compounds have been disclosed in EP 370269 and EP 463488. However, it is not mentioned that these compounds inhibit fungal pathogens containing a F129L substitution in the mitochondrial cytochrome b protein conferring resistance to Qo inhibitors. The compounds according to the present invention differ from those described in the abovementioned publications by the specific substituent R⁴ attached to the oxime linker and/or by containing a specific group attached to the central phenyl ring in ortho position to the methyl oxime side chain defined herein as R³.

Therefore, according to a second aspect, the invention provides novel compounds of formula I 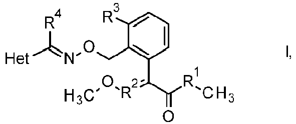 wherein
- R¹: is selected from O and NH;
- R²: is selected from CH and N;
- R³: is selected from halogen, CN, C₁-C₄-alkyl, C₂-C₄-alkenyl, C₂-C₄-alkynyl, C₁-C₄-haloalkyl, C₂-C₄-haloalkenyl, C₂-C₄-haloalkynyl, C₃-C₆-cycloalkyl, -O-C₁-C₄-alkyl, -O-C₁-C₄-haloalkyl, -O-C₃-C₆-cycloalkyl, -C₁-C₂-alkyl-C₃-C₆-cycloalkyl, phenyl, 3- to 6-membered heterocycloalkyl and 5- or 6-membered heteroaryl,
wherein said heterocycloalkyl and heteroaryl besides carbon atoms contain 1, 2 or 3 heteroatoms selected from N, O and S, provided that such heterocycloalkyl and heteroaryl cannot contain 2 contiguous atoms selected from O and S;
wherein said phenyl, heterocycloalkyl and heteroaryl are bound directly or via an oxygen atom or via a C₁-C₂-alkylene linker, and wherein said phenyl and heteroaryl are unsubstituted or substituted by 1, 2 or 3 identical or different substituents selected from halogen, CN, NH₂, NO₂, C₁-C₄-alkyl, C₁-C₄-haloalkyl, -O-C₁-C₄-alkyl and -O-C₁-C₄-haloalkyl;
- R⁴: is selected from C₁-C₆-alkyl, C₂-C₄-alkenyl, C₂-C₄-alkynyl, C₁-C₆-haloalkyl, C₂-C₄-haloalkenyl, C₂-C₄-haloalkynyl, O-C₁-C₄-alkyl, -C(=O)-C₁-C₄-alkyl, -(C₁-C₂-alkyl)-O-(C₁-C₂-alkyl), -(C₁-C₂-alkyl)-O-(C₁-C₂-haloalkyl), C₃-C₆-cycloalkyl, C₃-C₆-halocycloalkyl and -C₁-C₄-alkyl-C₃-C₆-cycloalkyl;
- Het: is 5- or 6-membered heteroaryl, wherein said heteroaryl besides carbon atoms contains 1, 2 or 3 heteroatoms selected from N, O and S, provided that such heteroaryl cannot contain 2 contiguous atoms selected from O and S;
wherein said heteroaryl is unsubstituted or carries 1, 2, 3 or up to the maximum number of identical or different groups R^{a}:
R^{a} is selected from halogen, CN, -NR⁵R⁶, C₁-C₄-alkyl, C₂-C₄-alkenyl, C₂-C₄-alkynyl, -O-C₁-C₄-alkyl, -C(=N-O-C₁-C₄-alkyl)-C₁-C₄-alkyl, -C(=O)-C₁-C₄-alkyl, -O-CH₂-C(=N-O-C₁-C₄-alkyl)-C₁-C₄-alkyl, C₃-C₆-cycloalkyl, C₃-C₆-cycloalkenyl, -C₁-C₂-alkyl-C₃-C₆-cycloalkyl, -O-C₃-C₆-cycloalkyl, phenyl, 3- to 6-membered heterocycloalkyl, 3- to 6-membered heterocycloalkenyl and 5- or 6-membered heteroaryl,
wherein said heterocycloalkyl, heterocycloalkenyl and heteroaryl besides carbon atoms contain 1, 2 or 3 heteroatoms selected from N, O and S, provided that such heterocycloalkyl, heterocycloalkenyl and heteroaryl cannot contain 2 contiguous atoms selected from O and S;
   and/or
2 R^{a} substituents bound to neighboring carbon ring atoms, together with the two interjacent carbon ring atoms, form a partially unsaturated or aromatic 5- to 6-membered fused carbo- or heterocycle,
wherein the heterocycle includes beside carbon atoms 1 or 2 heteroatoms independently selected from N, O and S as ring member atoms, provided that such heterocycle cannot contain 2 contiguous atoms selected from O and S;
and wherein the aliphatic and cyclic moieties of R^{a} and the abovementioned fused carbo- or heterocycle are unsubstituted or carry 1, 2, 3, 4 or up to the maximum number of identical or different groups R^{b}:
   R^{b} is selected from halogen, CN, NH₂, NO₂, C₁-C₄-alkyl, C₁-C₄-haloalkyl, -O-C₁-C₄-alkyl, -O-C₁-C₄-haloalkyl and C₃-C₆-cycloalkyl;

R⁵, R⁶ are independently of each other selected from the group consisting of H, C₁-C₆-alkyl, C₁-C₆-haloalkyl and C₂-C₄-alkynyl;
and in form or stereoisomers and tautomers thereof, and the N-oxides and the agriculturally acceptable salts thereof.

One embodiment of the invention relates to preferred compounds I, wherein R¹ is selected from O and NH; and R² is selected from CH and N, provided that R² is N in case R¹ is NH. More preferably R¹ is NH. In particular, R¹ is NH and R² is N. Another embodiment of the invention relates to preferred compounds I, wherein R¹ is selected from O and NH; and R² is selected from CH and N, provided that R² is CH in case R¹ is O. More preferably, R² is N and R¹ is NH or R² is CH and R' is O.

According to another embodiment, R³ is selected from CN, C₁-C₄-alkyl, C₂-C₄-alkenyl, C₁-C₄-haloalkyl, C₂-C₄-haloalkenyl, C₃-C₆-cycloalkyl, -O-C₁-C₄-alkyl, -O-C₁-C₄-haloalkyl, -C₁-C₂-alkyl-C₃-C₆-cycloalkyl and 3- to 6-membered heterocycloalkyl; more preferably from is selected from CN, C₁-C₄-alkyl, C₂-C₄-alkenyl, C₂-C₄-alkynyl, C₁-C₄-haloalkyl, C₃-C₄-cycloalkyl, -O-C₁-C₄-alkyl, -O-C₁-C₄-haloalkyl and 3- to 4-membered heterocycloalkyl, wherein said heterocycloalkyl besides carbon atoms contain 1 or 2 heteroatoms selected from N, O and S, and wherein said heterocycloalkyl is bound directly or via an oxygen atom or via a C₁-C₂-alkylene linker; even more preferably from C₁-C₂-alkyl, C₂-alkenyl, C₁-C₂-haloalkyl, -O-C₁-C₂-alkyl, -O-C₁-C₂-haloalkyl, C₃-C₄-cycloalkyl, -C₁-C₂-alkyl-C₃-C₄-cycloalkyl, and 3- to 4-membered heterocycloalkyl; further more preferably form C₁-C₂-alkyl, C₁-C₂-haloalkyl, C₃-C₄-cycloalkyl, -O-C₁-C₂-alkyl and -O-C₁-C₂-haloalkyl; particularly preferred from methyl and C₁-C₂-haloalkyl, in particular methyl.

According to a further embodiment, R⁴ is selected from is selected from C₁-C₄-alkyl, C₂-C₄-alkenyl, -C(=O)-C₁-C₂-alkyl, C₁-C₄-haloalkyl, C₂-C₄-haloalkenyl, -(C₁-C₂-alkyl)-O-(C₁-C₂-alkyl) , cyclopropyl and -CH₂-cyclopropyl; more preferably from C₁-C₄-alkyl, C₁-C₄-haloalkyl and cyclopropyl, even more preferably from methyl and C₁-haloalkyl; in particular methyl.

According to a further embodiment, Het is 5-membered heteroaryl, wherein said heteroaryl besides carbon atoms contains 1, 2 or 3 heteroatoms selected from N, O and S, provided that such heteroaryl cannot contain 2 contiguous atoms selected from O and S; preferably said 5-membered heteroaryl besides carbon atoms contains 1 nitrogen atom and 0 or 1 further heteroatom selected from N, O and S; more preferably said 5-membered heteroaryl is selected from 2-thienyl, 3-thienyl, 3-pyrazolyl, 4-pyrazolyl, 5-pyrazolyl, 2-oxazolyl, 4-oxazolyl, 5-oxazolyl, 3-isoxazolyl, 2-thiazolyl, 4-thiazolyl, 5-thiazolyl, 3-isothiiazolyl, 2-imidazolyl, 4-imidazolyl, 5-imidazolyl and 1,3,4-triazol-2-yl; even more selected from 4-pyrazolyl, 5-pyrazolyl, 3-isoxazolyl, 2-thiazolyl, 4-thiazolyl, 3-isothiazolyl and 5-imidazolyl, in particular 3-isothazolyl, 2-thiazolyl or 4-thazolyl.

According to a further embodiment, Het is 6-membered heteroaryl, wherein said heteroaryl besides carbon atoms contains 1, 2 or 3 heteroatoms selected from N, O and S, provided that such heteroaryl cannot contain 2 contiguous atoms selected from O and S; preferably said 6-membered heteroaryl besides carbon atoms contains 1 or 2 nitrogen atoms; more preferably said heteroaryl is selected from 2-pyridinyl, 3-pyridinyl, 4-pyridinyl, 3-pyridazinyl, 4-pyridazinyl, 2-pyrimidinyl, 4-pyrimidinyl, 5-pyrimidinyl and 2-pyrazinyl; even more preferably selected from 2-pyridinyl, 3-pyridinyl, 4-pyridinyl, 2-pyrimidinyl, 4-pyrimidinyl, 3-pyridazinyl, 2-pyrazinyl; in particular 2-pyridinyl.

According to a further embodiment, Het is pyridyl or thiazolyl.

According to a further embodiment, Het caries 1, 2, 3, 4 or 5 R^{a} substituents; more preferably 1, 2 or 3 R^{a} substituents, even more preferably 1 or 2 R^{a} substituents; in particular 1 R^{a} substituent.

According to a further embodiment, Het is unsubstituted or carries 1, 2 or 3 R^{a} substituents, more preferably Het is unsubstituted or carries 1 or 2 R^{a} substituents, in particular Het is unsubstituted.

According to a further embodiment, R^{a} is selected from halogen, CN, NH-C₁-C₄-alkyl, N(C₁-C₄-alkyl)₂, C₁-C₄-alkyl, C₂-C₄-alkenyl, C₂-C₄-alkynyl, -O-C₁-C₄-alkyl, -C(=N-O-C₁-C₄-alkyl)-C₁-C₄-alkyl, -C(=O)-C₁-C₄-alkyl, -O-CH₂-C(=N-O-C₁-C₄-alkyl)-C₁-C₄-alkyl, C₃-C₆-cycloalkyl, -C₁-C₂-alkyl-C₃-C₄-cycloalkyl, -O-C₃-C₄-cycloalkyl, phenyl, 3- to 5-membered heterocycloalkyl, 3- to 5-membered heterocycloalkenyl and 5- or 6-membered heteroaryl, wherein said heterocycloalkyl, heterocycloalkenyl and heteroaryl besides carbon atoms contain 1 or 2 heteroatoms selected from N, O and S. Preferably, R^{a} is selected from halogen, CN, NH-C₁-C₂-alkyl, N(C₁-C₂-alkyl)₂, C₁-C₄-alkyl, C₂-C₄-alkenyl, C₂-C₄-alkynyl, -O-C₁-C₄-alkyl, -C(=N-O-C₁-C₄-alkyl)-C₁-C₄-alkyl, -C(=O)-C₁-C₂-alkyl, C₃-C₄-cycloalkyl, -O-C₃-C₄-cycloalkyl, phenyl, 3- to 5-membered heterocycloalkyl and 5- or 6-membered heteroaryl, wherein said heterocycloalkyl and heteroaryl besides carbon atoms contain 1 or 2 heteroatoms selected from N, O and S. More preferably, R^{a} is selected from halogen, CN, C₁-C₃-alkyl, -O-C₁-C₃-alkyl, -C(=N-O- CH₃)-CH₃, C₃-C₄-cycloalkyl,
-O-C₃-C₄-cycloalkyl, phenyl, 3- to 5-membered heterocycloalkyl and 5- or 6-membered heteroaryl, wherein said heterocycloalkyl and heteroaryl besides carbon atoms contain 1 or 2 heteroatoms selected from N, O and S. In particular, R^{a} is selected from halogen, CN, C₁-C₂-alkyl, -O-C₁-C₂-alkyl, ethenyl, ethynyl and -C(=N-O-CH₃)-CH₃.

According to the abovementioned embodiments for R^{a}, the abovementioned heterocycloalkyl is more preferably a 4-membered heterocycloalkyl, wherein said heterocycloalkyl besides carbon atoms contains 1 heteroatom selected from N, O and S, preferably N.

According to the abovementioned embodiments for R^{a}, the abovementioned heteroaryl is more preferably a 5-membered heteroaryl, wherein said heteroaryl besides carbon atoms contains 1 or 2 heteroatoms selected from N, O and S, preferably from N and O.

According to the abovementioned embodiments for R^{a}, the aliphatic and cyclic moieties are unsubstituted or carry 1, 2, 3, 4 or up to the maximum number of identical or different groups R^{b} selected from halogen, CN, NH₂, NO₂, C₁-C₄-alkyl, C₁-C₄-haloalkyl, -O-C₁-C₄-alkyl and -O-C₁-C₄-haloalkyl; more preferably only the cyclic moieties of R^{a} are unsubstituted or carry 1, 2, 3, 4 or up to the maximum number of identical or different groups R^{b} selected from halogen, CN, NH₂, NO₂, C₁-C₄-alkyl, C₁-C₄-haloalkyl, -O-C₁-C₄-alkyl and -O-C₁-C₄-haloalkyl; even more preferably only the phenyl moiety of R^{a} is unsubstituted or carries 1, 2, 3, 4 or 5 identical or different groups R^{b} selected from halogen, CN, C₁-C₄-alkyl, C₁-C₄-haloalkyl, -O-C₁-C₄-alkyl and -O-C₁-C₄-haloalkyl; in particular said phenyl moiety of R^{a} is unsubstituted or carry 1, 2 or 3 identical or different groups R^{b} selected from halogen, CN, C₁-C₂-alkyl, C₁-C₂-haloalkyl, -O-C₁-C₂-alkyl and -O-C₁-C₂-haloalkyl.

According to a further preferred embodiment, the present invention relates to compounds of formula I wherein:
- R¹: is selected from O and NH; and
- R²: is selected from CH and N, provided that R² is N in case R¹ is NH;
- R³: is selected from halogen, CN, C₁-C₄-alkyl, C₂-C₄-alkenyl, C₁-C₄-haloalkyl and C₃-C₄-cycloalkyl;
- R⁴: is selected from C₁-C₆-alkyl, C₂-C₄-alkenyl, C₂-C₄-alkynyl, C₁-C₆-haloalkyl, C₂-C₄-haloalkenyl, -C(=O)-C₁-C₄-alkyl, -(C₁-C₂-alkyl)-O-(C₁-C₂-alkyl), -(C₁-C₂-alkyl)-O-(C₁-C₂-haloalkyl), C₃-C₆-cycloalkyl, C₃-C₆-halocycloalkyl and -C₁-C₄-alkyl-C₃-C₆-cycloalkyl;
- Het: is a 5- or 6-membered heteroaryl, wherein said heteroaryl besides carbon atoms contain 1 or 2 heteroatoms selected from N, O and S, provided that such heteroaryl cannot contain 2 contiguous atoms selected from O and S;
wherein said heteroaryl is unsubstituted or carries 1, 2 or 3 identical or different groups R^{a}:
R^{a} is selected from halogen, CN, -NR⁵R⁶, C₁-C₄-alkyl, C₂-C₄-alkenyl, C₂-C₄-alkynyl, -O-C₁-C₄-alkyl, -C(=N-O-C₁-C₄-alkyl)-C₁-C₄-alkyl, -C(=O)-C₁-C₄-alkyl, -O-CH₂-C(=N-O-C₁-C₄-alkyl)-C₁-C₄-alkyl, C₃-C₆-cycloalkyl, C₃-C₆-cycloalkenyl, -C₁-C₂-alkyl-C₃-C₆-cycloalkyl, -O-C₃-C₆-cycloalkyl, phenyl, 3- to 6-membered heterocycloalkyl, 3- to 6-membered heterocycloalkenyl and 5- or 6-membered heteroaryl,
wherein said heterocycloalkyl, heterocycloalkenyl and heteroaryl besides carbon atoms contain 1 or 2 heteroatoms selected from N, O and S,
   and/or
2 R^{a} substituents bound to neighboring carbon ring atoms, together with the two interjacent carbon ring atoms, form a fused phenyl ring,
and wherein the aliphatic and cyclic moieties of R^{a} and the abovementioned fused phenyl ring are unsubstituted or carry 1, 2, 3, 4 or up to the maximum number of identical or different groups R^{b}:
   R^{b} is selected from halogen, CN, NH₂, NO₂, C₁-C₄-alkyl, C₁-C₄-haloalkyl, -O-C₁-C₄-alkyl, -O-C₁-C₄-haloalkyl and C₃-C₆-cycloalkyl;
   R⁵, R⁶ are independently of each other selected from the group consisting of H, C₁-C₆-alkyl and C₂-C₄-alkynyl;
and in form or stereoisomers and tautomers thereof, and the N-oxides and the agriculturally acceptable salts thereof.

According to a further embodiment, R¹ is O and R² is N, which compounds are of formula I.1:

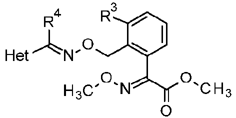

According to a further embodiment, R¹ is O and R² is CH, which compounds are of formula I.2:

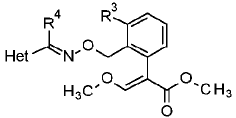

According to a further embodiment, R¹ is NH and R² is N, which compounds are of formula I.3:

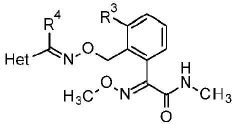

Preferably, R³ of compounds I is one of the following radicals 3-1 to 3-8:

| **No**. | **R³** |
|---|---|
| 3-1 | CH₃ |
| 3-2 | OCH₃ |
| 3-3 | CHF₂ |
| 3-4 | C₃H₅ |
| 3-5 | CH=CH₂ |
| 3-6 | CH₂CH=C(CH₃)₂ |
| 3-7 | CF₃ |
| 3-8 | C(=NOCH₃)CH₃ |

Even more preferably R³ is CH₃, OCH₃, CF₃, CHF₂ or C₃H₅, in particular CH₃.

Particularly preferred embodiments of the invention relate to compounds I, wherein the R⁴ is one of the following radicals 4-1 to 4-10:

| **No**. | **R⁴** |
|---|---|
| 4-1 | CH₃ |
| 4-2 | C₂H₅ |
| 4-3 | CH₂OCH₃ |
| 4-4 | CH₂CF₃ |
| 4-5 | CH₂C₆H₅ |
| 4-6 | CHF₂ |
| 4-7 | CH₂C₃H₅ |
| 4-8 | CH₂-C(=NOCH₃)CH₃ |
| 4-9 | C≡CH |
| 4-10 | C≡CCH₃ |

Particularly preferred embodiments of the invention relate to compounds I, wherein the R^{a} is selected of one of the following radicals a-1 to a-18:

| **No**. | **R^{a}** |
|---|---|
| a-1 | F |
| a-2 | Cl |
| a-3 | Br |
| a-4 | CH₃ |
| a-5 | CHF₂ |
| a-6 | CF₃ |
| a-7 | OCH₃ |
| a-8 | OCHF₂ |
| a-9 | OCF₃ |
| a-10 | C₂H₅ |
| a-11 | CH₂CF₃ |
| a-12 | CH=CH₂ |
| a-13 | C₆H₅ |
| a-14 | C≡CH |
| a-15 | C≡CCH₃ |
| a-16 | C₃H₅ |
| a-17 | C(=NOCH₃)CH₃ |
| a-18 | CN |

According to a further embodiment, R¹ is O, R² is N and R³ is CH₃, which compounds are of formula I.1.1:

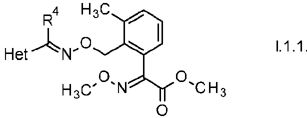

According to a further embodiment, R¹ is O, R² is CH and R³ is CH₃, which compounds are of formula I.2.1:

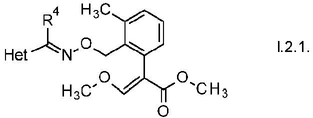

According to a further embodiment, R¹ is NH, R² is N and R³ is CH₃, which compounds are of formula I.3.1:

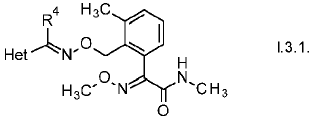

In an embodiment, compounds I are of formula I.3.1 and Het, R^{a} and R⁴ are as per any row of per Table A below, which compounds are named I.3.1-A-1 to I.3.1-A-441.

In another embodiment, compounds I are of formula I.2 and Het, R^{a} and R⁴ are as per any row of Table A below, which compounds are named I.2.1-A-1 to I.2.1-A-441.

In an embodiment, compounds I are of formula I.1.1 and n, R^{a} and R⁴ are as per any row of Table A below, which compounds are named I.1.1-A-1 to I.1.1-A-441.

**Table A:**

| **No.** | **Het** | **R^{a}** | **R⁴** |
|---|---|---|---|
| A-1 | pyridin-2-yl | - | CH₃ |
| A-2 | pyridin-2-yl | 3-F | CH₃ |
| A-3 | pyridin-2-yl | 3-CI | CH₃ |
| A-4 | pyridin-2-yl | 3-Br | CH₃ |
| A-5 | pyridin-2-yl | 3-CH₃ | CH₃ |
| A-6 | pyridin-2-yl | 3-CHF₂ | CH₃ |
| A-7 | pyridin-2-yl | 3-CF₃ | CH₃ |
| A-8 | pyridin-2-yl | 3-OCH₃ | CH₃ |
| A-9 | pyridin-2-yl | 3-OCHF₂ | CH₃ |
| A-10 | pyridin-2-yl | 3-OCF₃ | CH₃ |
| A-11 | pyridin-2-yl | 3-CH₂OCH₃ | CH₃ |
| A-12 | pyridin-2-yl | 3-C₂H₅ | CH₃ |
| A-13 | pyridin-2-yl | 3-CH₂CF₃ | CH₃ |
| A-14 | pyridin-2-yl | 3-CH=CH₂ | CH₃ |
| A-15 | pyridin-2-yl | 3-C≡CH | CH₃ |
| A-16 | pyridin-2-yl | 3-C≡CCH₃ | CH₃ |
| A-17 | pyridin-2-yl | 3-C₃H₅ | CH₃ |
| A-18 | pyridin-2-yl | 3-C(=NOCH₃)CH₃ | CH₃ |
| A-19 | pyridin-2-yl | 3-CN | CH₃ |
| A-20 | pyridin-2-yl | 4-F | CH₃ |
| A-21 | pyridin-2-yl | 4-CI | CH₃ |
| A-22 | pyridin-2-yl | 4-Br | CH₃ |
| A-23 | pyridin-2-yl | 4-CH₃ | CH₃ |
| A-24 | pyridin-2-yl | 4-CHF₂ | CH₃ |
| A-25 | pyridin-2-yl | 4-CF₃ | CH₃ |
| A-26 | pyridin-2-yl | 4-OCH₃ | CH₃ |
| A-27 | pyridin-2-yl | 4-OCHF₂ | CH₃ |
| A-28 | pyridin-2-yl | 4-OCF₃ | CH₃ |
| A-29 | pyridin-2-yl | 4-CH₂OCH₃ | CH₃ |
| A-30 | pyridin-2-yl | 4-C₂H₅ | CH₃ |
| A-31 | pyridin-2-yl | 4-CH₂CF₃ | CH₃ |
| A-32 | pyridin-2-yl | 4-CH=CH₂ | CH₃ |
| A-33 | pyridin-2-yl | 4-C≡CH | CH₃ |
| A-34 | pyridin-2-yl | 4-C≡CCH₃ | CH₃ |
| A-35 | pyridin-2-yl | 4-C₃H₅ | CH₃ |
| A-36 | pyridin-2-yl | 4-C(=NOCH₃)CH₃ | CH₃ |
| A-37 | pyridin-2-yl | 4-CN | CH₃ |
| A-38 | pyridin-2-yl | 5-F | CH₃ |
| A-39 | pyridin-2-yl | 5-CI | CH₃ |
| A-40 | pyridin-2-yl | 5-Br | CH₃ |
| A-41 | pyridin-2-yl | 5-CH₃ | CH₃ |
| A-42 | pyridin-2-yl | 5-CHF₂ | CH₃ |
| A-43 | pyridin-2-yl | 5-CF₃ | CH₃ |
| A-44 | pyridin-2-yl | 5-OCH₃ | CH₃ |
| A-45 | pyridin-2-yl | 5-OCHF₂ | CH₃ |
| A-46 | pyridin-2-yl | 5-OCF₃ | CH₃ |
| A-47 | pyridin-2-yl | 5-CH₂OCH₃ | CH₃ |
| A-48 | pyridin-2-yl | 5-C₂H₅ | CH₃ |
| A-49 | pyridin-2-yl | 5-CH₂CF₃ | CH₃ |
| A-50 | pyridin-2-yl | 5-CH=CH₂ | CH₃ |
| A-51 | pyridin-2-yl | 5-C≡CH | CH₃ |
| A-52 | pyridin-2-yl | 5-C≡CCH₃ | CH₃ |
| A-53 | pyridin-2-yl | 5-C₃H₅ | CH₃ |
| A-54 | pyridin-2-yl | 5-C(=NOCH₃)CH₃ | CH₃ |
| A-55 | pyridin-2-yl | 5-CN | CH₃ |
| A-56 | pyridin-2-yl | 6-F | CH₃ |
| A-57 | pyridin-2-yl | 6-CI | CH₃ |
| A-58 | pyridin-2-yl | 6-Br | CH₃ |
| A-59 | pyridin-2-yl | 6-CH₃ | CH₃ |
| A-60 | pyridin-2-yl | 6-CHF₂ | CH₃ |
| A-61 | pyridin-2-yl | 6-CF₃ | CH₃ |
| A-62 | pyridin-2-yl | 6-OCH₃ | CH₃ |
| A-63 | pyridin-2-yl | 6-OCHF₂ | CH₃ |
| A-64 | pyridin-2-yl | 6-OCF₃ | CH₃ |
| A-65 | pyridin-2-yl | 6-CH₂OCH₃ | CH₃ |
| A-66 | pyridin-2-yl | 6-C₂H₅ | CH₃ |
| A-67 | pyridin-2-yl | 6-CH₂CF₃ | CH₃ |
| A-68 | pyridin-2-yl | 6-CH=CH₂ | CH₃ |
| A-69 | pyridin-2-yl | 6-C≡CH | CH₃ |
| A-70 | pyridin-2-yl | 6-C≡CCH₃ | CH₃ |
| A-71 | pyridin-2-yl | 6-C₃H₅ | CH₃ |
| A-72 | pyridin-2-yl | 6-C(=NOCH₃)CH₃ | CH₃ |
| A-73 | pyridin-2-yl | 6-CN | CH₃ |
| A-74 | 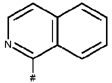 | | CH₃ |
| A-75 | pyridin-3-yl | - | CH₃ |
| A-76 | pyridin-3-yl | 2-F | CH₃ |
| A-77 | pyridin-3-yl | 2-CI | CH₃ |
| A-78 | pyridin-3-yl | 2-Br | CH₃ |
| A-79 | pyridin-3-yl | 2-CH₃ | CH₃ |
| A-80 | pyridin-3-yl | 2-CHF₂ | CH₃ |
| A-81 | pyridin-3-yl | 2-CF₃ | CH₃ |
| A-82 | pyridin-3-yl | 2-OCH₃ | CH₃ |
| A-83 | pyridin-3-yl | 2-OCHF₂ | CH₃ |
| A-84 | pyridin-3-yl | 2-OCF₃ | CH₃ |
| A-85 | pyridin-3-yl | 2-CH₂OCH₃ | CH₃ |
| A-86 | pyridin-3-yl | 2-C₂H₅ | CH₃ |
| A-87 | pyridin-3-yl | 2-CH₂CF₃ | CH₃ |
| A-88 | pyridin-3-yl | 2-CH=CH₂ | CH₃ |
| A-89 | pyridin-3-yl | 2-C≡CH | CH₃ |
| A-90 | pyridin-3-yl | 2-C≡CCH₃ | CH₃ |
| A-91 | pyridin-3-yl | 2-C₃H₅ | CH₃ |
| A-92 | pyridin-3-yl | 2-C(=NOCH₃)CH₃ | CH₃ |
| A-93 | pyridin-3-yl | 2-CN | CH₃ |
| A-94 | pyridin-3-yl | 4-F | CH₃ |
| A-95 | pyridin-3-yl | 4-CI | CH₃ |
| A-96 | pyridin-3-yl | 4-Br | CH₃ |
| A-97 | pyridin-3-yl | 4-CH₃ | CH₃ |
| A-98 | pyridin-3-yl | 4-CHF₂ | CH₃ |
| A-99 | pyridin-3-yl | 4-CF₃ | CH₃ |
| A-100 | pyridin-3-yl | 4-OCH₃ | CH₃ |
| A-101 | pyridin-3-yl | 4-OCHF₂ | CH₃ |
| A-102 | pyridin-3-yl | 4-OCF₃ | CH₃ |
| A-103 | pyridin-3-yl | 4-CH₂OCH₃ | CH₃ |
| A-104 | pyridin-3-yl | 4-C₂H₅ | CH₃ |
| A-105 | pyridin-3-yl | 4-CH₂CF₃ | CH₃ |
| A-106 | pyridin-3-yl | 4-CH=CH₂ | CH₃ |
| A-107 | pyridin-3-yl | 4-C≡CH | CH₃ |
| A-108 | pyridin-3-yl | 4-C≡CCH₃ | CH₃ |
| A-109 | pyridin-3-yl | 4-C₃H₅ | CH₃ |
| A-110 | pyridin-3-yl | 4-C(=NOCH₃)CH₃ | CH₃ |
| A-111 | pyridin-3-yl | 4-CN | CH₃ |
| A-112 | pyridin-3-yl | 5-F | CH₃ |
| A-113 | pyridin-3-yl | 5-CI | CH₃ |
| A-114 | pyridin-3-yl | 5-Br | CH₃ |
| A-115 | pyridin-3-yl | 5-CH₃ | CH₃ |
| A-116 | pyridin-3-yl | 5-CHF₂ | CH₃ |
| A-117 | pyridin-3-yl | 5-CF₃ | CH₃ |
| A-118 | pyridin-3-yl | 5-OCH₃ | CH₃ |
| A-119 | pyridin-3-yl | 5-OCHF₂ | CH₃ |
| A-120 | pyridin-3-yl | 5-OCF₃ | CH₃ |
| A-121 | pyridin-3-yl | 5-CH₂OCH₃ | CH₃ |
| A-122 | pyridin-3-yl | 5-C₂H₅ | CH₃ |
| A-123 | pyridin-3-yl | 5-CH₂CF₃ | CH₃ |
| A-124 | pyridin-3-yl | 5-CH=CH₂ | CH₃ |
| A-125 | pyridin-3-yl | 5-C≡CH | CH₃ |
| A-126 | pyridin-3-yl | 5-C≡CCH₃ | CH₃ |
| A-127 | pyridin-3-yl | 5-C₃H₅ | CH₃ |
| A-128 | pyridin-3-yl | 5-C(=NOCH₃)CH₃ | CH₃ |
| A-129 | pyridin-3-yl | 5-CN | CH₃ |
| A-130 | pyridin-3-yl | 6-F | CH₃ |
| A-131 | pyridin-3-yl | 6-CI | CH₃ |
| A-132 | pyridin-3-yl | 6-Br | CH₃ |
| A-133 | pyridin-3-yl | 6-CH₃ | CH₃ |
| A-134 | pyridin-3-yl | 6-CHF₂ | CH₃ |
| A-135 | pyridin-3-yl | 6-CF₃ | CH₃ |
| A-136 | pyridin-3-yl | 6-OCH₃ | CH₃ |
| A-137 | pyridin-3-yl | 6-OCHF₂ | CH₃ |
| A-138 | pyridin-3-yl | 6-OCF₃ | CH₃ |
| A-139 | pyridin-3-yl | 6-CH₂OCH₃ | CH₃ |
| A-140 | pyridin-3-yl | 6-C₂H₅ | CH₃ |
| A-141 | pyridin-3-yl | 6-CH₂CF₃ | CH₃ |
| A-142 | pyridin-3-yl | 6-CH=CH₂ | CH₃ |
| A-143 | pyridin-3-yl | 6-C≡CH | CH₃ |
| A-144 | pyridin-3-yl | 6-C≡CCH₃ | CH₃ |
| A-145 | pyridin-3-yl | 6-C₃H₅ | CH₃ |
| A-146 | pyridin-3-yl | 6-C(=NOCH₃)CH₃ | CH₃ |
| A-147 | pyridin-3-yl | 6-CN | CH₃ |
| A-148 | 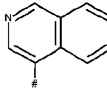 | | CH₃ |
| A-149 | pyridin-4-yl | - | CH₃ |
| A-150 | pyridin-4-yl | 2-F | CH₃ |
| A-151 | pyridin-4-yl | 2-CI | CH₃ |
| A-152 | pyridin-4-yl | 2-Br | CH₃ |
| A-153 | pyridin-4-yl | 2-CH₃ | CH₃ |
| A-154 | pyridin-4-yl | 2-CHF₂ | CH₃ |
| A-155 | pyridin-4-yl | 2-CF₃ | CH₃ |
| A-156 | pyridin-4-yl | 2-OCH₃ | CH₃ |
| A-157 | pyridin-4-yl | 2-OCHF₂ | CH₃ |
| A-158 | pyridin-4-yl | 2-OCF₃ | CH₃ |
| A-159 | pyridin-4-yl | 2-CH₂OCH₃ | CH₃ |
| A-160 | pyridin-4-yl | 2-C₂H₅ | CH₃ |
| A-161 | pyridin-4-yl | 2-CH₂CF₃ | CH₃ |
| A-162 | pyridin-4-yl | 2-CH=CH₂ | CH₃ |
| A-163 | pyridin-4-yl | 2-C=CH | CH₃ |
| A-164 | pyridin-4-yl | 2-C≡CCH₃ | CH₃ |
| A-165 | pyridin-4-yl | 2-C₃H₅ | CH₃ |
| A-166 | pyridin-4-yl | 2-C(=NOCH₃)CH₃ | CH₃ |
| A-167 | pyridin-4-yl | 2-CN | CH₃ |
| A-168 | pyridin-4-yl | 3-F | CH₃ |
| A-169 | pyridin-4-yl | 3-CI | CH₃ |
| A-170 | pyridin-4-yl | 3-Br | CH₃ |
| A-171 | pyridin-4-yl | 3-CH₃ | CH₃ |
| A-172 | pyridin-4-yl | 3-CHF₂ | CH₃ |
| A-173 | pyridin-4-yl | 3-CF₃ | CH₃ |
| A-174 | pyridin-4-yl | 3-OCH₃ | CH₃ |
| A-175 | pyridin-4-yl | 3-OCHF₂ | CH₃ |
| A-176 | pyridin-4-yl | 3-OCF₃ | CH₃ |
| A-177 | pyridin-4-yl | 3-CH₂OCH₃ | CH₃ |
| A-178 | pyridin-4-yl | 3-C₂H₅ | CH₃ |
| A-179 | pyridin-4-yl | 3-CH₂CF₃ | CH₃ |
| A-180 | pyridin-4-yl | 3-CH=CH₂ | CH₃ |
| A-181 | pyridin-4-yl | 3-C≡CH | CH₃ |
| A-182 | pyridin-4-yl | 3-C≡CCH₃ | CH₃ |
| A-183 | pyridin-4-yl | 3-C₃H₅ | CH₃ |
| A-184 | pyridin-4-yl | 3-C(=NOCH₃)CH₃ | CH₃ |
| A-185 | pyridin-4-yl | 3-CN | CH₃ |
| A-186 | pyridin-4-yl | 5-F | CH₃ |
| A-187 | pyridin-4-yl | 5-CI | CH₃ |
| A-188 | pyridin-4-yl | 5-Br | CH₃ |
| A-189 | pyridin-4-yl | 5-CH₃ | CH₃ |
| A-190 | pyridin-4-yl | 5-CHF₂ | CH₃ |
| A-191 | pyridin-4-yl | 5-CF₃ | CH₃ |
| A-192 | pyridin-4-yl | 5-OCH₃ | CH₃ |
| A-193 | pyridin-4-yl | 5-OCHF₂ | CH₃ |
| A-194 | pyridin-4-yl | 5-OCF₃ | CH₃ |
| A-195 | pyridin-4-yl | 5-CH₂OCH₃ | CH₃ |
| A-196 | pyridin-4-yl | 5-C₂H₅ | CH₃ |
| A-197 | pyridin-4-yl | 5-CH₂CF₃ | CH₃ |
| A-198 | pyridin-4-yl | 5-CH=CH₂ | CH₃ |
| A-199 | pyridin-4-yl | 5-C=CH | CH₃ |
| A-200 | pyridin-4-yl | 5-C≡CCH₃ | CH₃ |
| A-201 | pyridin-4-yl | 5-C₃H₅ | CH₃ |
| A-202 | pyridin-4-yl | 5-C(=NOCH₃)CH₃ | CH₃ |
| A-203 | pyridin-4-yl | 5-CN | CH₃ |
| A-204 | pyridin-4-yl | 6-F | CH₃ |
| A-205 | pyridin-4-yl | 6-Cl | CH₃ |
| A-206 | pyridin-4-yl | 6-Br | CH₃ |
| A-207 | pyridin-4-yl | 6-CH₃ | CH₃ |
| A-208 | pyridin-4-yl | 6-CHF₂ | CH₃ |
| A-209 | pyridin-4-yl | 6-CF₃ | CH₃ |
| A-210 | pyridin-4-yl | 6-OCH₃ | CH₃ |
| A-211 | pyridin-4-yl | 6-OCHF₂ | CH₃ |
| A-212 | pyridin-4-yl | 6-OCF₃ | CH₃ |
| A-213 | pyridin-4-yl | 6-CH₂OCH₃ | CH₃ |
| A-214 | pyridin-4-yl | 6-C₂H₅ | CH₃ |
| A-215 | pyridin-4-yl | 6-CH₂CF₃ | CH₃ |
| A-216 | pyridin-4-yl | 6-CH=CH₂ | CH₃ |
| A-217 | pyridin-4-yl | 6-C=CH | CH₃ |
| A-218 | pyridin-4-yl | 6-C≡CCH₃ | CH₃ |
| A-219 | pyridin-4-yl | 6-C₃H₅ | CH₃ |
| A-220 | pyridin-4-yl | 6-C(=NOCH₃)CH₃ | CH₃ |
| A-221 | pyridin-4-yl | 6-CN | CH₃ |
| A-222 | 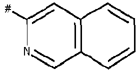 | | CH₃ |
| A-223 | thiazol-2-yl | - | CH₃ |
| A-224 | thiazol-2-yl | 3-F | CH₃ |
| A-225 | thiazol-2-yl | 3-CI | CH₃ |
| A-226 | thiazol-2-yl | 3-Br | CH₃ |
| A-227 | thiazol-2-yl | 3-CH₃ | CH₃ |
| A-228 | thiazol-2-yl | 3-CHF₂ | CH₃ |
| A-229 | thiazol-2-yl | 3-CF₃ | CH₃ |
| A-230 | thiazol-2-yl | 3-OCH₃ | CH₃ |
| A-231 | thiazol-2-yl | 3-OCHF₂ | CH₃ |
| A-232 | thiazol-2-yl | 3-OCF₃ | CH₃ |
| A-233 | thiazol-2-yl | 3-CH₂OCH₃ | CH₃ |
| A-234 | thiazol-2-yl | 3-C₂H₅ | CH₃ |
| A-235 | thiazol-2-yl | 3-CH₂CF₃ | CH₃ |
| A-236 | thiazol-2-yl | 3-CH=CH₂ | CH₃ |
| A-237 | thiazol-2-yl | 3-C≡CH | CH₃ |
| A-238 | thiazol-2-yl | 3-C≡CCH₃ | CH₃ |
| A-239 | thiazol-2-yl | 3-C₃H₅ | CH₃ |
| A-240 | thiazol-2-yl | 3-C(=NOCH₃)CH₃ | CH₃ |
| A-241 | thiazol-2-yl | 3-CN | CH₃ |
| A-242 | thiazol-2-yl | 4-F | CH₃ |
| A-243 | thiazol-2-yl | 4-CI | CH₃ |
| A-244 | thiazol-2-yl | 4-Br | CH₃ |
| A-245 | thiazol-2-yl | 4-CH₃ | CH₃ |
| A-246 | thiazol-2-yl | 4-CHF₂ | CH₃ |
| A-247 | thiazol-2-yl | 4-CF₃ | CH₃ |
| A-248 | thiazol-2-yl | 4-OCH₃ | CH₃ |
| A-249 | thiazol-2-yl | 4-OCHF₂ | CH₃ |
| A-250 | thiazol-2-yl | 4-OCF₃ | CH₃ |
| A-251 | thiazol-2-yl | 4-CH₂OCH₃ | CH₃ |
| A-252 | thiazol-2-yl | 4-C₂H₅ | CH₃ |
| A-253 | thiazol-2-yl | 4-CH₂CF₃ | CH₃ |
| A-254 | thiazol-2-yl | 4-CH=CH₂ | CH₃ |
| A-255 | thiazol-2-yl | 4-C≡CH | CH₃ |
| A-256 | thiazol-2-yl | 4-C≡CCH₃ | CH₃ |
| A-257 | thiazol-2-yl | 4-C₃H₅ | CH₃ |
| A-258 | thiazol-2-yl | 4-C(=NOCH₃)CH₃ | CH₃ |
| A-259 | thiazol-2-yl | 4-CN | CH₃ |
| A-260 | thiazol-2-yl | 5-F | CH₃ |
| A-261 | thiazol-2-yl | 5-CI | CH₃ |
| A-262 | thiazol-2-yl | 5-Br | CH₃ |
| A-263 | thiazol-2-yl | 5-CH₃ | CH₃ |
| A-264 | thiazol-2-yl | 5-CHF₂ | CH₃ |
| A-265 | thiazol-2-yl | 5-CF₃ | CH₃ |
| A-266 | thiazol-2-yl | 5-OCH₃ | CH₃ |
| A-267 | thiazol-2-yl | 5-OCHF₂ | CH₃ |
| A-268 | thiazol-2-yl | 5-OCF₃ | CH₃ |
| A-269 | thiazol-2-yl | 5-CH₂OCH₃ | CH₃ |
| A-270 | thiazol-2-yl | 5-C₂H₅ | CH₃ |
| A-271 | thiazol-2-yl | 5-CH₂CF₃ | CH₃ |
| A-272 | thiazol-2-yl | 5-CH=CH₂ | CH₃ |
| A-273 | thiazol-2-yl | 5-C≡CH | CH₃ |
| A-274 | thiazol-2-yl | 5-C≡CCH₃ | CH₃ |
| A-275 | thiazol-2-yl | 5-C₃H₅ | CH₃ |
| A-276 | thiazol-2-yl | 5-C(=NOCH₃)CH₃ | CH₃ |
| A-277 | thiazol-2-yl | 5-CN | CH₃ |
| A-278 | thiazol-2-yl | 6-F | CH₃ |
| A-279 | thiazol-2-yl | 6-CI | CH₃ |
| A-280 | thiazol-2-yl | 6-Br | CH₃ |
| A-281 | thiazol-2-yl | 6-CH₃ | CH₃ |
| A-282 | thiazol-2-yl | 6-CHF₂ | CH₃ |
| A-283 | thiazol-2-yl | 6-CF₃ | CH₃ |
| A-284 | thiazol-2-yl | 6-OCH₃ | CH₃ |
| A-285 | thiazol-2-yl | 6-OCHF₂ | CH₃ |
| A-286 | thiazol-2-yl | 6-OCF₃ | CH₃ |
| A-287 | thiazol-2-yl | 6-CH₂OCH₃ | CH₃ |
| A-288 | thiazol-2-yl | 6-C₂H₅ | CH₃ |
| A-289 | thiazol-2-yl | 6-CH₂CF₃ | CH₃ |
| A-290 | thiazol-2-yl | 6-CH=CH₂ | CH₃ |
| A-291 | thiazol-2-yl | 6-C≡CH | CH₃ |
| A-292 | thiazol-2-yl | 6-C≡CCH₃ | CH₃ |
| A-293 | thiazol-2-yl | 6-C₃H₅ | CH₃ |
| A-294 | thiazol-2-yl | 6-C(=NOCH₃)CH₃ | CH₃ |
| A-295 | thiazol-2-yl | 6-CN | CH₃ |
| A-296 | thiazol-4-yl | - | CH₃ |
| A-297 | thiazol-4-yl | 3-F | CH₃ |
| A-298 | thiazol-4-yl | 3-CI | CH₃ |
| A-299 | thiazol-4-yl | 3-Br | CH₃ |
| A-300 | thiazol-4-yl | 3-CH₃ | CH₃ |
| A-301 | thiazol-4-yl | 3-CHF₂ | CH₃ |
| A-302 | thiazol-4-yl | 3-CF₃ | CH₃ |
| A-303 | thiazol-4-yl | 3-OCH₃ | CH₃ |
| A-304 | thiazol-4-yl | 3-OCHF₂ | CH₃ |
| A-305 | thiazol-4-yl | 3-OCF₃ | CH₃ |
| A-306 | thiazol-4-yl | 3-CH₂OCH₃ | CH₃ |
| A-307 | thiazol-4-yl | 3-C₂H₅ | CH₃ |
| A-308 | thiazol-4-yl | 3-CH₂CF₃ | CH₃ |
| A-309 | thiazol-4-yl | 3-CH=CH₂ | CH₃ |
| A-310 | thiazol-4-yl | 3-C≡CH | CH₃ |
| A-311 | thiazol-4-yl | 3-C≡CCH₃ | CH₃ |
| A-312 | thiazol-4-yl | 3-C₃H₅ | CH₃ |
| A-313 | thiazol-4-yl | 3-C(=NOCH₃)CH₃ | CH₃ |
| A-314 | thiazol-4-yl | 3-CN | CH₃ |
| A-315 | thiazol-4-yl | 4-F | CH₃ |
| A-316 | thiazol-4-yl | 4-CI | CH₃ |
| A-317 | thiazol-4-yl | 4-Br | CH₃ |
| A-318 | thiazol-4-yl | 4-CH₃ | CH₃ |
| A-319 | thiazol-4-yl | 4-CHF₂ | CH₃ |
| A-320 | thiazol-4-yl | 4-CF₃ | CH₃ |
| A-321 | thiazol-4-yl | 4-OCH₃ | CH₃ |
| A-322 | thiazol-4-yl | 4-OCHF₂ | CH₃ |
| A-323 | thiazol-4-yl | 4-OCF₃ | CH₃ |
| A-324 | thiazol-4-yl | 4-CH₂OCH₃ | CH₃ |
| A-325 | thiazol-4-yl | 4-C₂H₅ | CH₃ |
| A-326 | thiazol-4-yl | 4-CH₂CF₃ | CH₃ |
| A-327 | thiazol-4-yl | 4-CH=CH₂ | CH₃ |
| A-328 | thiazol-4-yl | 4-C≡CH | CH₃ |
| A-329 | thiazol-4-yl | 4-C≡CCH₃ | CH₃ |
| A-330 | thiazol-4-yl | 4-C₃H₅ | CH₃ |
| A-331 | thiazol-4-yl | 4-C(=NOCH₃)CH₃ | CH₃ |
| A-332 | thiazol-4-yl | 4-CN | CH₃ |
| A-333 | thiazol-4-yl | 5-F | CH₃ |
| A-334 | thiazol-4-yl | 5-CI | CH₃ |
| A-335 | thiazol-4-yl | 5-Br | CH₃ |
| A-336 | thiazol-4-yl | 5-CH₃ | CH₃ |
| A-337 | thiazol-4-yl | 5-CHF₂ | CH₃ |
| A-338 | thiazol-4-yl | 5-CF₃ | CH₃ |
| A-339 | thiazol-4-yl | 5-OCH₃ | CH₃ |
| A-340 | thiazol-4-yl | 5-OCHF₂ | CH₃ |
| A-341 | thiazol-4-yl | 5-OCF₃ | CH₃ |
| A-342 | thiazol-4-yl | 5-CH₂OCH₃ | CH₃ |
| A-343 | thiazol-4-yl | 5-C₂H₅ | CH₃ |
| A-344 | thiazol-4-yl | 5-CH₂CF₃ | CH₃ |
| A-345 | thiazol-4-yl | 5-CH=CH₂ | CH₃ |
| A-346 | thiazol-4-yl | 5-C≡CH | CH₃ |
| A-347 | thiazol-4-yl | 5-C≡CCH₃ | CH₃ |
| A-348 | thiazol-4-yl | 5-C₃H₅ | CH₃ |
| A-349 | thiazol-4-yl | 5-C(=NOCH₃)CH₃ | CH₃ |
| A-350 | thiazol-4-yl | 5-CN | CH₃ |
| A-351 | thiazol-4-yl | 6-F | CH₃ |
| A-352 | thiazol-4-yl | 6-CI | CH₃ |
| A-353 | thiazol-4-yl | 6-Br | CH₃ |
| A-354 | thiazol-4-yl | 6-CH₃ | CH₃ |
| A-355 | thiazol-4-yl | 6-CHF₂ | CH₃ |
| A-356 | thiazol-4-yl | 6-CF₃ | CH₃ |
| A-357 | thiazol-4-yl | 6-OCH₃ | CH₃ |
| A-358 | thiazol-4-yl | 6-OCHF₂ | CH₃ |
| A-359 | thiazol-4-yl | 6-OCF₃ | CH₃ |
| A-360 | thiazol-4-yl | 6-CH₂OCH₃ | CH₃ |
| A-361 | thiazol-4-yl | 6-C₂H₅ | CH₃ |
| A-362 | thiazol-4-yl | 6-CH₂CF₃ | CH₃ |
| A-363 | thiazol-4-yl | 6-CH=CH₂ | CH₃ |
| A-364 | thiazol-4-yl | 6-C≡CH | CH₃ |
| A-365 | thiazol-4-yl | 6-C≡CCH₃ | CH₃ |
| A-366 | thiazol-4-yl | 6-C₃H₅ | CH₃ |
| A-367 | thiazol-4-yl | 6-C(=NOCH₃)CH₃ | CH₃ |
| A-368 | thiazol-4-yl | 6-CN | CH₃ |
| A-369 | isothiazol-3-yl | - | CH₃ |
| A-370 | isothiazol-3-yl | 3-F | CH₃ |
| A-371 | isothiazol-3-yl | 3-CI | CH₃ |
| A-372 | isothiazol-3-yl | 3-Br | CH₃ |
| A-373 | isothiazol-3-yl | 3-CH₃ | CH₃ |
| A-374 | isothiazol-3-yl | 3-CHF₂ | CH₃ |
| A-375 | isothiazol-3-yl | 3-CF₃ | CH₃ |
| A-376 | isothiazol-3-yl | 3-OCH₃ | CH₃ |
| A-377 | isothiazol-3-yl | 3-OCHF₂ | CH₃ |
| A-378 | isothiazol-3-yl | 3-OCF₃ | CH₃ |
| A-379 | isothiazol-3-yl | 3-CH₂OCH₃ | CH₃ |
| A-380 | isothiazol-3-yl | 3-C₂H₅ | CH₃ |
| A-381 | isothiazol-3-yl | 3-CH₂CF₃ | CH₃ |
| A-382 | isothiazol-3-yl | 3-CH=CH₂ | CH₃ |
| A-383 | isothiazol-3-yl | 3-C≡CH | CH₃ |
| A-384 | isothiazol-3-yl | 3-C≡CCH₃ | CH₃ |
| A-385 | isothiazol-3-yl | 3-C₃H₅ | CH₃ |
| A-386 | isothiazol-3-yl | 3-C(=NOCH₃)CH₃ | CH₃ |
| A-387 | isothiazol-3-yl | 3-CN | CH₃ |
| A-388 | isothiazol-3-yl | 4-F | CH₃ |
| A-389 | isothiazol-3-yl | 4-CI | CH₃ |
| A-390 | isothiazol-3-yl | 4-Br | CH₃ |
| A-391 | isothiazol-3-yl | 4-CH₃ | CH₃ |
| A-392 | isothiazol-3-yl | 4-CHF₂ | CH₃ |
| A-393 | isothiazol-3-yl | 4-CF₃ | CH₃ |
| A-394 | isothiazol-3-yl | 4-OCH₃ | CH₃ |
| A-395 | isothiazol-3-yl | 4-OCHF₂ | CH₃ |
| A-396 | isothiazol-3-yl | 4-OCF₃ | CH₃ |
| A-397 | isothiazol-3-yl | 4-CH₂OCH₃ | CH₃ |
| A-398 | isothiazol-3-yl | 4-C₂H₅ | CH₃ |
| A-399 | isothiazol-3-yl | 4-CH₂CF₃ | CH₃ |
| A-400 | isothiazol-3-yl | 4-CH=CH₂ | CH₃ |
| A-401 | isothiazol-3-yl | 4-C≡CH | CH₃ |
| A-402 | isothiazol-3-yl | 4-C≡CCH₃ | CH₃ |
| A-403 | isothiazol-3-yl | 4-C₃H₅ | CH₃ |
| A-404 | isothiazol-3-yl | 4-C(=NOCH₃)CH₃ | CH₃ |
| A-405 | isothiazol-3-yl | 4-CN | CH₃ |
| A-406 | isothiazol-3-yl | 5-F | CH₃ |
| A-407 | isothiazol-3-yl | 5-CI | CH₃ |
| A-408 | isothiazol-3-yl | 5-Br | CH₃ |
| A-409 | isothiazol-3-yl | 5-CH₃ | CH₃ |
| A-410 | isothiazol-3-yl | 5-CHF₂ | CH₃ |
| A-411 | isothiazol-3-yl | 5-CF₃ | CH₃ |
| A-412 | isothiazol-3-yl | 5-OCH₃ | CH₃ |
| A-413 | isothiazol-3-yl | 5-OCHF₂ | CH₃ |
| A-414 | isothiazol-3-yl | 5-OCF₃ | CH₃ |
| A-415 | isothiazol-3-yl | 5-CH₂OCH₃ | CH₃ |
| A-416 | isothiazol-3-yl | 5-C₂H₅ | CH₃ |
| A-417 | isothiazol-3-yl | 5-CH₂CF₃ | CH₃ |
| A-418 | isothiazol-3-yl | 5-CH=CH₂ | CH₃ |
| A-419 | isothiazol-3-yl | 5-C≡CH | CH₃ |
| A-420 | isothiazol-3-yl | 5-C≡CCH₃ | CH₃ |
| A-421 | isothiazol-3-yl | 5-C₃H₅ | CH₃ |
| A-422 | isothiazol-3-yl | 5-C(=NOCH₃)CH₃ | CH₃ |
| A-423 | isothiazol-3-yl | 5-CN | CH₃ |
| A-424 | isothiazol-3-yl | 6-F | CH₃ |
| A-425 | isothiazol-3-yl | 6-CI | CH₃ |
| A-426 | isothiazol-3-yl | 6-Br | CH₃ |
| A-427 | isothiazol-3-yl | 6-CH₃ | CH₃ |
| A-428 | isothiazol-3-yl | 6-CHF₂ | CH₃ |
| A-429 | isothiazol-3-yl | 6-CF₃ | CH₃ |
| A-430 | isothiazol-3-yl | 6-OCH₃ | CH₃ |
| A-431 | isothiazol-3-yl | 6-OCHF₂ | CH₃ |
| A-432 | isothiazol-3-yl | 6-OCF₃ | CH₃ |
| A-433 | isothiazol-3-yl | 6-CH₂OCH₃ | CH₃ |
| A-434 | isothiazol-3-yl | 6-C₂Hs | CH₃ |
| A-435 | isothiazol-3-yl | 6-CH₂CF₃ | CH₃ |
| A-436 | isothiazol-3-yl | 6-CH=CH₂ | CH₃ |
| A-437 | isothiazol-3-yl | 6-C≡CH | CH₃ |
| A-438 | isothiazol-3-yl | 6-C≡CCH₃ | CH₃ |
| A-439 | isothiazol-3-yl | 6-C₃H₅ | CH₃ |
| A-440 | isothiazol-3-yl | 6-C(=NOCH₃)CH₃ | CH₃ |
| A-441 | isothiazol-3-yl | 6-CN | CH₃ |

The compounds can be obtained by various routes in analogy to prior art processes known (e.g. EP 414153, WO 98/23156) and, advantageously, by the synthesis shown in the following schemes 1 to 4 and in the experimental part of this application.

A suitable method to prepare compounds I is illustrated in Scheme 1.

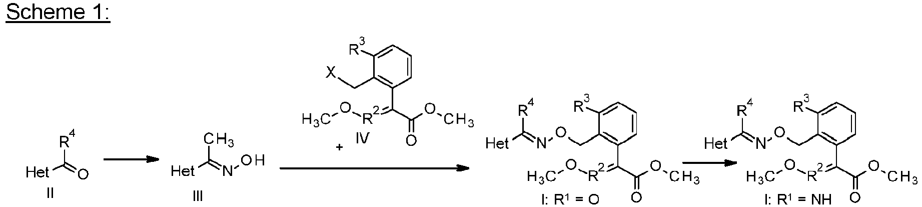

It starts with the conversion of a ketone to the corresponding oxime using hydxroxylamine hydrochloride and a base such as pyridine, sodium hydroxide or sodium acetate in polar solvents such as methanol, methanol-water mixture, or ethanol at reaction temperatures of 60 to 100 °C, preferably at about 65 °C. In cases where a *E*/*Z* mixture was obtained, the isomers could be separated by purification techniques known in art (e.g. column chromatography, crystallization, distillation etc.). Then, coupling with the intermediate IV, wherein X is a leaving group such as halogen, toluene- and methanesulfonates, preferably X is Cl or Br, is carried out under basic conditions using e.g. sodium hydride, cesium carbonate or potassium carbonate as a base and using an organic solvent such as dimethyl formamide (DMF) or acetonitrile (ACN), preferably cesium carbonate as base and ACN as solvent at room temperature (RT) of about 24 °C . The ester compound I wherein R¹ is O can be converted to the amide of formula I wherein R¹ is NH by reaction with methyl amine (preferably 40% aq. solution) using tetrahydrofuran (THF) as solvent at RT.

Another general method to prepare the compounds I is depicted in Scheme 2.

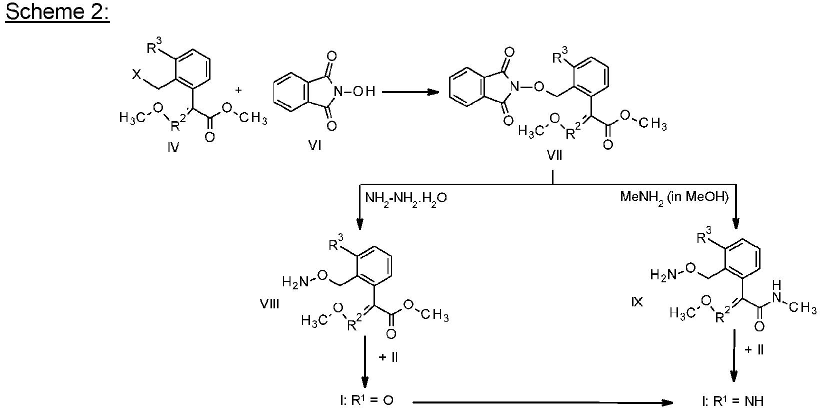

Intermediate IV is reacted with N-hydroxysuccimide VI, using a base such as triethylamine in DMF. The reaction temperature is usually 50 to 70 °C preferably about 70 °C. Conversion to the corresponding O-benzylhydroxyl amine, intermediate VIII, was achieved through removal of the phthalimide group, preferably using hydrazine hydrate in methanol as solvent at 25 °C. Alternatively, removal of the phthalimide group using methyl amine in methanol as solvent at 25 °C can provide intermediate IX. Intermediate VIII and intermediate IX, respectively can be condensed with ketones using acetic acid or pyridine in methanol as solvent at temperature of 50 to 65 °C. Alternatively, the condensation could also carried out with titanium (IV) ethoxide (Ti(OEt)₄) using THF as solvent at about 70 °C. The desired product is usually accompanied by an undesired isomer, which can be removed e.g by column chromatography, crystallization.

A general method for preparation of intermediate IV is shown in Scheme 3.

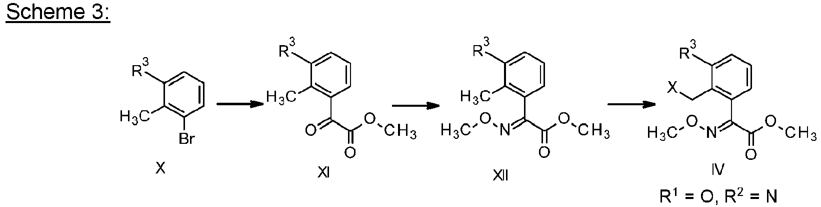

Compound XI could be obtained from X by lithium-halogen exchange or by generating Grignard reagent and further reaction with dimethyl oxalate or chloromethyl oxalate in presence of a solvent. The preferred solvent is THF, 2-methyl-THF and the temperature can be between -70 to -78 °C. Conversion of intermediate XI to intermediate XII can be achieved using N-methylhydroxylamine hydrochloride and a base such as pyridine or sodium acetate in polar solvents such as methanol. The reaction temperature is preferably about 65 °C. An *E*/*Z* mixture is usually obtained, the isomers can be separated by purification techniques known in art (e.g. column chromatography, crystallization). Bromination of intermediate XII provides the desired intermediate compounds IV, wherein R¹ is O and R² = N. This reaction of intermediate XII with N-bromosuccinimide in solvents such as carbon tetrachloride, chlorobenzene, ACN, using radical initiators such as 1,1'-azobis (cyclohexanecarbonitrile) or azobisisobutyronitrile and is carried out at temperatures of 70 to 100 °C. The preferred radical initiator is 1,1-azobis (cyclohexanecarbonitrile), preferred solvent chlorobenzene and preferred temperature 80 °C.

The synthesis of compounds containing different substituents R³ follows similar sequence as in Scheme 3, wherein R³ is bromo. Coupling of intermediate III with intermediate IV, wherein R³ is bromo, provides compounds I as described above. Using standard chemical reactions, such as Suzuki or Stille reaction, the bromo group can be converted e.g. to other R³ substituents such as cycloalkyl, alkoxy and alkenyl. Additional transformations e.g. of ethenyl provide compounds I with other R³ substituents such as ethyl, CN and haloalkyl.

Most of the ketones of general formula II were commercially available, however for the ones which were not commercially available, preparation of these was carried out in house using methods known in prior art. Scheme 4 depicts various methods known in literature for the synthesis of these ketones.

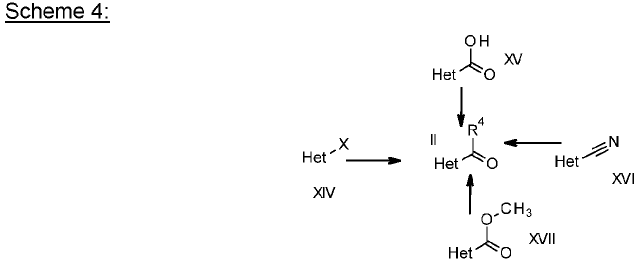

The ketone II can be obtained from the corresponding halogen bearing precursors XIV, wherein X is preferably bromine or iodine. Lithium-halogen exchange (J Org Chem, 1998, 63 (21), 7399-7407) in compound XIII using n-butyllithium or synthesis of the corresponding Grignard reagent (Nature Comm, 2017, 8(1), 1-7) using THF as solvent, and subsequent reaction with N-methoxy-N-methylacetamide at about -70 to -78 °C can provide the ketone II. Alternatively, the coupling reaction of compound XIV and tributyl(1-ethoxyvinyl)stannane in presence of a transition metal catalyst, preferably palladium, with suitable ligands in a solvent such as dioxane and at a reaction temperature of about 100 °C, followed by treatment with 1N HCl can provide ketone II (Org Lett, 2016, 18(7), 1630-1633, WO 2018/115380). Reaction of XIV with 1,4-butanediol vinyl ether in the presence of transition metal catalyst, preferably palladium with suitable ligands and solvent such as 1,2-propane diol and base such as sodium carbonate and reaction temperature of about 120 °C followed by treatment with 1N HCl can provide ketone II (Chem A Eur J, 2008, 14(18), 5555-5566). Another method uses acid compounds XV, which can be converted to the corresponding Weinreb amide or carboxylic ester XVII and subsequent reaction with methylmagnesium bromide (MeMgBr) in solvent such as THF and temperatures of -78 to 0 °C, preferably 0 °C, to provide ketone II. Another method uses the reaction of nitrile XVI with MeMgBr which is carried out in solvent such as THF or toluene, preferably THF, and reaction temperature is 25 to 60 °C, preferably 60 °C, followed by treatment with 1N HCl (Eur J Med Chem, 2015, 102, 582-593).

The compounds I and the compositions thereof, respectively, are suitable as fungicides effective against a broad spectrum of phytopathogenic fungi, including soil-borne fungi, in particular from the classes of Plasmodiophoromycetes, Peronosporomycetes (syn. Oomycetes), Chytridiomycetes, Zygomycetes, Ascomycetes, Basidiomycetes, and Deuteromycetes (syn. Fungi imperfecti). They can be used in crop protection as foliar fungicides, fungicides for seed dressing, and soil fungicides.

The compounds I and the compositions thereof are preferably useful in the control of phytopathogenic fungi on various cultivated plants, such as cereals, e. g. wheat, rye, barley, triticale, oats, or rice; beet, e. g. sugar beet or fodder beet; fruits, e. g. pomes (apples, pears, etc.), stone fruits (e.g. plums, peaches, almonds, cherries), or soft fruits, also called berries (strawberries, raspberries, blackberries, gooseberries, etc.); leguminous plants, e. g. lentils, peas, alfalfa, or soybeans; oil plants, e. g. oilseed rape, mustard, olives, sunflowers, coconut, cocoa beans, castor oil plants, oil palms, ground nuts, or soybeans; cucurbits, e. g. squashes, cucumber, or melons; fiber plants, e. g. cotton, flax, hemp, or jute; citrus fruits, e. g. oranges, lemons, grapefruits, or mandarins; vegetables, e. g. spinach, lettuce, asparagus, cabbages, carrots, onions, tomatoes, potatoes, cucurbits, or paprika; lauraceous plants, e. g. avocados, cinnamon, or camphor; energy and raw material plants, e. g. corn, soybean, oilseed rape, sugar cane, or oil palm; corn; tobacco; nuts; coffee; tea; bananas; vines (table grapes and grape juice grape vines); hop; turf; sweet leaf (also called Stevia); natural rubber plants; or ornamental and forestry plants, e. g. flowers, shrubs, broad-leaved trees, or evergreens (conifers, eucalypts, etc.); on the plant propagation material, such as seeds; and on the crop material of these plants.

More preferably, compounds I and compositions thereof, respectively are used for controlling fungi on field crops, such as potatoes, sugar beets, tobacco, wheat, rye, barley, oats, rice, corn, cotton, soybeans, oilseed rape, legumes, sunflowers, coffee or sugar cane; fruits; vines; ornamentals; or vegetables, such as cucumbers, tomatoes, beans or squashes.

The term "plant propagation material" is to be understood to denote all the generative parts of the plant, such as seeds; and vegetative plant materials, such as cuttings and tubers (e. g. potatoes), which can be used for the multiplication of the plant. This includes seeds, roots, fruits, tubers, bulbs, rhizomes, shoots, sprouts and other parts of plants; including seedlings and young plants to be transplanted after germination or after emergence from soil.

According to the invention all of the above cultivated plants are understood to comprise all species, subspecies, variants, varieties and/or hybrids which belong to the respective cultivated plants, including but not limited to winter and spring varieties, in particular in cereals such as wheat and barley, as well as oilseed rape, e.g. winter wheat, spring wheat, winter barley etc.

Corn is also known as Indian corn or maize (*Zea mays*) which comprises all kinds of corn such as field corn and sweet corn. According to the invention all maize or corn subspecies and/or varieties are comprised, in particular flour corn *(Zea mays* var. *amylacea*), popcorn (*Zea mays* var. *everta*), dent corn (*Zea mays var. indentata*), flint corn *(Zea mays* var. *indurata*), sweet corn *(Zea mays* var. *saccharata* and var. *rugosa*), waxy corn (*Zea mays var. ceratina*), amylomaize (high amylose *Zea mays* varieties), pod corn or wild maize *(Zea mays* var. *tunicata)* and striped maize (*Zea mays var. japonica*).

Most soybean cultivars are classifiable into indeterminate and determinate growth habit, whereas *Glycine soja,* the wild progenitor of soybean, is indeterminate (PNAS 2010, 107 (19) 8563-8568). The indeterminate growth habit (Maturity Group, MG 00 to MG 4.9) is characterized by a continuation of vegetative growth after flowering begins whereas determinate soybean varieties (MG 5 to MG 8) characteristically have finished most of their vegetative growth when flowering begins. According to the invention all soybean cultivars or varieties are comprised, in particular indeterminate and determinate cultivars or varieties.

Preferably, treatment of plant propagation materials with compounds I and compositions thereof, respectively, is used for controlling fungi on cereals, such as wheat, rye, barley and oats; rice, corn, cotton and soybeans.

The term "cultivated plants" is to be understood as including plants which have been modified by mutagenesis or genetic engineering to provide a new trait to a plant or to modify an already present trait. Mutagenesis includes random mutagenesis using X-rays or mutagenic chemicals, but also targeted mutagenesis to create mutations at a specific locus of a plant genome. Targeted mutagenesis frequently uses oligonucleotides or proteins like CRISPR/Cas, zinc-finger nucleases, TALENs or meganucleases. Genetic engineering usually uses recombinant DNA techniques to create modifications in a genome which under natural circumstances cannot readily be obtained by cross breeding, mutagenesis or natural recombination. Typically, one or more genes are integrated into the genome of a plant to add a trait or improve or modify a trait. These integrated genes are also referred to as transgenes, while plant comprising such transgenes are referred to as transgenic plants. The process of plant transformation usually produces several transformation events, which differ in the genomic locus in which a transgene has been integrated. Plants comprising a specific transgene on a specific genomic locus are usually described as comprising a specific "event", which is referred to by a specific event name. Traits which have been introduced in plants or have been modified include herbicide tolerance, insect resistance, increased yield and tolerance to abiotic conditions, like drought. Herbicide tolerance has been created by using mutagenesis and genetic engineering. Plants which have been rendered tolerant to acetolactate synthase (ALS) inhibitor herbicides by mutagenesis and breeding are e.g. available under the name Clearfield^{®}. Herbicide tolerance to glyphosate, glufosinate, 2,4-D, dicamba, oxynil herbicides, like bromoxynil and ioxynil, sulfonyl-urea herbicides, ALS inhibitors and 4-hydroxyphenylpyruvate dioxygenase (HPPD) inhibitors, like isoxaflutole and mesotrione, has been created via the use of transgenes.

The compounds I and compositions thereof, respectively, are particularly suitable for controlling the following causal agents of plant diseases: rusts on soybean and cereals (e.g. *Phakopsora pachyrhizi* and *P. meibomiae* on soy; *Puccinia tritici, P. graminis, P. recondita* and *P. striiformis* on wheat); molds on specialty crops, soybean, oil seed rape and sunflowers (e.g. *Botrytis cinerea* on strawberries and vines, *Sclerotinia sclerotiorum, S. minor and S. rolfsii* on oil seed rape, sunflowers and soybean); Fusarium diseases on cereals (e.g. *Fusarium culmorum* and *F. graminearum* on wheat); downy mildews on specialty crops (e.g. *Plasmopara viticola* on vines, *Phytophthora infestans* on potatoes); powdery mildews on specialty crops and cereals (e.g. *Uncinula necator* on vines, *Erysiphe* spp. on various specialty crops, *Blumeria graminis* on cereals); and leaf spots on cereals, soybean and corn (e.g. *Zymoseptoria tritici* and *Septoria nodorum* on cereals, *S. glycines* on soybean, *Cercospora* spp. on corn and soybean).

A further embodiment relates to the use of compound of formula (I) for combating soybean rust on soybean plants and on the plant propagation material, such as seeds, and the crop material of these plants. Soybean rust is cause by two fungal pathogens called *Phakopsora pachyrhizi* and *P. meibomiae.*

Consequently, a further embodiment relates to the use of compounds I for combating *Phakopsora pachyrhizi* and/or *P. meibomiae* on soybean plants and on the plant propagation material, such as seeds, and the crop material of these plants. A more preferred embodiment the use of compounds I for combating *Phakopsora pachyrhizi* on soybean plants and on the plant propagation material, such as seeds, and the crop material of these plants.

Accordingly, the present invention relates to the method for combating soybean rust *(Phakopsora pachyrhizi* and/or *P. meibomiae*), comprising:
treating the soybean plants or soybean plant propagation material to be protected against attack *by Phakopsora pachyrhizi* and/or *P. meibomiae* with an effective amount of at least one compound I, or a composition comprising such compound I.

Treatment against soybean rust can be preventive or curative.

Preferably treatment of soybean plants against soybean rust shall be preventive. Preventive treatment shall be performed when the soybean plants are at risk of infection latest shortly after the first symptoms are visible. According to one embodiment, the first treating of the soybean plants shall take place at the vegetative growth stages V3 to V4 (meaning 4 to 4 fully expanded trifoliate leaves) onwards to the reproductive growth stage R2 (full bloom), more preferably place at the vegetative growth stages V6 to V8 (meaning 6 to 8 fully expanded trifoliate leaves) onwards to the reproductive growth stage R3 (beginning bloom). Depending on the disease pressure, two to four and under extreme conditions up to five applications may be necessary at application intervals of 14 to 28 days.

When employed as foliar spray against soybean rust, the amounts of the compounds I applied are, depending on the specific compound used and on the disease pressure, from 5 g to 500 g per ha, preferably from 10 to 200 per ha, more preferably from 15 to 150 g per ha, and in particular from 30 to 125 g per ha.

Furthermore, the present invention relates to the use of compounds of formula I as defined herein for combating phytopathogenic fungi containing an amino acid substitution F129L in the mitochondrial cytochrome b protein conferring resistance to Qo inhibitors.

The mutation F129L in the cytochrome b (cytb, also referred to as cob) gene shall mean any substitution of nucleotides of codon 129 encoding "F" (phenylalanine; e.g. TTT or TTC) that leads to a codon encoding "L" (leucine; e.g. TTA, TTG, TTG, CTT, CTC, CTA or CTG), for example the substitution of the first nucleotide of codon 129 'T' to 'C' (TTT to CTT), in the cytochrome b gene resulting in a single amino acid substitution in the position 129 from F (phenylalanine) to L (leucine) (F129L) in the cytochrome b protein (Cytb). In the present invention, the mutation F129L in the cytochrome b gene shall be understood to be a single amino acid substitution in the position 129 from F (phenylalanine) to L (leucine) (F129L) in the cytochrome b protein.

Many other phytopathogenic fungi acquired the F129L mutation in the cytochrome b gene conferring resistance to Qo inhibitors, such as rusts, in particular soybean rust (*Phakopsora pachyrhizi* and *Phakopsora meibromiae*) as well as fungi from the genera Alternaria, Pyrenophora and Rhizoctonia.

Preferred fungal species are *Alternaria solani, Phakopsora pachyrhizi, Phakopsora meibromiae, Pyrenophora teres, Pyrenophora tritici-repentis* and *Rhizoctonia solani;* in particular *Phakopsora pachyrhizi.*

In one aspect, the present invention relates to the method of protecting plants susceptible to and/or under attack by phytopathogenic fungi containing an amino acid substitution F129L in the mitochondrial cytochrome b protein conferring resistance to Qo inhibitors, which method comprises applying to said plants, treating plant propagation material of said plants with, and/or applying to said phytopathogenic fungi, at least one compound of formula I or a composition comprising at least one compound of formula I.

According to another embodiment, the method for combating phytopathogenic fungi, comprises: a) identifying the phytopathogenic fungi containing an amino acid substitution F129L in the mitochondrial cytochrome b protein conferring resistance to Qo inhibitors, or the materials, plants, the soil or seeds that are at risk of being diseased from phytopathogenic fungi as defined herein, and b) treating said fungi or the materials, plants, the soil or plant propagation material with an effective amount of at least one compound of formula I, or a composition comprising it thereof.

The term "phytopathogenic fungi an amino acid substitution F129L in the mitochondrial cytochrome b protein conferring resistance to Qo inhibitors" is to be understood that at least 10% of the fungal isolates to be controlled contain a such F129L substitution in the mitochondrial cytochrome b protein conferring resistance to Qo inhibitors, preferably at least 30%, more preferably at least 50%, even more preferably at at least 75% of the fungi, most preferably between 90 and 100%; in particular between 95 and 100%.

The compounds I and compositions thereof, respectively, are also suitable for controlling harmful microorganisms in the protection of stored products or harvest, and in the protection of materials.

When used in the protection of materials or stored products, the amount of active substance applied depends on the kind of application area and on the desired effect. Amounts customarily applied in the protection of materials are 0.001 g to 2 kg, preferably 0.005 g to 1 kg, of active substance per cubic meter of treated material.

The compounds I and compositions thereof, respectively, may be used for improving the health of a plant. The invention also relates to a method for improving plant health by treating a plant, its propagation material, and/or the locus where the plant is growing or is to grow with an effective amount of compounds I and compositions thereof, respectively.

The compounds I are employed as such or in form of compositions by treating the fungi, the plants, plant propagation materials, such as seeds; soil, surfaces, materials, or rooms to be protected from fungal attack with a fungicidally effective amount of the active substances. The application can be carried out both before and after the infection of the plants, plant propagation materials, such as seeds; soil, surfaces, materials or rooms by the fungi.

Plant propagation materials may be treated with compounds I as such or a composition comprising at least one compound I prophylactically either at or before planting or transplanting.

The invention also relates to agrochemical compositions comprising an auxiliary and at least one compound I.

When employed in plant protection, the amounts of active substances applied are, depending on the kind of effect desired, from 0.001 to 2 kg per ha, preferably from 0.005 to 2 kg per ha, more preferably from 0.05 to 0.9 kg per ha, even more preferably from 0.075 to 0.75 kg per ha, and in particular from 0.1 to 0.3 kg per ha.

In treatment of plant propagation materials, such as seeds, e. g. by dusting, coating, or drenching, amounts of active substance of generally from 0.1 to 1000 g, preferably from 1 to 1000 g, more preferably from 1 to 100 g and most preferably from 5 to 100 g, per 100 kg of plant propagation material (preferably seeds) are required.

An agrochemical composition comprises a fungicidally effective amount of a compound I. The term "fungicidally effective amount" denotes an amount of the composition or of the compounds I, which is sufficient for controlling phytopathogenic fungi on cultivated plants or in the protection of stored products or harvest or of materials and which does not result in a substantial damage to the treated plants, the treated stored products or harvest, or to the treated materials. Such an amount can vary in a broad range and is dependent on various factors, such as the fungal species to be controlled, the treated cultivated plant, stored product, harvest or material, the climatic conditions and the specific compound I used.

The user applies the agrochemical composition usually from a predosage device, a knapsack sprayer, a spray tank, a spray plane, or an irrigation system. Usually, the agrochemical composition is made up with water, buffer, and/or further auxiliaries to the desired application concentration and the ready-to-use spray liquor or the agrochemical composition according to the invention is thus obtained. Usually, 20 to 2000 liters, preferably 50 to 400 liters, of the ready-to-use spray liquor are applied per hectare of agricultural useful area.

The compounds I, their N-oxides and salts can be converted into customary types of agrochemical compositions, e. g. solutions, emulsions, suspensions, dusts, powders, pastes, granules, pressings, capsules, and mixtures thereof. Examples for composition types (see also "Catalogue of pesticide formulation types and international coding system", Technical Monograph No. 2, 6^{th} Ed. May 2008, CropLife International) are suspensions (e. g. SC, OD, FS), emulsifiable concentrates (e. g. EC), emulsions (e. g. EW, EO, ES, ME), capsules (e. g. CS, ZC), pastes, pastilles, wettable powders or dusts (e. g. WP, SP, WS, DP, DS), pressings (e. g. BR, TB, DT), granules (e. g. WG, SG, GR, FG, GG, MG), insecticidal articles (e. g. LN), as well as gel formulations for the treatment of plant propagation materials, such as seeds (e. g. GF). The compositions are prepared in a known manner, such as described by Mollet and Grubemann, Formulation technology, Wiley VCH, Weinheim, 2001; or by Knowles, New developments in crop protection product formulation, Agrow Reports DS243, T&F Informa, London, 2005.

Suitable auxiliaries are solvents, liquid carriers, solid carriers or fillers, surfactants, dispersants, emulsifiers, wetters, adjuvants, solubilizers, penetration enhancers, protective colloids, adhesion agents, thickeners, humectants, repellents, attractants, feeding stimulants, compatibilizers, bactericides, anti-freezing agents, anti-foaming agents, colorants, tackifiers, and binders.

The agrochemical compositions generally comprise between 0.01 and 95 %, preferably between 0.1 and 90 %, more preferably between 1 and 70 %, and in particular between 10 and 60 %, by weight of active substances (e.g. at least one compound I). The agrochemical compositions generally comprise between 5 and 99.9 %, preferably between 10 and 99.9 %, more preferably between 30 and 99 %, and in particular between 40 and 90 %, by weight of at least one auxiliary. The active substances (e.g. compounds I) are employed in a purity of from 90 % to 100 %, preferably from 95-% to 100 % (according to NMR spectrum).

For the purposes of treatment of plant propagation materials, particularly seeds, solutions for seed treatment (LS), suspoemulsions (SE), flowable concentrates (FS), powders for dry treatment (DS), water-dispersible powders for slurry treatment (WS), water-soluble powders (SS), emulsions (ES), emulsifiable concentrates (EC), and gels (GF) are usually employed. The compositions in question give, after two-to-tenfold dilution, active substance concentrations of from 0.01 to 60 % by weight, preferably from 0.1 to 40 %, in the ready-to-use preparations. Application can be carried out before or during sowing. Methods for applying compound I and compositions thereof, respectively, onto plant propagation material, especially seeds, include dressing, coating, pelleting, dusting, soaking, as well as in-furrow application methods. Preferably, compound I or the compositions thereof, respectively, are applied on to the plant propagation material by a method such that germination is not induced, e. g. by seed dressing, pelleting, coating, and dusting.

Various types of oils, wetters, adjuvants, fertilizers, or micronutrients, and further pesticides (e. g. fungicides, growth regulators, herbicides, insecticides, safeners) may be added to the compounds I or the compositions thereof as premix, or, not until immediately prior to use (tank mix). These agents can be admixed with the compositions according to the invention in a weight ratio of 1:100 to 100:1, preferably 1:10 to 10:1.

Mixing the compounds I or the compositions comprising them in the use form as fungicides with other fungicides results in many cases in an expansion of the fungicidal spectrum of activity or in a prevention of fungicide resistance development. Furthermore, in many cases, synergistic effects are obtained (synergistic mixtures).

The following list of pesticides II, in conjunction with which the compounds I can be used, is intended to illustrate the possible combinations but does not limit them:
A) Respiration inhibitors
   - Inhibitors of complex III at Qₒ site: azoxystrobin (A.1.1), coumethoxystrobin (A.1.2), coumoxystrobin (A.1.3), dimoxystrobin (A.1.4), enestroburin (A.1.5), fenaminstrobin (A.1.6), fenoxystrobin/flufenoxystrobin (A.1.7), fluoxastrobin (A.1.8), kresoxim-methyl (A.1.9), mandestrobin (A.1.10), metominostrobin (A.1.11), orysastrobin (A.1.12), picoxystrobin (A.1.13), pyraclostrobin (A.1.14), pyrametostrobin (A.1.15), pyraoxystrobin (A.1.16), trifloxy-strobin (A.1.17), 2-(2-(3-(2,6-dichlorophenyl)-1-methyl-allylideneaminooxymethyl)-phenyl)-2-methoxyimino-*N*-methyl-acetamide (A.1.18), pyribencarb (A.1.19), triclopyricarb/chloro-dincarb (A.1.20), famoxadone (A.1.21), fenamidone (A.1.21), methyl-*N*-[2-[(1,4-dimethyl-5-phenyl-pyrazol-3-yl)oxylmethyl]phenyl]-*N*-methoxy-carbamate (A.1.22), metyltetraprole (A.1.25), (*Z*,2*E*)-5-[1-(2,4-dichlorophenyl)pyrazol-3-yl]-oxy-2-methoxyimino-*N*,3-dimethyl-pent-3-enamide (A.1.34), (*Z*,2*E*)-5-[1-(4-chlorophenyl)pyrazol-3-yl]oxy-2-methoxyimino-*N*,3-dimethyl-pent-3-enamide (A.1.35), pyriminostrobin (A.1.36), bifujunzhi (A.1.37), 2-(ortho-((2,5-dimethylphenyl-oxymethylen)phenyl)-3-methoxy-acrylic acid methylester (A.1.38);
      - inhibitors of complex III at Qᵢ site: cyazofamid (A.2.1), amisulbrom (A.2.2), [(6*S*,7*R*,8*R*)-8-benzyl-3-[(3-hydroxy-4-methoxy-pyridine-2-carbonyl)amino]-6-methyl-4,9-di-oxo-1,5-dioxonan-7-yl] 2-methylpropanoate (A.2.3), fenpicoxamid (A.2.4), florylpicoxamid (A.2.5), metarylpicoxamid (A.2.6);
      - inhibitors of complex II: benodanil (A.3.1), benzovindiflupyr (A.3.2), bixafen (A.3.3), boscalid (A.3.4), carboxin (A.3.5), fenfuram (A.3.6), fluopyram (A.3.7), flutolanil (A.3.8), fluxapyroxad (A.3.9), furametpyr (A.3.10), isofetamid (A.3.11), isopyrazam (A.3.12), mepronil (A.3.13), oxycarboxin (A.3.14), penflufen (A.3.15), penthiopyrad (A.3.16), pydiflumetofen (A.3.17), pyraziflumid (A.3.18), sedaxane (A.3.19), tecloftalam (A.3.20), thifluzamide (A.3.21), inpyrfluxam (A.3.22), pyrapropoyne (A.3.23), fluindapyr (A.3.28), N-[2-[2-chloro-4-(trifluoromethyl)phenoxy]phenyl]-3-(difluoromethyl)-5-fluoro-1-methyl-pyrazole-4-carboxamide (A.3.29), methyl (*E*)-2-[2-[(5-cyano-2-methyl-phenoxy)methyl]phenyl]-3-methoxy-prop-2-enoate (A.3.30), isoflucypram (A.3.31), 2-(difluoromethyl)-*N*-(1,1,3-trimethyl-indan-4-yl)-pyridine-3-carboxamide (A.3.32), 2-(difluoromethyl)-*N*-[(3R)-1,1,3-trimethylindan-4-yl]-pyridine-3-carboxamide (A.3.33), 2-(difluoromethyl)-*N*-(3-ethyl-1,1-dimethyl-indan-4-yl)-pyridine-3-carboxamide (A.3.34), 2-(difluoromethyl)-*N*-[(3R)-3-ethyl-1,1-dimethyl-indan-4-yl]-pyridine-3-carboxamide (A.3.35), 2-(difluoromethyl)-*N*-(1,1-dimethyl-3-propyl-indan-4-yl)pyridine-3-carboxamide (A.3.36), 2-(difluoromethyl)-*N*-[(3R)-1,1-dimethyl-3-propyl-indan-4-yl]-pyridine-3-carboxamide (A.3.37), 2-(difluoromethyl)-*N*-(3-isobutyl-1,1-dimethyl-indan-4-yl)-pyridine-3-carboxamide (A.3.38), 2-(difluoromethyl)-*N*-[(3R)-3-isobutyl-1,1-dimethyl-indan-4-yl]pyridine-3-carboxamide (A.3.39) cyclobutrifluram (A.3.24);
      - other respiration inhibitors: diflumetorim (A.4.1); nitrophenyl derivates: binapacryl (A.4.2), dinobuton (A.4.3), dinocap (A.4.4), fluazinam (A.4.5), meptyldinocap (A.4.6), ferimzone (A.4.7); organometal compounds: fentin salts, e. g. fentin-acetate (A.4.8), fentin chloride (A.4.9) or fentin hydroxide (A.4.10); ametoctradin (A.4.11); silthiofam (A.4.12);
B) Sterol biosynthesis inhibitors (SBI fungicides)
   - C14 demethylase inhibitors: triazoles: azaconazole (B.1.1), bitertanol (B.1.2), bromuconazole (B.1.3), cyproconazole (B.1.4), difenoconazole (B.1.5), diniconazole (B.1.6), diniconazole-M (B.1.7), epoxiconazole (B.1.8), fenbuconazole (B.1.9), fluquinconazole (B.1.10), flusilazole (B.1.11), flutriafol (B.1.12), hexaconazole (B.1.13), imibenconazole (B.1.14), ipconazole (B.1.15), metconazole (B.1.17), myclobutanil (B.1.18), oxpoconazole (B.1.19), paclobutrazole (B.1.20), penconazole (B.1.21), propiconazole (B.1.22), prothioconazole (B.1.23), simeconazole (B.1.24), tebuconazole (B.1.25), tetraconazole (B.1.26), triadimefon (B.1.27), triadimenol (B.1.28), triticonazole (B.1.29), uniconazole (B.1.30), 2-(2,4-difluorophenyl)-1,1-difluoro-3-(tetrazol-1-yl)-1-[5-[4-(2,2,2-trifluoroethoxy)phenyl]-2-pyridyl]propan-2-ol (B.1.31), 2-(2,4-difluorophenyl)-1,1-difluoro-3-(tetrazol-1-yl)-1-[5-[4-(tri-fluoromethoxy)phenyl]-2-pyridyl]propan-2-ol (B.1.32), fluoxytioconazole (B.1.33), ipfentrifluconazole (B.1.37), mefentrifluconazole (B.1.38), (2*R*)-2-[4-(4-chlorophenoxy)-2-(trifluoromethyl)phenyl]-1-(1,2,4-triazol-1-yl)propan-2-ol, (2*S*)-2-[4-(4-chlorophenoxy)-2-(trifluoromethyl)phenyl]-1-(1,2,4-triazol-1-yl)propan-2-ol, 2-(chloromethyl)-2-methyl-5-(*p*-tolylmethyl)-1-(1,2,4-triazol-1-ylmethyl)cyclopentanol (B.1.43); imidazoles: imazalil (B.1.44), pefurazoate (B.1.45), prochloraz (B.1.46), triflumizol (B.1.47); pyrimidines, pyridines, piperazines: fenarimol (B.1.49), pyrifenox (B.1.50), triforine (B.1.51), [3-(4-chloro-2-fluoro-phenyl)-5-(2,4-difluorophenyl)isoxazol-4-yl]-(3-pyridyl)methanol (B.1.52), 4-[[6-[2-(2,4-difluorophenyl)-1,1-difluoro-2-hydroxy-3-(1,2,4-triazol-1-yl)propyl]-3-pyridyl]oxy]benzonitrile (B.1.53), 2-[6-(4-bromo-phenoxy)-2-(trifluoromethyl)-3-pyridyl]-1-(1,2,4-triazol-1-yl)propan-2-ol (B.1.54), 2-[6-(4-chlorophenoxy)-2-(trifluoromethyl)-3-pyridyl]-1-(1,2,4-triazol-1-yl)propan-2-ol (B.1.55);
   - Delta14-reductase inhibitors: aldimorph (B.2.1), dodemorph (B.2.2), dodemorph-acetate (B.2.3), fenpropimorph (B.2.4), tridemorph (B.2.5), fenpropidin (B.2.6), piperalin (B.2.7), spiroxamine (B.2.8);
   - Inhibitors of 3-keto reductase: fenhexamid (B.3.1);
   - Other Sterol biosynthesis inhibitors: chlorphenomizole (B.4.1);
C) Nucleic acid synthesis inhibitors
   - phenylamides or acyl amino acid fungicides: benalaxyl (C.1.1), benalaxyl-M (C.1.2), kiralaxyl (C.1.3), metalaxyl (C.1.4), metalaxyl-M (C.1.5), ofurace (C.1.6), oxadixyl (C.1.7);
   - other nucleic acid synthesis inhibitors: hymexazole (C.2.1), octhilinone (C.2.2), oxolinic acid (C.2.3), bupirimate (C.2.4), 5-fluorocytosine (C.2.5), 5-fluoro-2-(p-tolylmethoxy)pyrimidin-4-amine (C.2.6), 5-fluoro-2-(4-fluorophenylmethoxy)pyrimidin-4-amine (C.2.7), 5-fluoro-2-(4-chlorophenylmethoxy)pyrimidin-4 amine (C.2.8);
D) Inhibitors of cell division and cytoskeleton
   - tubulin inhibitors: benomyl (D.1.1), carbendazim (D.1.2), fuberidazole (D1.3), thiabendazole (D.1.4), thiophanate-methyl (D.1.5), pyridachlometyl (D.1.6), *N*-ethyl-2-[(3-ethynyl-8-methyl-6-quinolyl)oxy]butanamide (D.1.8), *N*-ethyl-2-[(3-ethynyl-8-methyl-6-quinolyl)oxy]-2-methyl-sulfanyl-acetamide (D.1.9), 2-[(3-ethynyl-8-methyl-6-quinolyl)oxy]-*N*-(2-fluoroethyl)butanamide (D.1.10), 2-[(3-ethynyl-8-methyl-6-quinolyl)oxy]-*N*-(2-fluoroethyl)-2-methoxy-acetamide (D.1.11), 2-[(3-ethynyl-8-methyl-6-quinolyl)oxy]-*N*-propyl-butanamide (D.1.12), 2-[(3-ethynyl-8-methyl-6-quinolyl)oxy]-2-methoxy-*N*-propyl-acetamide (D.1.13), 2-[(3-ethynyl-8-methyl-6-quinolyl)oxy]-2-methylsulfanyl-*N*-propyl-acetamide (D.1.14), 2-[(3-ethynyl-8-methyl-6-quinolyl)oxy]-*N*-(2-fluoroethyl)-2-methylsulfanyl-acetamide (D.1.15), 4-(2-bromo-4-fluorophenyl)-*N*-(2-chloro-6-fluoro-phenyl)-2,5-dimethyl-pyrazol-3-amine (D.1.16);
   - other cell division inhibitors: diethofencarb (D.2.1), ethaboxam (D.2.2), pencycuron (D.2.3), fluopicolide (D.2.4), zoxamide (D.2.5), metrafenone (D.2.6), pyriofenone (D.2.7), phenamacril (D.2.8);
E) Inhibitors of amino acid and protein synthesis
   - methionine synthesis inhibitors: cyprodinil (E.1.1), mepanipyrim (E.1.2), pyrimethanil (E.1.3);
   - protein synthesis inhibitors: blasticidin-S (E.2.1), kasugamycin (E.2.2), kasugamycin hydrochloride-hydrate (E.2.3), mildiomycin (E.2.4), streptomycin (E.2.5), oxytetracyclin (E.2.6);
F) Signal transduction inhibitors
   - MAP / histidine kinase inhibitors: fluoroimid (F.1.1), iprodione (F.1.2), procymidone (F.1.3), vinclozolin (F.1.4), fludioxonil (F.1.5);
   - G protein inhibitors: quinoxyfen (F.2.1);
G) Lipid and membrane synthesis inhibitors
   - Phospholipid biosynthesis inhibitors: edifenphos (G.1.1), iprobenfos (G.1.2), pyrazophos (G.1.3), isoprothiolane (G.1.4);
   - lipid peroxidation: dicloran (G.2.1), quintozene (G.2.2), tecnazene (G.2.3), tolclofos-methyl (G.2.4), biphenyl (G.2.5), chloroneb (G.2.6), etridiazole (G.2.7), zinc thiazole (G.2.8);
   - phospholipid biosynthesis and cell wall deposition: dimethomorph (G.3.1), flumorph (G.3.2), mandipropamid (G.3.3), pyrimorph (G.3.4), benthiavalicarb (G.3.5), iprovalicarb (G.3.6), valifenalate (G.3.7);
   - compounds affecting cell membrane permeability and fatty acides: propamocarb (G.4.1);
   - inhibitors of oxysterol binding protein: oxathiapiprolin (G.5.1), fluoxapiprolin (G.5.3), 4-[1-[2-[3-(difluoromethyl)-5-methyl-pyrazol-1-yl]acetyl]-4-piperidyl]-*N*-tetralin-1-yl-pyridine-2-carboxamide (G.5.4), 4-[1-[2-[3,5-bis(difluoromethyl)pyrazol-1-yl]acetyl]-4-piperidyl]-*N*-tetralin-1-yl-pyridine-2-carboxamide (G.5.5), 4-[1-[2-[3-(difluoromethyl)-5-(trifluoromethyl)pyrazol-1-yl]acetyl]-4-piperidyl]-*N*-tetralin-1-yl-pyridine-2-carboxamide (G.5.6), 4-[1-[2-[5-cyclopropyl-3-(difluoromethyl)pyrazol-1-yl]acetyl]-4-piperidyl]-*N*-tetralin-1-yl-pyridine-2-carboxamide (G.5.7), 4-[1-[2-[5-methyl-3-(trifluoromethyl)pyrazol-1-yl]acetyl]-4-piperidyl]-*N*-tetralin-1-yl-pyridine-2-carboxamide (G.5.8), 4-[1-[2-[5-(difluoromethyl)-3-(trifluoromethyl)pyrazol-1-yl]acetyl]-4-piperidyl]-*N*-tetralin-1-yl-pyridine-2-carboxamide (G.5.9), 4-[1-[2-[3,5-bis(trifluoromethyl)pyrazol-1-yl]acetyl]-4-piperidyl]-*N*-tetralin-1-yl-pyridine-2-carboxamide (G.5.10), (4-[1-[2-[5-cyclopropyl-3-(trifluoromethyl)pyrazol-1-yl]acetyl]-4-piperidyl]-*N*-tetralin-1-yl-pyridine-2-carboxamide (G.5.11);
H) Inhibitors with Multi Site Action
   - inorganic active substances: Bordeaux mixture (H.1.1), copper (H.1.2), copper acetate (H.1.3), copper hydroxide (H.1.4), copper oxychloride (H.1.5), basic copper sulfate (H.1.6), sulfur (H.1.7);
   - thio- and dithiocarbamates: ferbam (H.2.1), mancozeb (H.2.2), maneb (H.2.3), metam (H.2.4), metiram (H.2.5), propineb (H.2.6), thiram (H.2.7), zineb (H.2.8), ziram (H.2.9);
   - organochlorine compounds: anilazine (H.3.1), chlorothalonil (H.3.2), captafol (H.3.3), captan (H.3.4), folpet (H.3.5), dichlofluanid (H.3.6), dichlorophen (H.3.7), hexachlorobenzene (H.3.8), pentachlorphenole (H.3.9) and its salts, phthalide (H.3.10), tolylfluanid (H.3.11);
   - guanidines and others: guanidine (H.4.1), dodine (H.4.2), dodine free base (H.4.3), guazatine (H.4.4), guazatine-acetate (H.4.5), iminoctadine (H.4.6), iminoctadine-triacetate (H.4.7), iminoctadine-tris(albesilate) (H.4.8), dithianon (H.4.9), 2,6-dimethyl-1*H*,5*H*-[1,4]dithiino[2,3-c:5,6-c']dipyrrole-1,3,5,7(2*H*,6*H*)-tetraone (H.4.10);
I) Cell wall synthesis inhibitors
   - inhibitors of glucan synthesis: validamycin (1.1.1), polyoxin B (1.1.2);
   - melanin synthesis inhibitors: pyroquilon (1.2.1), tricyclazole (1.2.2), carpropamid (1.2.3), dicyclomet (1.2.4), fenoxanil (1.2.5);
J) Plant defence inducers
   - acibenzolar-S-methyl (J.1.1), probenazole (J.1.2), isotianil (J.1.3), tiadinil (J.1.4), prohexadione-calcium (J.1.5); phosphonates: fosetyl (J.1.6), fosetyl-aluminum (J.1.7), phosphorous acid and its salts (J.1.8), calcium phosphonate (J.1.11), potassium phosphonate (J.1.12), potassium or sodium bicarbonate (J.1.9), 4-cyclopropyl-*N*-(2,4-dimethoxyphenyl)thiadiazole-5-carboxamide (J.1.10);
K) Unknown mode of action
   - bronopol (K.1.1), chinomethionat (K.1.2), cyflufenamid (K.1.3), cymoxanil (K.1.4), dazomet (K.1.5), debacarb (K.1.6), diclocymet (K.1.7), diclomezine (K.1.8), difenzoquat (K.1.9), difenzoquat-methylsulfate (K.1.10), diphenylamin (K.1.11), fenitropan (K.1.12), fenpyrazamine (K.1.13), flumetover (K.1.14), flumetylsulforim (K.1.60), flusulfamide (K1.15), flutianil (K.1.16), harpin (K.1.17), methasulfocarb (K.1.18), nitrapyrin (K.1.19), nitrothal-isopropyl (K.1.20), tolprocarb (K.1.21), oxin-copper (K.1.22), proquinazid (K.1.23), seboctylamine (K.1.61), tebufloquin (K.1.24), tecloftalam (K.1.25), triazoxide (K.1.26), *N*'-(4-(4-chloro-3-trifluoromethyl-phenoxy)-2,5-dimethyl-phenyl)-*N*-ethyl-*N*-methyl formamidine (K.1.27), *N*'-(4-(4-fluoro-3-trifluoromethyl-phenoxy)-2,5-dimethyl-phenyl)-*N*-ethyl-*N*-methyl formamidine (K.1.28), *N*'-[4-[[3-[(4-chlorophenyl)methyl]-1,2,4-thiadiazol-5-yl]oxy]-2,5-dimethyl-phenyl]-*N-*ethyl-*N*-methyl-formamidine (K.1.29), N'-(5-bromo-6-indan-2-yloxy-2-methyl-3-pyridyl)-*N-*ethyl-*N*-methyl-formamidine (K.1.30), *N*'-[5-bromo-6-[1-(3,5-difluorophenyl)ethoxy]-2-methyl-3-pyridyl]-*N*-ethyl-*N*-methyl-formamidine (K.1.31), *N*'-[5-bromo-6-(4-isopropylcyclohexoxy)-2-methyl-3-pyridyl]-*N*-ethyl-*N*-methyl-formamidine (K.1.32), *N*'-[5-bromo-2-methyl-6-(1-phenylethoxy)-3-pyridyl]-*N*-ethyl-*N*-methyl-formamidine (K.1.33), *N*'-(2-methyl-5-trifluoromethyl-4-(3-trimethylsilanyl-propoxy)-phenyl)-*N*-ethyl-*N*-methyl formamidine (K.1.34), *N*'-(5-difluoromethyl-2-methyl-4-(3-trimethylsilanyl-propoxy)-phenyl)-*N*-ethyl-*N*-methyl formamidine (K.1.35), 2-(4-chloro-phenyl)-*N*-[4-(3,4-dimethoxy-phenyl)-isoxazol-5-yl]-2-prop-2-ynyloxy-acetamide (K.1.36), 3-[5-(4-chloro-phenyl)-2,3-dimethyl-isoxazolidin-3-yl]-pyridine (pyrisoxazole) (K.1.37), 3-[5-(4-methylphenyl)-2,3-dimethyl-isoxazolidin-3-yl]-pyridine (K.1.38), 5-chloro-1-(4,6-dimethoxy-pyrimidin-2-yl)-2-methyl-1*H*-benzoimidazole (K.1.39), ethyl (*Z*)-3-amino-2-cyano-3-phenyl-prop-2-enoate (K.1.40), picarbutrazox (K.1.41), pentyl *N*-[6-[[(*Z*)-[(1-methyltetrazol-5-yl)-phenyl-methylene]amino]oxymethyl]-2-pyridyl]carbamate (K.1.42), but-3-ynyl *N*-[6-[[(*Z*)-[(1-methyltetrazol-5-yl)-phenyl-methylene]amino]oxymethyl]-2-pyridyl]carbamate (K.1.43), ipflufenoquin (K.1.44), quinofumelin (K.1.47), benziothiazolinone (K.1.48), bromothalonil (K.1.49), 2-(6-benzyl-2-pyridyl)quinazoline (K.1.50), 2-[6-(3-fluoro-4-methoxy-phenyl)-5-methyl-2-pyridyl]quinazoline (K.1.51), dichlobentiazox (K.1.52), *N*'-(2,5-dimethyl-4-phenoxy-phenyl)-*N*-ethyl-*N*-methyl-formamidine (K.1.53), aminopyrifen (K.1.54), fluopimomide (K.1.55), *N*'-[5-bromo-2-methyl-6-(1-methyl-2-propoxy-ethoxy)-3-pyridyl]-N-ethyl-*N*-methyl-formamidine (K.1.56), *N*'-[4-(4,5-dichlorothiazol-2-yl)oxy-2,5-dimethylphenyl]-*N*-ethyl-*N*-methyl-formamidine (K.1.57), flufenoxadiazam (K.1.58), *N*-methyl-4-[5-(trifluoromethyl)-1,2,4-oxadiazol-3-yl]benzenecarbothioamide (K.1.59), *N*-methoxy-*N*-[[4-[5-(trifluoromethyl)-1,2,4-oxadiazol-3-yl]phenyl]methyl]cyclopropanecarboxamide (K.1.60; WO2018/177894, WO 2020/212513), *N*-((4-[5-(trifluoromethyl)-1,2,4-oxadiazol-3-yl]phenyl)methyl)propanamide (K.1.62), 3,3,3-trifluoro-*N*-[[3-fluoro-4-[5-(trifluoromethyl)-1,2,4-oxadiazol-3-yl]phenyl]methyl]propanamide (K.1.63), 3,3,3-trifluoro-*N*-[[2-fluoro-4-[5-(trifluoromethyl)-1,2,4-oxadiazol-3-yl]phenyl]methyl]propanamide (K.1.64), *N*-[2,3-difluoro-4-[5-(trifluoromethyl)-1,2,4-oxadiazol-3-yl]benzyl]butanamide (K.1.65), *N*-[[2,3-difluoro-4-[5-(trifluoromethyl)-1,2,4-oxadiazol-3-yl]phenyl]methyl]-3,3,3-trifluoro-propanamide (K.1.66), 1-methoxy-1-methyl-3-[[4-[5-(trifluoromethyl)-1,2,4-oxadiazol-3-yl]phenyl]methyl]-urea (K.1.67), 1,1-diethyl-3-[[4-[5-[trifluoromethyl]-1,2,4-oxadiazol-3-yl]phenyl]methyl]urea (K.1.68), *N*,2-dimethoxy-*N*-[[4-[5-(trifluoromethyl)-1,2,4-oxadiazol-3-yl]phenyl]methyl]propan-amide (K.1.69), *N*-ethyl-2-methyl-*N*-[[4-[5-(trifluoromethyl)-1,2,4-oxadiazol-3-yl]phenyl]methyl]propanamide (K.1.70), 1-methoxy-3-methyl-1-[[4-[5-(trifluoromethyl)-1,2,4-oxadiazol-3-yl]-phenyl]methyl]urea (K.1.71), 1-[[4-[5-(trifluoromethyl)-1,2,4-oxadiazol-3-yl]phenyl]methyl]pyrrolidin-2-one (K.1.72), 1-[[4-[5-(trifluoromethyl)-1,2,4-oxadiazol-3-yl]phenyl]methyl]piperidin-2-one (K.1.73), 4-[[4-[5-(trifluoromethyl)-1,2,4-oxadiazol-3-yl]phenyl]methyl]morpholin-3-one (K.1.74), 4,4-dimethyl-2-[[4-[5-(trifluoromethyl)-1,2,4-oxadiazol-3-yl]phenyl]methyl]isoxazolidin-3-one (K.1.75), 2-[[4-[5-(trifluoromethyl)-1,2,4-oxadiazol-3-yl]phenyl]methyl]isoxazolidin-3-one (K.1.76), 5,5-dimethyl-2-[[4-[5-(trifluoromethyl)-1,2,4-oxadiazol-3-yl]phenyl]methyl]-isoxazolidin-3-one (K.1.77), 3,3-dimethyl-1-[[4-[5-(trifluoromethyl)-1,2,4-oxadiazol-3-yl]phenyl]methyl]piperidin-2-one (K.1.78), 2-[[4-[5-(trifluoromethyl)-1,2,4-oxadiazol-3-yl]phenyl]methyl]oxazinan-3-one (K.1.79), 1-[[3-fluoro-4-[5-(trifluoromethyl)-1,2,4-oxadiazol-3-yl]phenyl]-methyl]azepan-2-one (K.1.80), 4,4-dimethyl-1-[[4-[5-(trifluoromethyl)-1,2,4-oxadiazol-3-yl]-phenyl]methyl]pyrrolidin-2-one (K.1.81), 5-methyl-1-[[4-[5-(trifluoromethyl)-1,2,4-oxadiazol-3-yl]phenyl]methyl]pyrrolidin-2-one (K.1.82), ethyl 1-[[4-[5-(trifluoromethyl)-1,2,4-oxadiazol-3-yl]phenyl]methyl]pyrazole-4-carboxylate (K.1.83), *N*-methyl-1-[[4-[5-(trifluoromethyl)-1,2,4-oxadiazol-3-yl]phenyl]methyl]pyrazole-4-carboxamide (K.1.84), *N*,*N*-dimethyl-1-[4-[5-(trifluoromethyl)-1,2,4-oxadiazol-3-yl]benzyl]-1H-1,2,4-triazol-3-amine (K.1.85), *N*-methoxy-*N*-methyl-1-[[4-[5-(trifluoromethyl)-1,2,4-oxadiazol-3-yl]phenyl]methyl]pyrazole-4-carboxamide (K.1.86), propyl-1-[[4-[5-(trifluoromethyl)-1,2,4-oxadiazol-3-yl]phenyl]methyl]-pyrazole-4-carboxamide (K.1.87), *N*-methoxy-1-[[4-[5-(trifluoromethyl)-1,2,4-oxadiazol-3-yl]phenyl]methyl]pyrazole-4-carboxamide (K.1.88), *N*-allyl-*N*-[[4-[5-(trifluoromethyl)-1,2,4-oxadiazol-3-yl]phenyl]methyl]propanamide (K.1.89), 3-ethyl-1-methoxy-1-[[4-[5-(trifluoromethyl)-1,2,4-oxadiazol-3-yl]phenyl]methyl]urea (K.1.90), 1,3-dimethoxy-1-[[4-[5-(trifluoromethyl)-1,2,4-oxadiazol-3-yl]phenyl]methyl]urea (K.1.91), *N*-allyl-*N*-[[4-[5-(trifluoromethyl)-1,2,4-oxadiazol-3-yl]phenyl]methyl]acetamide (K.1.92), *N*-[4-[5-(trifluoromethyl)-1,2,4-oxadiazol-3-yl]benzyl]cyclopropanecarboxamide (K.1.93), 1-methyl-3-[[4-[5-(trifluoromethyl)-1,2,4-oxadiazol-3-yl]phenyl]methyl]urea (K.1.94), *N*'-[2-chloro-4-(2-fluorophenoxy)-5-methyl-phenyl]-*N*-ethyl-*N*-methyl-formamidine (K.1.95), *N*'-[2-chloro-4-[(4-methoxyphenyl)methyl]-5-methyl-phenyl]-*N*-ethyl-*N*-methyl-formamidine (K.1.96), *N*'-[2-chloro-4-[(4-cyano-phenyl)methyl]-5-methyl-phenyl]-*N*-ethyl-*N*-methyl-formamidine (K.1.97), *N*'-[2,5-dimethyl-4-(o-tolylmethyl)phenyl]-*N*-ethyl-*N*-methyl-formamidine (K.1.98).

The active substances referred to as component 2, their preparation and their activity e. g. against harmful fungi is known (cf.: http://www.alanwood.netipesticides/); these substances are commercially available. The compounds described by IUPAC nomenclature, their preparation and their pesticidal activity are also known.

In the binary mixtures the weight ratio of the component 1) and the component 2) generally depends from the properties of the components used, usually it is in the range of from 1:10,000 to 10,000:1, often from 1:100 to 100:1, regularly from 1:50 to 50:1, preferably from 1:20 to 20:1, more preferably from 1:10 to 10:1, even more preferably from 1:4 to 4:1 and in particular from 1:2 to 2:1. According to further embodiments, the weight ratio of the component 1) and the component 2) usually is in the range of from 1000:1 to 1:1, often from 100: 1 to 1:1, regularly from 50:1 to 1:1, preferably from 20:1 to 1:1, more preferably from 10:1 to 1:1, even more preferably from 4:1 to 1:1 and in particular from 2:1 to 1:1. According to further embodiments, the weight ratio of the component 1) and the component 2) usually is in the range of from 20,000:1 to 1:10, often from 10,000:1 to 1:1, regularly from 5,000:1 to 5:1, preferably from 5,000:1 to 10:1, more preferably from 2,000:1 to 30:1, even more preferably from 2,000:1 to 100:1 and in particular from 1,000:1 to 100:1. According to further embodiments, the weight ratio of the component 1) and the component 2) usually is in the range of from 1:1 to 1:1000, often from 1:1 to 1:100, regularly from 1:1 to 1:50, preferably from 1:1 to 1:20, more preferably from 1:1 to 1:10, even more preferably from 1:1 to 1:4 and in particular from 1:1 to 1:2. According to further embodiments, the weight ratio of the component 1) and the component 2) usually is in the range of from 10:1 to 1:20,000, often from 1:1 to 1:10,000, regularly from 1:5 to 1:5,000, preferably from 1:10 to 1:5,000, more preferably from 1:30 to 1:2,000, even more preferably from 1:100 to 1:2,000 to and in particular from 1:100 to 1:1,000.

In the ternary mixtures, i.e. compositions comprising the component 1) and component 2) and a compound III (component 3), the weight ratio of component 1) and component 2) depends from the properties of the active substances used, usually it is in the range of from 1:100 to 100:1, regularly from 1:50 to 50:1, preferably from 1:20 to 20:1, more preferably from 1:10 to 10:1 and in particular from 1:4 to 4:1, and the weight ratio of component 1) and component 3) usually it is in the range of from 1:100 to 100:1, regularly from 1:50 to 50:1, preferably from 1:20 to 20:1, more preferably from 1:10 to 10:1 and in particular from 1:4 to 4:1. Any further active components are, if desired, added in a ratio of from 20:1 to 1:20 to the component 1). These ratios are also suitable for mixtures applied by seed treatment.

Preference is given to mixtures comprising as component 2) at least one active substance selected from inhibitors of complex III at Qₒ site in group A), more preferably selected from compounds (A.1.1), (A.1.4), (A.1.8), (A.1.9), (A.1.10), (A.1.12), (A.1.13), (A.1.14), (A.1.17), (A.1.21), (A.1.25), (A.1.34) and (A.1.35); particularly selected from (A.1.1), (A.1.4), (A.1.8), (A.1.9), (A.1.13), (A.1.14), (A.1.17), (A.1.25), (A.1.34) and (A.1.35).

Preference is also given to mixtures comprising as component 2) at least one active substance selected from inhibitors of complex III at Qᵢ site in group A), more preferably selected from compounds (A.2.1), (A.2.3), (A.2.4) and (A.2.6); particularly selected from (A.2.3), (A.2.4) and (A.2.6).

Preference is also given to mixtures comprising as component 2) at least one active substance selected from inhibitors of complex II in group A), more preferably selected from compounds (A.3.2), (A.3.3), (A.3.4), (A.3.7), (A.3.9), (A.3.11), (A.3.12), (A.3.15), (A.3.16), (A.3.17), (A.3.18), (A.3.19), (A.3.20), (A.3.21), (A.3.22), (A.3.23), (A.3.24), (A.3.28), (A.3.31), (A.3.32), (A.3.33), (A.3.34), (A.3.35), (A.3.36), (A.3.37), (A.3.38) and (A.3.39); particularly selected from (A.3.2), (A.3.3), (A.3.4), (A.3.7), (A.3.9), (A.3.12), (A.3.15), (A.3.17), (A.3.19), (A.3.22), (A.3.23), (A.3.24), (A.3.31), (A.3.32), (A.3.33), (A.3.34), (A.3.35), (A.3.36), (A.3.37), (A.3.38) and (A.3.39).

Preference is also given to mixtures comprising as component 2) at least one active substance selected from other respiration inhibitors in group A), more preferably selected from compounds (A.4.5) and (A.4.11); in particular (A.4.11).

Preference is also given to mixtures comprising as component 2) at least one active substance selected from C14 demethylase inhibitors in group B), more preferably selected from compounds (B.1.4), (B.1.5), (B.1.8), (B.1.10), (B.1.11), (B.1.12), (B.1.13), (B.1.17), (B.1.18), (B.1.21), (B.1.22), (B.1.23), (B.1.25), (B.1.26), (B.1.29), (B.1.33), (B.1.34), (B.1.37), (B.1.38), (B.1.43), (B.1.46), (B.1.53), (B.1.54) and (B.1.55); particularly selected from (B.1.5), (B.1.8), (B.1.10), (B.1.17), (B.1.22), (B.1.23), (B.1.25), (B.1.33), (B.1.34), (B.1.37), (B.1.38), (B.1.43) and (B.1.46).

Preference is also given to mixtures comprising as component 2) at least one active substance selected from Delta14-reductase inhibitors in group B), more preferably selected from compounds (B.2.4), (B.2.5), (B.2.6) and (B.2.8); in particular (B.2.4).

Preference is also given to mixtures comprising as component 2) at least one active substance selected from phenylamides and acyl amino acid fungicides in group C), more preferably selected from compounds (C.1.1), (C.1.2), (C.1.4) and (C.1.5); particularly selected from (C.1.1) and (C.1.4).

Preference is also given to mixtures comprising as component 2) at least one active substance selected from other nucleic acid synthesis inhibitors in group C), more preferably selected from compounds (C.2.6), (C.2.7) and (C.2.8).

Preference is also given to mixtures comprising as component 2) at least one active substance selected from group D), more preferably selected from compounds (D.1.1), (D.1.2), (D.1.5), (D.2.4) and (D.2.6); particularly selected from (D.1.2), (D.1.5) and (D.2.6).

Preference is also given to mixtures comprising as component 2) at least one active substance selected from group E), more preferably selected from compounds (E.1.1), (E.1.3), (E.2.2) and (E.2.3); in particular (E.1.3).

Preference is also given to mixtures comprising as component 2) at least one active substance selected from group F), more preferably selected from compounds (F.1.2), (F.1.4) and (F.1.5).

Preference is also given to mixtures comprising as component 2) at least one active substance selected from group G), more preferably selected from compounds (G.3.1), (G.3.3), (G.3.6), (G.5.1), (G.5.3), (G.5.4), (G.5.5), G.5.6), G.5.7), (G.5.8), (G.5.9), (G.5.10) and (G.5.11); particularly selected from (G.3.1), (G.5.1) and (G.5.3).

Preference is also given to mixtures comprising as component 2) at least one active substance selected from group H), more preferably selected from compounds (H.2.2), (H.2.3), (H.2.5), (H.2.7), (H.2.8), (H.3.2), (H.3.4), (H.3.5), (H.4.9) and (H.4.10); particularly selected from (H.2.2), (H.2.5), (H.3.2), (H.4.9) and (H.4.10).

Preference is also given to mixtures comprising as component 2) at least one active substance selected from group I), more preferably selected from compounds (I.2.2) and (I.2.5).

Preference is also given to mixtures comprising as component 2) at least one active substance selected from group J), more preferably selected from compounds (J.1.2), (J.1.5), (J.1.8), (J.1.11) and (J.1.12); in particular (J.1.5).

Preference is also given to mixtures comprising as component 2) at least one active substance selected from group K), more preferably selected from compounds (K.1.41), (K.1.42), (K.1.44), (K.1.47), (K.1.57), (K.1.58) and (K.1.59); particularly selected from (K.1.41), (K.1.44), (K.1.47), (K.1.57), (K.1.58) and (K.1.59).

The compositions comprising mixtures of active ingredients can be prepared by usual means, e. g. by the means given for the compositions of compounds I.

### Examples:

### Synthetic process

### Example 4 (numbering according to Table S below): Methyl (2E)-2-methoxyimino-2-[3-methyl-2-[[(E)-1-[4-(trifluoromethyl)-2-pyridyl]ethylideneamino]oxymethyl]phenyl]acetate

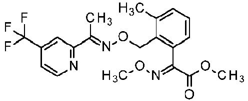

### Step 1: 1-[4-(trifluoromethyl)-2-pyridyl]ethanone

To a solution of 2-chloro-4-(trifluoromethyl)pyridine (3.0 g, 16.52 mmol) in butane-2,3-diol (10 ml), 4-vinyloxybutan-1-ol (3.06 ml, 25 mmol) was added in one portion at 25 °C. Sodium carbonate (3.5 g, 33 mmol) was added and the reaction mixture was degassed for 20 min using N₂ gas at 25 °C. Palladium acetate (0.186 g, 0.82 mmol) and 3-diphenylphosphanylpropyl(diphenyl)phosphane (0.68 g, 2 mmol) were added and the reaction mixture was stirred for 3 h at 120 °C under N₂. TLC showed the starting materials were consumed completely. The reaction mixture was cooled to 25 °C and to this 1N HCl (10 ml) was added. The reaction mixture was again heated for 1 h at 110 °C. Reaction mixture was quenched with saturated solution of NaHCO₃ (50 ml) and filtered through Celite bed. The aqueous phase filtrate was extracted with ethyl acetate (EtOAc, 2x 20 ml). Combined organic layer was dried over Na₂SO₄ and concentrated and purified by silica gel column (EtOAc:heptane = 20:80) to give 1-[4-(trifluoromethyl)-2-pyridyl]ethanone (2.5 g, 80 %) as brown liquid. ¹H NMR (500 MHz, DMSO-d₆): δ 9.03 (s, 1H), 8.15-8.10 (m, 1H), 8.09-8.08 (m,1H), 2.69 (s, 3H).

### Step 2: 1-[4-(trifluoromethyl)-2-pyridyl]ethanone oxime

To a solution of 1-[4-(trifluoromethyl)-2-pyridyl]ethanone (10 g, 52.87 mmol) in MeOH (100 ml), hydroxylamine hydrochloride (9.25 g, 132 mmol) and NaOAc (10.83 g, 132 mmol) were added under N₂. The mixture was stirred for 2 h at 70 °C under N₂. TLC showed that the reaction was completed. The reaction mixture was concentrated and then dissolved in EtOAc (100 ml) and H₂O (100 ml). The aqueous phase was extracted with EtOAc (2x 50 ml). Combined organic layer was washed with brine (100 ml), dried over Na₂SO₄ and concentrated and purified by silica gel column (EtOAc:heptane = 20:80) to give 1-[4-(trifluoromethyl)-2-pyridyl]ethanone oxime (4.3 g, 39.8%) as white solid. ¹H NMR (500 MHz, DMSO-d₆) δ = 11.8 (s, 1H), 8.88 (d, *J* = 5.0 Hz, 1H), 8.06 (s, 1H), 7.76 (d, *J =* 5.0 Hz, 1H), 2.25 (s, 3H).

### Step 3: Methyl (2E)-2-methoxyimino-2-[3-methyl-2-[[(E)-1-[4-(trifluoromethyl)-2-pyridyl]ethyl-ideneamino]oxymethyl]phenyl]acetate

To a solution of 1-[4-(trifluoromethyl)-2-pyridyl]ethanone oxime (4.3 g, 21.06 mmol) in AcN (50 ml), Cs₂CO₃ (17.11 g, 52.65 mmol) was added. The mixture was stirred for 10 min at 25 °C. Methyl (2*E*)-2-[2-(bromomethyl)-3-methyl-phenyl]-2-methoxyimino-acetate (6.95 g, 23.16 mmol) was added and the reaction mixture was stirred for 12 h at 25 °C. TLC showed that the reaction was completed. The reaction mixture was quenched with H₂O (100 ml), extracted with EtOAc (2x 100 ml). The organic phase was washed with brine (100 ml), dried over Na₂SO₄, concentrated and purified by silica gel column (heptane:EtOAc = ~ 90:10) to give the title compound (5.67 g, 63.6%) as off-white solid. ¹H NMR (500 MHz, DMSO-d₆) δ = 8.89 (d, *J =* 5.0 Hz, 1H), 8.00 (s, 1H), 7.81 - 7.79 (m, 1H), 7.34 - 7.30 (m, 2H), 7.03-7.02 (m, 1H), 5.12 (br s, 2H), 3.91 (s, 3H), 3.68 (s, 3H), 2.45 (s, 3H), 2.20 (s, 3H).

### Example 5: (2E)-2-methoxyimino-N-methyl-2-[3-methyl-2-[[(E)-1-[4-(trifluoromethyl)-2-pyridyl]-ethylideneamino]oxymethyl]phenyllacetamide

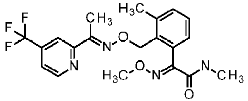

To a solution of methyl (2*E*)-2-methoxyimino-2-[3-methyl-2-[[(*E*)-1-[4-(trifluoromethyl)-2-pyridyl]ethylideneamino]oxymethyl]phenyl]acetate (12 g, 28.34 mmol) in THF (120 ml), MeNH₂ (15 ml, 40% aq. sol.) was added. The mixture was stirred for 2 h at 25 °C. TLC (EtOAc:heptane = 20:80) showed the reaction was completed. Solvent was evaporated. Crude product was diluted with H₂O (150 ml), extracted with EtOAc (3x 100 ml). Organic phase was washed with brine (100 ml), dried over Na₂SO₄ and concentrated. Crude mass was washed with n-pentane (2x 50 ml) to give the title compound (11.0 g, 91.4%) as off-white solid. ¹H NMR (500 MHz, DMSO-d₆): δ 8.88 (d, *J =* 5.0 Hz, 1H), 8.28-8.23 (m, 1H), 8.05 (s, 1H), 7.79-7.78 (m, 1H), 7.31-7.28 (m, 2H), 6.97-6.95 (m, 1H), 5.12 (br s, 2H), 3.86 (s, 3H), 2.67 (s, 3H), 2.45 (s, 3H), 2.20 (s, 3H).

### Example 11: Methyl (2E)-2-[2-[[(E)-1-[5-(4-fluorophenyl)isothiazol-3-yl]ethylideneaminoloxy-methyl]-3-methyl-phenyl]-2-methoxyimino-acetate

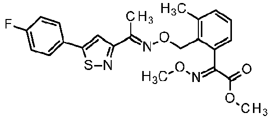

### Step 1: Methyl 3-(4-fluorophenyl)-3-oxo-propanoate

To a solution of 1-(4-fluorophenyl)ethenone (22 g, 159.26 mmol) and dimethyl oxalate (20.68 g, 175 mmol) in toluene (250 ml), potassium tertiary butoxide (26.75 g, 238.89 mmol) was added at 25°C. The mixture was stirred for 2 h at 25 °C under N₂. TLC (heptane:EtOAc = 10:1) showed that the reaction was completed. The reaction mixture was quenched with 1N HCl (70 ml) and extracted with EtOAc (2x 100 ml). The organic phase was washed with brine (50 ml), dried over Na₂SO₄ and concentrated. Crude compound was washed with heptane (200 ml) to give pure methyl 3-(4-fluorophenyl)-3-oxo-propanoate (31 g, 86.82 %). ¹H NMR: (300 MHz, DMSO-d₆): δ 8.18 - 8.15 (m, 2H), 8.14 (bs, 1H), 7.43 - 7.35 (m, 2H), 7.11 (s, 1H), 3.85 (3H, s).

### Step 2: Methyl 3-(4-fluorophenyl)-3-oxo-propanimidate

To a solution of methyl 3-(4-fluorophenyl)-3-oxo-propanoate (31 g, 138.27 mmol) in toluene (300 ml), ammonium acetate (31.94 g, 414.83 mmol) and acetic acid (1.65 g, 0.2 mmol) were added at 25°C. The mixture was stirred for 2 h at 110 °C under N₂. TLC (heptane: EtOAc = 5:1) showed that the reaction was completed. The reaction mixture was quenched with saturated aqueous sodium bicarbonate solution (30 ml) and extracted with EtOAc (2x 100 ml), the organic phase was washed with brine (50 ml), dried over Na₂SO₄ and concentrated to give pure methyl 3-(4-fluorophenyl)-3-oxo-propanimidate (30 g, 97.2%). ¹H NMR: (300 MHz, DMSO-d6):δ 9.45 (br s, 1H), 8.01 - 7.94 (m, 2H), 7.89 (br s, 1H), 7.33 - 7.27 (m, 2H), 6.43 (s, 1H), 3.89 (3H, s).

### Step 3: Methyl 5-(4-fluorophenyl) isothiazole-3-carboxylate

To a solution of methyl 3-(4-fluorophenyl)-3-oxo-propanimidate (30 g, 134.4 mmol) in THF (300 ml), P₂S₅ (20.887 g, 94 mmol) was added at 25°C. The mixture was stirred for 2 h at 25 °C under N₂ and then concentrated under reduced pressure. Crude compound was dissolved in EtOAc (200ml) followed by dropwise addition of 30% H₂O₂ (7.6 ml, 67.02 mmol) at 0°C under N₂ and stirred for 2 h. TLC (petroleum ether (PE):EtOAc = 5:1) showed that the reaction was completed. Reaction was quenched with water (50 ml) and filtered through Celite. The residue was washed with EtOAc. The filtrate was concentrated. Crude compound methyl 5-(4-fluorophenyl) isothiazole-3-carboxylate (20 g) was used without purification for next reaction. ¹H NMR (300 MHz, DMSO-d₆): δ 8.25 (s, 1H), 7.94 - 7.89 (m, 2H), 7.39 - 7.33 (m, 2H), 3.89 (3H, s).

### Step 4: 5-(4-Fluorophenyl)isothiazole-3-carboxylic acid

To a solution of methyl 5-(4-fluorophenyl) isothiazole-3-carboxylate (3 g, 12.64 mmol) in THF (12ml), MeOH (12 ml), water (6 ml) and LiOH (1.07 g, 250 mmol) were added at 25 °C. The mixture was stirred for 16 h at 25 °C under N₂. TLC (heptane: EtOAc = 5:1) showed that the reaction was completed. The mixture was concentrated under reduced pressure, dried and acidified with 1 N HCl (20ml). The precipitated compound was filtered and dried under high vacuum to afford 5-(4-fluorophenyl)isothiazole-3-carboxylic acid (2.5 g, 88.6 %) as solid. ¹H NMR (300 MHz, DMSO-d₆): δ 8.20 (s, 1H), 7.89 - 7.83 (m, 2H), 7.52 - 7.25 (m, 2H).

### Step 5: 5-(4-Fluorophenyl)isothiazole-3-carbonyl chloride

To a solution of 5-(4-fluorophenyl)isothiazole-3-carboxylic acid (2.2 g, 9.103 mmol) in dichloromethane (DCM, 30 ml), oxalyl chloride (1.06 g, 10.92 mmol) was added in small portions at 0 °C, followed by addition of a catalytic amount of DMF. The mixture was stirred for 2 h at 0 °C under N₂. TLC (PE: EtOAc = 5:1) showed that the reaction was completed. The mixture was concentrated under reduced pressure. The crude compound 5-(4-fluorophenyl) isothiazole-3-carbonyl chloride (2.5 g) was used without purification for next step.

### Step 6: 5-(4-Fluorophenyl)-N-methoxy-N-methyl-isothiazole-3-carboxamide

To a solution of 5-(4-fluorophenyl)isothiazole-3-carbonyl chloride (2.2 g, 9.103 mmol) in DCM (30 ml), methoxy methylamine hydrochloride (1.06 g, 10.92 mmol) was added in small portions at 0 °C, followed by addition of triethyl amine (1.83 g,18.20 mmol). The mixture was stirred for 4 h at 25 °C under N₂. TLC (heptane:EtOAc = 5:1) showed that the reaction was completed. The mixture was quenched with water (15 ml) and extracted with DCM (2x 50 ml). The organic phase was dried over Na₂SO₄ and concentrated to give 5-(4-fluorophenyl)-N-methoxy-N-methyl-isothiazole-3-carboxamide. ¹H NMR (300 MHz, DMSO-d₆): δ 8.01 (s, 1H), 7.89 - 7.85 (m, 2H), 7.40 - 7.33 (m, 2H), 3.75 (s, 3H), 3.34 (3H, s).

### Step 7: 1-[5-(4-Fluorophenyl) isothiazol-3-yl]ethenone

To a solution of 5-(4-fluorophenyl)-N-methoxy-N-methyl-isothiazole-3-carboxamide (2 g, 8 mmol) in THF (30 ml), 2.1 M methyl magnesium bromide (5.25 ml, 15.77 mmol) was added dropwise at -70°C. The mixture was stirred for 20 minutes at -70 °C under N₂. TLC (PE:EtOAc = 5:1) showed that the reaction was completed. The mixture was quenched with sat. aqueous ammonium chloride solution (15 ml) and extracted with EtOAc (2x 50 ml). The organic phase was washed with brine (25 ml), dried over Na₂SO₄ and concentrated to give 1-[5-(4-fluorophenyl)isothiazol-3-yl]ethenone (1.7 g, 96%). ¹H NMR (300 MHz, DMSO-d₆): δ 8.19 (s, 1H), 7.93 - 7.88 (m, 2H), 7.39 - 7.32 (m, 2H), 2.62 (s, 3H).

### Step 8: 1-[5-(4-Fluorophenyl) isothiazol-3-yl] ethenone oxime

To a solution of 1-[5-(4-fluorophenyl) isothiazol-3-yl]ethenone (1.8 g, 8.13 mmol) in MeOH / pyridine (20 ml / 2 ml), hydroxylamine hydrochloride (1.13 g, 16.26 mmol) was added under N₂. The mixture was stirred for 4 h at 65 °C under N₂. TLC (heptane:EtOAc = 5:1) showed that the reaction was completed. The mixture was concentrated and then dissolved in EtOAc (50 ml) and H₂O (20 ml). The aqueous phase was extracted with EtOAc (2x 30 ml), washed with brine (20 ml), dried over Na₂SO₄ and concentrated to give 1-[5-(4-fluorophenyl) isothiazol-3-yl]ethenone oxime (1.75 g, 86.10%). ¹H NMR (300 MHz, DMSO-d₆): δ 11.62 (s, 1H), 7.90 (s, 1H), 7.85 - 7.79 (m, 2H), 7.36 - 7.27 (m, 2H), 2.22 (s, 3H).

### Step 9: Methyl (2E)-2-[2-[[(E)-1-[5-(4-fluorophenyl)isothiazol-3-yl]ethylideneamino]oxymethyl]-3-methyl-phenyl]-2-methoxyimino-acetate

The solution of 1-[5-(4-fluorophenyl)isothiazol-3-yl]ethenone oxime (550 mg, 2.32 mmol) in DMF (6 ml), 60 % NaH (111 mg, 4.65 mmol) was added in small portions and stirred at 65 °C for 1.5 h followed by addition of methyl (2*E*)-2-[2-(bromomethyl)-3-methyl-phenyl]-2-methoxy-imino-acetate (769 mg, 2.56 mmol) and stirred at 65 °C for 2 h. TLC (heptane:EtOAc = 5:1) showed that the reaction was completed. The mixture was quenched with H₂O (30 ml) and extracted with EtOAc (2x 50 ml). The organic phase was washed with brine (30 ml), dried over Na₂SO₄, concentrated and purified by flash column chromatography to give the title compound (450 mg, 43 %). ¹H NMR (500 MHz, DMSO-d₆): δ 7.86 - 7.84 (m, 3 H), 7.38 - 7.29 (m, 4 H), 7.04 - 7.03 (m, 1 H), 5.07 (br s, 2 H), 3.92 (s, 3 H), 3.69 (s, 3 H), 2.50 (s, 3 H), 2.18 (3H, s).

### Example 12: (2E)-2-[2-[[(E)-1-[5-(4-Fluorophenyl) isothiazol-3-yl] ethylidene amino] oxymethyll-3-methyl-phenyl]-2-methoxyimino-N-methyl-acetamide

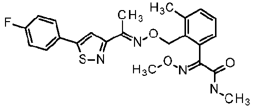

To a stirred solution of methyl (2*E*)-2-[2-[[(*E*)-1-[5-(4-fluorophenyl)isothiazol-3-yl]ethylidene-amino]oxymethyl]-3-methyl-phenyl]-2-methoxyimino-acetate (250 mg, 0.549 mmol) in THF (4 ml), methylamine (85 mg, 3 mmol) was added and the mixture was stirred for 16 h at 15 °C. TLC (heptane: EtOAc = 5:1) showed that the reaction was completed. The mixture was quenched with H₂O (10 ml) and extracted with EtOAc (3x 15 ml). The organic phase was washed with brine (50 ml), dried over Na₂SO₄ and concentrated to give the title compound (220 mg, 85.42 %) as solid. ¹H NMR (500 MHz, DMSO-d₆): δ 8.30 (d, J=5 Hz, 1 H), 7.94 - 7.89 (m, 3 H), 7.37 - 7.26 (m, 4 H), 6.96 ( d, J=6.5 Hz, 1 H), 5.01 (br s, 2 H), 3.87 (s, 3 H), 2.66 (d, J=4.5 Hz, 3 H), 2.44 (s, 3 H), 2.19 (s, 3H).

### Example 19: Methyl (2E)-2-methoxyimino-2-[2-[[(E)-1-[5-[3-(trifluoromethyl)phenyl]isoxazol-3-yl]ethylideneamino]oxymethyl]phenyl]acetate

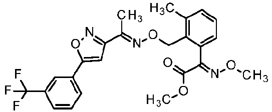

### Step 1. Methyl 2,4-dioxo-4-[3-(trifluoromethyl)phenyl]butanoate

To a solution of 1-[3-(trifluoromethyl)phenyl]ethanone (10 g, 0.05 mol) in Toluene (100 ml) , dimethyl oxalate (7.532 gm, 0.064 mol) was added. The reaction mixture was stirred at room temperature for 10 min under N₂ and then potassium tertiary butoxide (11.9 g, 0.106 mol) was added portion-wise. Then, the reaction mixture was stirred at room temperature for further 2 h. TLC (heptane:EtOAc = 5:1) showed that the reaction was completed. The mixture was diluted with 1N HCl (30 ml) and H₂O (70 ml). The aqueous phase was extracted with EtOAc (2x 100 ml). The combined EtOAc layer was washed with brine (100 ml), dried over Na₂SO₄ and concentrated. Crude product was stirred in 30 ml heptane to get solid which was filtered and washed with 20 ml heptane, dried under vacuum to afford methyl 2,4-dioxo-4-[3-(trifluoromethyl)phenyl]butanoate (6.0 g, 96.1 %) as a white solid. ¹H NMR (500 MHz, DMSO): δ 8.264 - 8.392 (m, 2H), 8.194 (t 1H), 7.802 - 7.841 (t, 1H), 7.186 (d, 1H), 3.910 (s, 3H).

### Step 2: Methyl 5-[3-(trifluoromethyl)phenyl]isoxazole-3-carboxylate

The solution of methyl 2,4-dioxo-4-[3-(trifluoromethyl)phenyl]butanoate (5.0 g, 0.018 mol) in MeOH (60 ml), hydroxylamine hydrochloride (1.9 g, 0.027 mol) was added at room temperature under N₂. The reaction mixture was stirred for 2 h at 50 °C. TLC (heptane:EtOAc = 9:1) showed that the reaction was completed. MeOH was evaporated under vacuum to get mass which was diluted with H₂O (30 ml), extracted with EtOAc (30 ml x 2 times). The organic phase was washed with brine (30 ml), dried over Na₂SO₄, concentrated and crude compound was stirred in diethyl ether (10 ml) and the obtained solid was filtered under vacuum and dried under vacuum to afford crude methyl 5-[3-(trifluoromethyl)phenyl]isoxazole-3-carboxylate (4.2 g, 85 %) as white solid.

### Step 3: 5-[3-(Trifluoromethyl)phenyl]isoxazole-3-carboxylic acid

To a stirred solution of methyl 5-[3-(trifluoromethyl)phenyl]isoxazole-3-carboxylate (4.2 g, 0.015 mol) in THF:MeOH (40ml:40 ml), LiOH.H₂O (3.252 g, 0.077 mol) dissolved in H₂O (40 ml) was added at 0 °C. The reaction mixture was stirred at RT for 16 h. TLC (40% EtOAc in heptane) showed that the reaction was completed. The mass was acidified with 1 N HCl (pH 2-3) and extracted with EtOAc (3x 40 ml). The organic phase was washed with brine (50 ml), dried over Na₂SO₄ and concentrated to give crude residue which was stirred in heptane (20 ml). The solid was filtered off and dried to afford 5-[3-(trifluoromethyl)phenyl]isoxazole-3-carboxylic acid (3.8 g, 95.4 % as white solid). ¹H NMR (500 MHz, DMSO): δ 14.2 (br s, 1H), 8.31 (s, 1H), 8.261 - 8.276 (dd, 1H), 7.912 - 7.928 (dd, 1H), 7.818-7.834 (dd, 1H), 7.679 (s, 1H).

### Step 4: N-methoxy-N-methyl-5-[3-(trifluoromethyl)phenyl]isoxazole-3-carboxamide

To a solution of 5-[3-(trifluoromethyl)phenyl]isoxazole-3-carboxylic acid (4.0 g, 0.016 mol) in DCM (50 ml), oxalyl chloride (2.0 ml, 0.023 mol) and catalytic amount of DMF were added at 0°C under N₂. The reaction mixture was brought to RT and stirred for 1 h. Progress of acid chloride formation was monitored by TLC (40 % EtOAc in heptane) and after complete conversion of acid, the reaction mass was concentrated under vacuum. The residue was dissolved in DCM (50 ml) and triethylamine (10.910 ml , 0.078 mol) added at 0°C followed by N-methoxymethanamine hydrochloride (1.8 g, 0.019 mol). The resulting mixture was stirred at RT for 4 h under N₂. TLC (heptane: EtOAc = 7:3) showed that the reaction was completed. The mixture was quenched with H₂O (50 ml), organic layer separated and the aq. phase extracted with DCM (2x 50 ml). The combined organic phase was dried over Na₂SO₄ and concentrated to give crude product which was purified by flash column chromatography (30% EtOAc in heptane) to afford N-methoxy-N-methyl-5-[3-(trifluoromethyl)phenyl]isoxazole-3-carboxamide (3.1 g, 66.4 %) as white solid. ¹H NMR: (500 MHz, DMSO): δ 8.301 (s, 1H), 8.224 - 8.283 (dd, 1H), 8.073 - 8.093 (dd, 1H), 7.58-7.864 (t, 1H), 7.527(s, 1H), 3.749 (s, 3H), 2.5 (s, 3H).

### Step 5: 1-[5-[3-(Trifluoromethyl)phenyl]isoxazol-3-yl]ethanone

To a solution of N-methoxy-N-methyl-5-[3-(trifluoromethyl)phenyl]isoxazole-3-carboxamide (2.3 g, 0.0076 mol) in THF (40 ml), methyl magnesium bromide (7.6 ml, 0.023 mol) was added dropwise at -78 °C and stirred at same temperature for 30 min under N₂. Completion of reaction was indicated by TLC (heptane:EtOAc = 5:1). The reaction mass was quenched with aq. ammonium chloride solution (10 ml) and diluted with H₂O (30 ml) and extracted with EtOAc (3x 40 ml). The organic phase was washed with brine (50 ml), dried over Na₂SO₄ and concentrated to give crude product which was purified by flash chromatography (20% EtOAc in heptane) to afford 1-[5-[3-(trifluoromethyl)phenyl]isoxazol-3-yl]ethenone (1.2 g, 61.4 %).

### Step 6: 1-[5-[3-(trifluoromethyl)phenyl]isoxazol-3-yl]ethanone oxime

To a solution of 1-[5-[3-(trifluoromethyl)phenyl]isoxazol-3-yl]ethenone (1.2 g, 0.005 mol) in MeOH (30 ml), pyridine (0.758 ml, 0.009 mol) was added at RT followed by hydroxylamine hydrochloride (0.654 g, 0.009 mol) under N₂. The resulting mixture was stirred at 65 °C for 4 h. Completion of reaction was indicated by TLC (heptane:EtOAc = 5:1). The reaction mass was evaporated under vacuum. The crude mass obtained was diluted with H₂O (30 ml) and extracted with EtOAc (3x 30 ml). The organic phase was washed with 1N HCl (50 ml) and brine (50 ml), dried over Na₂SO₄ and concentrated to give crude product which was purified by flash column chromatography (20% EtOAc in heptane) to afford 1-[5-[3-(trifluoromethyl)phenyl]-isoxazol-3-yl]ethanone oxime (1.0 g, 78.7 %). ¹H NMR: (500 MHz, DMSO): δ 11.9 (s, 1H), 8.342 - 8.309 (m, 3H), 7.884-7.899 (dd, 1H), 7.462(s, 1H), 3.749 (s, 3H), 2.2 (s, 3H).

### Step 7: Methyl (2E)-2-methoxyimino-2-[2-[[(E)-1-[5-[3-(trifluoromethyl)phenyl]isoxazol-3-yl]ethyl-ideneamino]oxymethyl]phenyl]acetate

To a stirred solution of sodium hydride (60% - 0.089 g, 2.221 mmol) in DMF (10 ml), 1-[5-[3-(trifluoromethyl)phenyl]isoxazol-3-yl]ethanone oxime (0.5 g, 1.850 mmol) dissolved in DMF (5.0 ml) was added dropwise under N₂. The mixture was stirred at RT for 1 h. Then, methyl (2E)-2-[2-(bromomethyl)-3-methyl-phenyl]-2-methoxyimino-acetate (0.611 g, 2.035 mmol) dissolved in DMF (5.0 ml) was added and stirred for 1 h. Completion of reaction was indicated by TLC (heptane:EtOAc = 5:1). The reaction mass was quenched with 1 N HCl (5 ml), diluted with H₂O (15 ml) and extracted with EtOAc (3x 20 ml). Organic phase was washed with brine (30 ml), dried over Na₂SO₄, concentrated and purified by flash chromatography (20% EtOAc in heptane) to give the title compound (0.6 g, 65.7%) as a white solid. ¹H NMR: (500 MHz, DMSO): δ 8.223 - 8.286 (m, 2H), 7.905 (d, 1H), 7.816 (t, 1H), 7.392 (m, 3H), 7.042 (d, 1H), 5.107 (br s, 2H), 3.927 (s, 3H), 3.723 (s, 3H), 2.508 (s, 3H), 2.164 (s, 3H).

### Example 22: (2E)-2-Methoxyimino-N-methyl-2-[3-methyl-2-[[(E)-1-[5-[3-(trifluoromethyl)phenyl]-isoxazol-3-yl]ethylideneaminoloxymethyllphenyl]acetamide

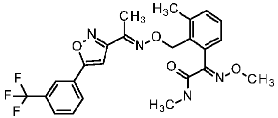

To a solution of methyl (2*E*)-2-methoxyimino-2-[2-[[(*E*)-1-[5-[3-trifluoromethyl)phenyl]isoxazol-3-yl]ethylideneamino]oxymethyl]phenyl]acetate (0.3 g, 0.613 mmol) in THF (5 ml), methyl amine (~33% in water, 2.0 ml) was added and the resulting mixture was stirred for 2 h at RT. TLC (PE:EtOAc = 5:1) showed the reaction was completed. The reaction mixture was quenched with saturated aq. NHCl₄ (10 ml) and extracted with EtOAc (3x 10ml). The organic phase was washed with brine (10 ml), dried over Na₂SO₄ and concentrated to give (2*E*)-2-methoxyimino-N-methyl-2-[3-methyl-2-[[(*E*)-1-[5-[3-(trifluoromethyl)phenyl]isoxazol-3-yl]ethylideneamino]oxymethyl]phenyl]acetamide (0.2 g, yield: 66.3 %) as white solid. ¹H NMR: (500 MHz, DMSO): δ 8.206 - 8.307 (m, 3H), 7.769 - 7.903 (m, 2H), 7.427 (t, 1H), 7.331 (m, 2H), 6.958 (d, 1H), 5.088 (br s, 2H), 3.877 (s, 3H), 2.696 (s, 3H), 2.440 (s, 3H), 2.160 (s, 3H).

### Example 27: Methyl (2E)-2-methoxyimino-2-[3-methyl-2-[[(E)-1-[3-(trifluoromethyl)-2-pyridyl]-ethylideneamino]oxymethyl]phenyl]acetate

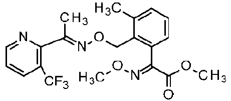

### Step 1: 3-(Trifluoromethyl)pyridine-2-carbonitrile

To a solution of 2-chloro-3-(trifluoromethyl)pyridine (5 g, 27.54 mmol) in DMF/water (50 ml/0.5 ml), Pd₂(dba)₃ (0.58 g, 0.633 mmol) catalyst, DPPF (0.702 g, 1.267 mmol) were added and degassed for 15 min. Then, zinc cyanide (1.487 g, 12.666 mmol) was added and stirred at 120 °C for 1 h under N₂. TLC (heptane:EtOAc = 10:1) showed that the reaction was completed. The reaction mixture was cooled to RT. Then, water (500 ml) was added and extracted with EtOAc (2x 50 ml). The organic phase was washed with brine (50 ml), dried over Na₂SO₄, concentrated and purified by silica gel column (heptane:EtOAc: 100:0~85:15) to give 3-(trifluoromethyl)pyridine-2-carbonitrile (3.7 g, 100%) as off-white solid. ¹H NMR (500 MHz, DMSO-d₆): δ 9.06 (d, *J =* 4.0 Hz, 1H), 8.53 - 8.51 (m, 1H), 8.03-8.01 (m, 1H).

### Step 2: 1-[3-(Trifluoromethyl)-2-pyridyl]ethanone

To a solution of 3-(trifluoromethyl) pyridine-2-carbonitrile (3.7 g, 21.50 mmol) in THF (40 ml) was added methyl magnesium bromide (3 M in diethyl ether, 14.33 ml, 42.99 mmol) dropwise at 0°C and stirred at 0°C for 1 hour. TLC (PE:EtOAc = 10:2) showed that the reaction was completed. To the reaction mixture, aq. ammonium chloride solution (50 ml) added and extracted with EtOAc (2x 50 ml). The organic phase was washed with brine (50 ml), dried over Na₂SO₄, concentrated and purified by silica gel column (heptane: EtOAc: 100:0~80:20) to give 1-[3-(trifluoromethyl)-2-pyridyl]ethenone (3.4 g, 84.6 %) as colorless oil. ¹H NMR (500 MHz, DMSO-d₆): δ 8.81 (d, *J =* 5.0 Hz, 1H), 8.11 (d, *J =* 8.0 Hz, 1H), 7.57 (dd, *J =* 5.0, 8.0 Hz, 1H), 2.71 (s, 3H).

### Step 3: (1E)-1-[3-(Trifluoromethyl)-2-pyridyl]ethanone oxime

To a solution of 1-[3-(trifluoromethyl)-2-pyridyl]ethanone (2.5 g, 13.218 mmol) in methanol (25 ml), HONH₂.HCl (1.01 g, 14.54 mmol) and 2,6-lutidine (1.847 ml, 15.8 mmol) were added. The mixture was stirred for 3 h at 60 °C under N₂. TLC (heptane: EtOAc = 10:2) showed that the reaction was completed. The mixture was cooled to RT, evaporated to dryness, quenched with H₂O (25 ml) and extracted with EtOAc (3x 25 ml). The organic phase was washed with brine (25 ml), dried over Na₂SO₄ and purified by silica gel column (heptane: EtOAc = 100:0~75:25) to give (1*E*)-1-[3-(trifluoromethyl)-2-pyridyl]ethanone oxime (2.1 g, 85%) as off-white solid. ¹H NMR (300 MHz, DMSO-d₆): δ 11.53 (s, 1H), 8.87 (d, *J =* 4.5 Hz, 1H), 8.26 (d, *J =* 8.1 Hz, 1H), 7.665-7.649 (m, 1H), 2.15 (s, 3H).

### Step 4: Methyl (2E)-2-methoxyimino-2-[3-methyl-2-[[(E)-1-[3-(trifluoromethyl)-2-pyridyl]ethyl-ideneamino]oxymethyl]phenyl]acetate

To a solution of (1*E*)-1-[3-(trifluoromethyl)-2-pyridyl]ethanone oxime (0.7 g, 3.429 mmol) in DMF (7 ml), methyl (2*E*)-2-[2-(bromomethyl)-3-methyl-phenyl]-2-methoxyimino-acetate (1.13 g, 3.772 mmol) and Cs₂CO₃ (2.229 g, 6.858 mmol) were added. The mixture was stirred for 2 h at 25 °C. TLC (heptane: EtOAc = 4:1) showed that the reaction was completed. The reaction mixture was quenched with H₂O (70 ml) and extracted with EtOAc (3x 20 ml). The organic phase was washed with brine (25 ml), dried over Na₂SO₄, concentrated and purified by silica gel column (heptane:EtOAc = 100:0~80:20) to give the title compound (1 g, yield: 68.1%) as a white solid. ¹H NMR (300 MHz, DMSO-d₆): δ 8.86 (d, *J = 4.2* Hz, 1H), 8.27 (d, *J* = 7.5 Hz, 1H), 7.68 - 7.64 (m, 1H), 7.33 - 7.26 (m, 2H), 7.02 - 6.99 (m, 1H), 4.96 (s, 2H), 3.90 (s, 3H), 3.65 (s, 3H), 2.38 (s, 3H), 2.10 (s, 3H).

### Example 28: (2E)-2-methoxyimino-N-methyl-2-[3-methyl-2-[[[E)-1-[3-(trifluoromethyl)-2-pyridyll-ethylideneamino]oxymethyl]phenyllacetamide

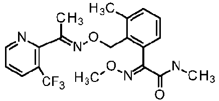

To a solution of methyl (2*E*)-2-methoxyimino-2-[3-methyl-2-[[(*E*)-1-[3-(trifluoromethyl)-2-pyridyl]ethylideneamino]oxymethyl]phenyl]acetate (500 mg, 1.181 mmol) in THF (5 ml), MeNH₂ (40% solution in water, 2 ml) was added and the mixture was stirred for 2 h at 25 °C. TLC (PE:EtOAc = 5:1) showed that the reaction was completed. The mixture was quenched with H₂O (15 ml) and extracted with EtOAc (3x 15 ml). The organic phase was washed with brine (25 ml), dried over Na₂SO₄ and concentrated to afford the title compound (400 mg, 79.2%) as white solid. ¹H NMR (300 MHz, DMSO-d₆): δ 8.86 (d, *J* = 4.5 Hz, 1H), 8.27 (d, *J* = 7.2 Hz, 1H), 8.13 (d, *J =* 4.5 Hz, 1H), 7.68 - 7.64 (m, 1H), 7.31 - 7.24 (m, 2H), 6.95 - 6.92 (m, 1H), 4.97 (s, 2H), 3.83 (s, 3H), 2.65 (d, *J =* 4.5 Hz, 3H), 2.38 (s, 3H), 1.99 (s, 3H).

### Example 29: Methyl (2E)-2-methoxyimino-2-[2-[[(E)-1-[4-[(E)-N-methoxy-C-methyl-carbon-imidoyl]-2-pyridyl]ethylideneamino]oxymethyl]-3-methyl-phenyl]acetate

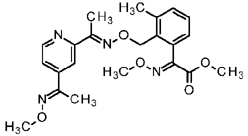

### Step 1: (E)-N-Methoxy-1-(4-pyridyl)ethanimine

To a solution of 1-(4-pyridyl)ethanone (25 g, 206 mmol) in MeOH (250 ml), methoxamine hydrochloride (25.86 g, 309 mmol) and pyridine (32.64 g, 413 mmol) were added under N₂. The mixture was stirred for 3 h at 70 °C under N₂. TLC (heptane:EtOAc = 5:1) showed that the reaction was completed. The mixture was concentrated and dissolved in EtOAc (250 ml) and H₂O (250 ml). The aqueous phase was extracted with EtOAc (2x 250 ml), washed with brine (250 ml), dried over Na₂SO₄ and concentrated to give (E)-N-methoxy-1-(4-pyridyl)ethanimine (21 g, 67.8%) as a light brown oil. ¹H NMR (500 MHz, DMSO-d₆): δ 8.62-8.61 (m, 2H), 7.63-7.61 (m, 2H), 3.97 (s, 1H), 2.19 (s, 3H).

### Step 2: 1-[4-[(E)-N-Methoxy-C-methyl-carbonimidoyl]-2-pyridyl]ethanone

To the solution of (*E*)-N-methoxy-1-(4-pyridyl)ethanimine (10 g, 67 mmol) in DCM (200 ml), 2-oxopropanoic(pyruvic) acid (17.59 g, 200 mmol), silver nitrate (0.906 g, 5.32 mmol), ammonium persulfate (22.77 g, 100 mmol) and water (200 ml) were added followed by dropwise addition of trifluoroacetic acid (22.8 g, 200 mmol). The mixture was stirred for 3 h at 40 °C. TLC (heptane:EtOAc = 4:1) showed that the reaction was completed. The mixture was diluted with DCM (200 ml) and H₂O (200 ml), filtered through celite and washed with DCM (200 ml). The aqueous phase was extracted with DCM (2x 200 ml), washed with brine (200 ml), dried over Na₂SO₄ and concentrated and purified by silica gel column (heptane: EtOAc = 100:0~70:30) to give 1-[4-[(*E*)-N-methoxy-C-methyl-carbonimidoyl]-2-pyridyl]ethanone oxime (2.6 g, 20.3%) as an off white solid. ¹H NMR (500 MHz, DMSO-d₆): δ 8.71-8.76 (m, 1H), 8.16 (s, 1H), 7.89-7.87 (m, 1H), 4.00 (s, 1H), 2.66 (s, 3H), 2.35 (s, 3H).

### Step 3: 1-[4-[(E)-N-Methoxy-C-methyl-carbonimidoyl]-2-pyridyl]ethanone oxime

To a solution of 1-[4-[(*E*)-N-methoxy-C-methyl-carbonimidoyl]-2-pyridyl]ethanone (9 g, 47 mmol) in MeOH (90 ml), hydroxylamine hydrochloride (4.881 g, 70 mmol) and pyridine (7.407 g, 94 mmol) were added under N₂. The mixture was stirred for 3 h at 70 °C under N₂. TLC (heptane:EtOAc = 4:1) showed that the reaction was completed. The mixture was concentrated, then dissolved in EtOAc (100 ml) and H₂O (100 ml). The aqueous phase was extracted with EtOAc (2x 100 ml), washed with brine (100 ml), dried over Na₂SO₄, concentrated and purified by silica gel column (heptane:EtOAc = 100:0~60:40) to give to give 1-[4-[(*E*)-N-methoxy-C-methyl-carbonimidoyl]-2-pyridyl]ethanone oxime (5.2 g, 53.6%) as a light brown solid. ¹H NMR (500 MHz, DMSO-d₆): δ 11.6 (s, 1H), 8.62-8.61 (m, 1H), 8.11 (s, 1H), 7.60-7.59 (m, 1H), 3.99 (s, 3H), 2.18 (s, 3H), 2.11 (s, 3H).

### Step 4: Methyl (2E)-2-methoxyimino-2-[2-[[(E)-1-[4-[(E)-N-methoxy-C-methyl-carbonimidoyl]-2-pyridyl]ethylideneamino]oxymethyl]-3-methyl-phenyl] acetate.

To a solution of 1-[4-[(*E*)-N-methoxy-C-methyl-carbonimidoyl]-2-pyridyl]ethanone oxime (11 g, 53 mmol) in ACN (260 ml), methyl (2*E*)-2-[2-(bromomethyl)-3-methyl-phenyl]-2-methoxy-imino-acetate (15.93 g, 53 mmol) and cesium carbonate (34.6 g, 106.1 mmol) were added. The mixture was stirred for 4 h at 20 °C. TLC (heptane:EtOAc = 5:1) showed that the reaction was completed. The mixture was quenched with H₂O (250 ml) and extracted with EtOAc (2x 200 ml). The organic phase was washed with brine (200 ml), dried over Na₂SO₄, concentrated and purified by silica gel column (heptane:EtOAc = 100:0~70:30) to give the title compound (20 g, yield: 85.7 %) as a white solid. ¹H NMR (500 MHz, DMSO-d₆): δ 8.61 (d, J=5 Hz, 1H), 7.98 (s, 1H), 7.63 - 7.62 (m, 1H) 7.33-7.29 (m, 2H) 7.03-7.02 (m, 1H) 5.08 (bs, 2 H) 4.00 (s, 3H) 3.98 (s, 3H), 3.91 (s,3H), 2.51 (s, 3H), 2.20 (s, 3 H), 2.16 (s, 3 H).

### Example 30: (2E)-2-Methoxyimino-2-[2-[[(E)-1-[4-[(E)-N-methoxy-C-methyl-carbonimidoyl]-2-pyridyl]ethylideneaminoloxymethyl]-3-methyl-phenyl]-N-methyl-acetamide

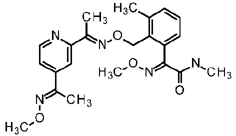

To a solution of methyl (2*E*)-2-methoxyimino-2-[2-[[(*E*)-1-[4-[(*E*)-N-methoxy-C-methyl-carbon-imidoyl]-2-pyridyl]ethylideneamino]oxymethyl]-3-methyl-phenyl]acetate (6 g, 14 mmol) in THF (60 ml), methyl amine (~40% in water, 6.54 g, 211 mmol) was added. The mixture was stirred for 2 h at 20 °C. TLC (heptane:EtOAc = 4:1) showed that the reaction was completed. The reaction mixture was quenched with H₂O (150 ml) and extracted with EtOAc (3 x 150 ml). The organic phase was washed with brine (150 ml), dried over Na₂SO₄, concentrated and purified by silica gel column (heptane:EtOAc = 100:0~60:40) to give to give the title compound (5.2 g, yield: 86%) as a white solid. ¹H NMR (500 MHz, DMSO-d₆):δ 8.61 (d, J=5 Hz, 1 H), 8.22 (d, *J*=5.00 Hz, 1 H), 8.01 (s, 1 H), 7.63 - 7.61 (m, 1 H), 7.31-7.27 (m, 2H), 6.96 - 6.94 (m, 1H), 5.08 (bs, 2H), 3.99 (s, 3H) 3.86 (s, 3H), 2.65(d, *J*=5.00 Hz, 3H), 2.47 (s, 3H) 2.21 (s, 3H), 2.16 (s, 3H).

### Example 123: Methyl (2E)-2-methoxyimino-2-[3-methyl-2-[[(E)-1-[5-(trifluoromethyl)thiazol-2-yl]-ethylideneamino]oxymethyl]phenyl]acetate

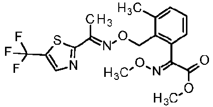

### Step 1: 2-(1,1-Dimethoxyethyl)thiazole

To a solution of 1-thiazol-2-ylethanone (50 g, 0.393 mol) in MeOH (300 ml), trimethoxymethane (258 ml, 2.34 mol) and *p*-toluene sulfonic acid (74g, 0.393 mol) were added under N₂. The reaction mixture was stirred for 16 h at 65 °C under N₂. TLC (heptane:EtOAc = 10:1) showed that the reaction was completed. The reaction mixture was cooled to RT, diluted with DCM (600ml). Saturated NaHCO₃ solution (250ml) was added to neutralize the acid. Aqueous phase was again extracted with DCM (2x 300 ml). The combined organic phase was washed with water (2x 300 ml) and brine (300 ml), dried over Na₂SO₄ and concentrated to give (2-(1,1-dimethoxyethyl)thiazole (53 g, 65.05 %) as colorless liquid. ¹H NMR (500 MHz, CHCl₃-d): δ 7.86 (d, 1H), 7.73 (d, 1H), 3.1 (s, 6H), 1.6 (s, 3H).

### Step 2: 2-(1,1-Dimethoxyethyl)-5-iodo-thiazole

To a solution of 2-(1,1-dimethoxyethyl)thiazole (42 g, 0.242 mol) in THF (420 ml) under N₂ at -78 °C, 2.5M n-butyllithium in hexane (121 ml, 0.303 mol) was added dropwise under stirring. After 45 min stirring at same temperature, iodine (64 g, 0254mol) in 100 ml THF was added dropwise under stirring. The mixture was stirred for 2 h at -78 °C under N₂. TLC (heptane:EtOAc = 10:1) showed that the reaction was completed. The mixture was quenched with 100 ml sat. ammonium chloride solution. The aqueous phase was extracted with EtOAc (2x 500 ml). The combined organic phase was washed with water (300ml) and brine (300 ml), dried over Na₂SO₄, concentrated and purified by flash column chromatography (10-15% EtOAc in heptane). Evaporation of solvent afforded 2-(1,1-dimethoxyethyl)-5-iodo-thiazole (48 g, 66.2 %) as light brown liquid. ¹H NMR (500 MHz, CHCl₃-d): δ 7.85 (s, 1H), 3.2 (s, 6H), 1.72 (s, 3H).

### Step 3: 2-(1,1-Dimethoxyethyl)-5-(trifluoromethyl)thiazole

To 2-(1,1-dimethoxyethyl)-5-iodo-thiazole (40 g, 133mmol), methyl 2,2-difluoro-2-fluorosulfonyl-acetate (28 g, 415mmol), copper iodide (28 g, 147mmol) and DMF (320 ml) were added under N₂. The reaction mixture was heated at 100 °C and stirred for 12 h. TLC (heptane:EtOAc = 10:1) showed that the reaction was completed. The reaction mass was cooled to RT. Ice water (100ml) was added followed by EtOAc (500ml). A solid was precipitated, filtered through Celite and washed thoroughly with EtOAc (50ml). The organic layer was separated. The aqueous layer was extracted with EtOAc (2x 400 ml). Combined organic layer was washed with water (2 x 300 ml) and brine (300 ml). Organic layer was dried over Na₂SO₄ and concentrated. Crude product was purified by flash column chromatography (10-15% EtOAc in heptane). Evaporation of solvent gave 2-(1,1-dimethoxyethyl)-5-(trifluoromethyl)thiazole (10.8 g, 33 %) as light brown liquid. ¹H NMR (500 MHz, CHCl₃-d): δ 8.11 (s, 1H), 3.27 (s, 6H), 1.74 (s, 3H).

### Step 4: 1-[5-(Trifluoromethyl)thiazol-2-yl]ethenone

To a solution of 2-(1,1-dimethoxyethyl)-5-(trifluoromethyl)thiazole (10 g, 41 mmol) in DCM (15 ml), trifluoracetic acid (27 ml, 410 mmol) and water ( ml) were added at 0°C. The reaction mixture was stirred for 2 h at RT. TLC (heptane:EtOAc = 10:1) showed that the reaction was completed. Reaction mass was diluted with DCM (200 ml) and neutralized with sat. NaHCO₃ solution (150 ml). The aqueous phase was extracted with DCM (2x 200 ml). The combined organic phase was washed with water (100 ml) and brine (100 ml), dried over Na₂SO₄ and concentrated to give 1-[5-(trifluoromethyl)thiazol-2-yl]ethenone (6.7 g, 82 %) as light brown oil. ¹H NMR (500 MHz, CHCl₃-d): δ 8.2 (s, 1H), 2.76 (s, 3H).

### Step 5: 1-[5-(Trifluoromethyl)thiazol-2-yl]ethenone oxime

To a solution of 1-[5-(trifluoromethyl)thiazol-2-yl]ethenone (6.7 g, 34 mmol) in MeOH (60 ml), hydroxylamine hydrochloride (3.5 g, 51 mmol) and pyridine (5.4 g, 69 mmol) were added under N₂. The reaction mixture was stirred for 4 h at 80 °C under N₂. TLC (PE: EtOAc = 10:1.5) showed that the reaction was completed. The reaction mixture was concentrated and then dissolved in EtOAc (100ml) and H₂O (100 ml). The aqueous phase was extracted with EtOAc (2x 50 ml). Combined organic layer was washed with brine (100 ml), dried over Na₂SO₄ and concentrated under vacuum. Crude product was stirred in MeOH (12 ml) for 15 min, filtered through Buchner funnel and dried under vacuum to afford 1-[5-(trifluoromethyl)thiazol-2-yl]-ethenone oxime (3.7 g, 51 %) as a white solid. ¹H NMR (500 MHz, CHCl₃-d): δ 13.2 (s, 1H), 8.6 (s, 1H), 2.2 (s, 3H).

### Step 6: Methyl (2E)-2-methoxyimino-2-[3-methyl-2-[[E-1-[5-(trifluoromethyl)thiazol-2-yl]ethyl-ideneamino]oxymethyl]phenyl]acetate

To a solution of 1-[5-(trifluoromethyl) thiazol-2-yl]ethenone oxime (3.7 g, 17.60 mmol) in ACN (40 ml), Cs₂CO₃ (11.44 g, 35.20 mmol) was added. The mixture was stirred for 10 min at 25 °C. Then, methyl (2*E*)-2-[2-(bromomethyl)-3-methyl-phenyl]-2-methoxyimino-acetate (5.8 g, 19.36 mmol) was added. The mixture was stirred for 12 h at 25 °C. TLC (PE:EtOAc = 80:20) showed that the reaction was completed. The mixture was quenched with H₂O (100 ml) and extracted with EtOAc (2x 100 ml). The combined organic phase was washed with brine (100 ml), dried over Na₂SO₄, concentrated and purified by flash column chromatography (n-heptane:EtOAc = ~80:20) to give the title compound (5.1 g, 66.8%) as white solid. ¹H NMR (500 MHz, DMSO-d): δ 8.68 (s, 1H), 7.32-7.37 (m, 2H), 7.05 (d, 1H), 5.23 (br s, 2H), 3.92 (s, 3H), 3.64 (s, 3H), 2.45 (s, 3H), 2.35 (s, 3H).

### Example 53: (2E)-2-Methoxyimino-N-methyl-2-[3-methyl-2-[[(E)-1-[5-(trifluoromethyl)thiazol-2-yllethylideneaminoloxymethyllphenyllacetamide

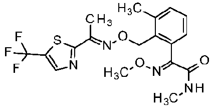

To a solution of methyl (2*E*)-2-methoxyimino-2-[3-methyl-2-[[(*E*)-1-[5-(trifluoromethyl)thiazol-2-yl]ethylideneamino]oxymethyl]phenyl]acetate (5.7 g, 13.27 mmol in THF (25 ml), methyl amine (20 ml, 40% aqueous solution) was added and the mixture was stirred for 2 h at 25 °C. TLC (50% PE:EtOAc) showed that the reaction was completed. Solvent was evaporated and crude mass obtained was diluted with H₂O (150 ml), extracted with EtOAc (3x 100 ml). The combined organic phase was washed with brine (100 ml), dried over Na₂SO₄ and concentrated. Crude product was purified by flash column chromatography (n-heptane:EtOAc = ~80:35 as eluent) to give the title compound (5.1 g, 89.7 %) as white solid. ¹H NMR (500 MHz, DMSO-d): δ 8.65 (s, 1H), 8.2 (d, 1H), 7.29-7.35 (m, 2H), 6.97 (d, 1H), 5.2 (br s, 2H), 3.81 (s, 3H), 2.62 (s, 3H), 2.48 (s, 3H), 2.35 (s, 3H).

### Example 72: Methyl (2E)-2-[2-[[(E)-[cyclopropyl-[4-(trifluoromethyl)-2-pyridyl]methylene]amino]-oxymethyl]-3-methyl-phenyl]-2-methoxyimino-acetate

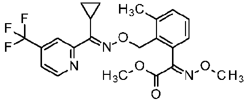

### Step 1: Cyclopropyl-[4-(trifluoromethyl)-2-pyridyl]methanone

A solution of 2-bromo-4-(trifluoromethyl)pyridine (10g, 44 mmol) in toluene (80ml) cooled to 0 °C under N₂, was charged with isopropyl magnesium chloride (2M in THF) (24.3ml, 48 mmol) in a dropwise manner for 10 min. The reaction was brought to RT and stirred for 1.5 h. The resulting dark brown mixture was cooled to 0 °C and a solution of N-methoxy-N-methylpyrimi-dine-5-carboxamide (6.28 g, 49mmol) in toluene (10ml) was added. The reaction was stirred at 0 °C for 1 h and quenched with saturated aqueous ammonium chloride (100 ml) solution. The phases were separated and the aqueous phase was extracted with EtOAc (50 ml). The combined organic layers were dried over magnesium sulfate and concentrated in vacuum. The crude material was purified using column chromatography (5-10 % EtOAc in heptane) to give cyclopropyl-[4-(trifluoromethyl)-2-pyridyl]methanone. Yield 6.28 g (63%). ¹H NMR (DMSO-d₆, 500 MHz): δ 9.07 (s,1H), 8.14-8.10 (m,2H), 3.46-3.42 (m,1H), 1.10-1.15 (m,4H).

### Step 2: Cyclopropyl-[4-(trifluoromethyl)-2-pyridyl]methanone oxime

To a solution of cyclopropyl-[4-(trifluoromethyl)-2-pyridyl]methanone (12.0g, 56 mmol) in methanol (120 ml), hydroxylamine hydrochloride (7.7g, 111 mmol) and sodium acetate (9.1 g, 111 mmol) were added and stirred for 4 h under reflux. The mixture was concentrated and partitioned between EtOAc (10 ml) and water (10 ml). The organic layer was separated, dried over Na2SO4 and concentrated under vacuum. The crude mass was purified by column chromatography (20 % EtOAc in heptane) to get cyclopropyl-[4-(trifluoromethyl)-2-pyridyl]meth-anone oxime. Yield 6.7g (52.2%). ¹H NMR (DMSO-d₆, 500MHz): δ 11.6 (s,1H), 8.81-8.80 (d,1H), 7.88 (s,1H), 7.76-7.75 (m,1H), 2.44-2.42 (m,1H), 1.27-1.25 (m,2H), 0.90-0.89 (m,2H).

### Step 3: Methyl (2E)-2-[2-[[(E)-[cyclopropyl-[4-(trifluoromethyl)-2-pyridyl]methylene]amino] oxymethyl]-3-methyl-phenyl]-2-methoxyimino-acetate

A solution of cyclopropyl-[4-(trifluoromethyl)-2-pyridyl]methanone oxime (5.5g, 24 mmol) in DMF (70 ml), methyl (2*E*)-2-[2-(bromomethyl)-3-methyl-phenyl]-2-methoxyimino-acetate (7.89g, 26 mmol) and Cs₂CO₃ (15.5g, 47.7 mmol) were added and stirred for 2 h at 25 °C. The mixture was quenched with H₂O (40ml), extracted with EtOAc (40 ml). The organic layer was separated, washed with brine (30 ml), dried over Na2SO4 and concentrated in vacuum. The crude mass was triturated with heptane (30ml) followed by crystallization in MeOH (10ml) at 0 °C. The solid was filtered and dried to give the title compound (yield 7.0 g, 65.2%). ¹H NMR (DMSO-d₆, 500 MHz): δ 8.82-8.81 (d,1H), 7.80-7.79 (m,2H) 7.34-7.30 (m,2H), 7.04-7.02 (m,1H), 5.03 (br s, 2H), 3.91 (s, 3 H), 3.63(s, 3 H), 2.33-2.29 (m,1H), 1.17-1.15 (m,2H), 0.90-0.88 (m,2H).

### Example 70: (2E)-2-[2-[[(E)-[cyclopropyl-[4-(trifluoromethyl)-2-pyridyl]methylene]amino]oxymethyl]-3-methyl-phenyl]-2-methoxyimino-N-methyl-acetamide

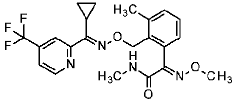

To a solution of (2*E*)-2-[2-[[(*E*)-[cyclopropyl-[4-(trifluoromethyl)-2-pyridyl]methylene]amino]-oxymethyl]-3-methyl-phenyl]-2-methoxyimino-acetate (7.0 g, 16 mmol) in THF (50ml), methyl amine (~33% in water, 20 ml) was added and the mixture was stirred for 12 h at 25 °C. The reaction was diluted with EtOAc (20 ml) and water (20 ml). The organic layer was separated and washed with water and brine, dried over Na2SO4 and concentrated under vacuum. The crude mass was purified by column chromatography (40% EtOAc in heptane) to give the title compound. Yield 6.0 g (85.3 %). ¹H NMR (DMSO-d₆, 500 MHz): δ 8.82-8.81 (d,1H), 8.23-8.22 (m,1H), 7.86-7.78 (m,2H), 7.31-7.29 (m,2H), 6.97-6.95 (m,1H), 5.03 (br s, 2 H) 3.85 (s, 3 H), 2.65(s, 3 H), 2.51 (s, 3 H), 2.34 (m,1H), 1.19-1.17 (m,2H), 0.97-0.85 (m,2H).

### Example 79: (2E)-2-[2-[[(E)-1-[5-(4-Fluorophenyl)thiazol-2-yl]ethylideneamino]oxymethyl]-3-methyl-phenyl]-2-methoxyimino-N-methyl-acetamide

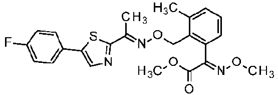

### Step 1: 1-(5-Bromothiazol-2-yl)ethenone

To a solution of 2-(1,1-dimethoxyethyl)thiazole (2.7 g, 16 mmol, 1. eq.) in THF (60 ml), n-BuLi (15.5 ml, 2.5 M, 2.5 eq.) was added slowly over a period of 15 min at -78°C under N₂, and the reaction was continued at -78°C under N₂ for 1 h. To this reaction mixture was added CBr₄ at -78°C under N₂ and continued for 2 h at same temperature. Reaction was quenched with 1 N HCl (25 ml) solution slowly at 25 °C and diluted with H₂O (100 ml) and extracted with EtOAc (3x 100 ml) followed by brine wash (2x 100 ml). Organic layer was dried over Na₂SO₄ and concentrated to afford crude compound (1.8 g). Crude compound which was purified using combi-flash column chromatography (0-10% EtOAc in heptane) to afford 1-(5-bromothiazol-2-yl)ethenone (1.7 g, 53 % yield). ¹H NMR (500 MHz, DMSO-d₆) δ = 8.24 (s, 1H), 2.60 (s, 3H).

### Step 2: 1-[5-(4-Fluorophenyl)thiazol-2-yl]ethenone

To a solution of 1-(5-bromothiazol-2-yl)ethenone (2 g, 9.70 mmol, 1 eq.) in dioxane (35 ml), K₂CO₃ (4 g, 29 mmol, 3 eq.) was added followed by H₂O (5 ml) and 4-fluorophenyboronic acid (1.6 g, 11.64 mmol, 1.2 eq.) at 25 °C. The reaction mixture was degassed by purging N₂ for 15 min followed by addition of PdCl₂(dppf)₂ (0.35 g, 0.485 mmol, 0.05 eq.) under N₂ and refluxed for 6 h at 110 °C. Reaction mixture was diluted with H₂O (50 ml) and extracted with EtOAc (3x 50 ml) followed by brine wash (2x 100 ml). Organic layer was dried over Na₂SO₄, concentrated and purified by flash chromatography (0-10% EtOAc in hexane) to afford 1-[5-(4-fluorophenyl)-thiazol-2-yl]ethenone as white solid (1.1 g, 50 % yield ). ¹H NMR (500 MHz, DMSO-d₆) δ = 8.6 (s, 1H), 8.1-7.9 (m, 2 H), 7.4-7.3 (m, 2H), 2.7 (s, 3H).

### Step 3: 1-[5-(4-Fluorophenyl)thiazol-2-yl]ethanone oxime

To a solution of 1-[5-(4-fluorophenyl)thiazol-2-yl]ethenone (1.5 g, 6.78 mmol, 1.0 eq) in MeOH (30 ml), hydroxylamine hydrochloride (0.7 g, 10 mmol, 1.5 eq) and pyridine ( 0.8 ml, 14 mmol, 2 eq) were added. The reaction mixture was refluxed for 6 h. The mixture was cooled to 25 °C and the solvent was removed under reduced pressure. The residue was diluted with water (50 ml) and extracted with EtOAc (3 x 50 ml). The organic layer was dried over anhydrous Na₂SO₄ and concentrated under reduced pressure to get the crude product which was purified by column chromatography (0-30% EtOAc in heptane) to afford 1-[5-(4-fluorophenyl)thiazol-2-yl]ethanone oxime as white solid (0.32 g, Yield: 20 % for E-isomer). ¹H NMR (500 MHz, DMSO-d₆) δ = 11.90 (s, 1H), 8.24 (s, 1H), 7.77-7.74 (m, 2H), 7.33-7.29 (m, 2 H), 2.4 (s, 3H).

### Step 4: Methyl (2E)-2-[2-[[(E)-1-[5-(4-fluorophenyl)thiazol-2-yl]ethylideneamino]oxymethyl]-3-methyl-phenyl]-2-methoxyimino-acetate

To a stirred suspension of NaH (0.076 g, 2.5 eq, 60 %, 3.174 mmol) in DMF (20 ml), 1-[5-(4-fluorophenyl)thiazol-2-yl]ethanone oxime (0.419 g, 1.397 mmol, 1. eq.) in DMF (5 ml) was added over period of 10 min at 25 °C under N₂. The mixture was stirred for 2 h at RT. To this mixture, methyl (2*E*)-2-[2-(bromomethyl)-3-methyl-phenyl]-2-methoxyimino-acetate (0.225 g, 0.931 mmol, 1.1 eq.) was added at RT and stirred at 50 °C for 2 h. The mixture was cooled to RT, quenched by H₂O (30 ml) and extracted with EtOAc (3x 30 ml), cold H₂O (3x 100 ml) followed by brine wash (3 x 50 ml). Organic layer was dried over Na₂SO₄ and concentrated. Crude compound was purified combi-flash column chromatography (0-15% EtOAc in heptane) to afford the title compound as white solid (0.2 g, 41 % yield). ¹H NMR (500 MHz, DMSO-d₆) δ = 8.4 (s, 1H), 7.7-7.8 (m, 2H), 7.3 - 7.4 (m, 4H), 7.03-7.02 (m, 1H), 5.12 (br s, 2H), 3.91 (s, 3H), 3.68 (s, 3H), 2.5 (s, 3H), 2.3 (s, 3H). MS: [M + H] + 456.

### Example 78: (2E)-2-[2-[[(E)-1-[5-(4-fluorophenyl)thiazol-2-yl]ethylideneamino]oxymethyl]-3-methyl-phenyl]-2-methoxyimino-N-methyl-acetamide

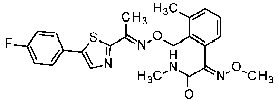

To a stirred solution of methyl (2*E*)-2-[2-[[(*E*)-1-[5-(4-fluorophenyl)thiazol-2-yl]ethylidene-amino]oxymethyl]-3-methyl-phenyl]-2-methoxyimino-acetate (0.120 g, 0.272 mmol, 1 eq.) in THF (10 ml), methyl amine hydrochloride (40 % in H₂O, 0.7 ml) was added and stirred for 1 h at 25 °C. Then, the mixture was diluted with H₂O (20 ml) and extracted with EtOAc (3x 20 ml) followed by brine wash (2x 20 ml). Organic layer was dried over Na₂SO₄ and concentrated. Crude compound was purified combi-flash column chromatography (0-30% EtOAc in heptane) to afford the title compound as white solid (0.1 g, 80 % yield). ¹H NMR (500 MHz, DMSO-d₆) δ = 8.37 (s, 1H), 8.20-8.19 (s, 1H), 7.78-7.74 (m, 2H), 7.34 - 7.28 (m, 4H), 6.97-6.96 (br s, 1H), 5.12 (br s, 2H), 3.83 (s, 3H), 2.60 (s, 3H), 2.52 (br s, 3H), 2.30 (s, 3H).

### Example 84: (2E)-2-methoxyimino-N-methyl-2-[3-methyl-2-[[(E)-1-[5-(trifluoromethyl)-3-pyridyl]-ethylideneamino]oxymethyl]phenyl]acetamide

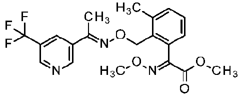

### Step 1: 1-[5-(Trifluoromethyl)-3-pyridyl]ethenone

To a solution of 3-bromo-5-(trifluoromethyl)pyridine (0.5g, 2.212 mmol, 1 eq.) in 1,4-dioxane (20 ml), 1-methoxyvinyl(tripropyl)stannane (1.012g, 0.003 mol, 1.5 eq) was added followed by addition of PdCl₂(PPh₃)₂ (0.070 g, 0.11 mmol, 0.05 eq) in one portion at 25 °C under N₂. The reaction mixture was stirred for 6 h at 100 °C under N₂. TLC (10 % EtOAc in heptane) showed that the SM was consumed completely. The reaction mixture was cooled to 25 °C and 1N HCI (10 ml) was added. The mixture was again heated for 1 h at 100 °C. Reaction was quenched with saturated solution of NaHCO₃ (20 ml), filtered through Celite bed and washed by EtOAc (30 ml). The filtrate aqueous phase was extracted with EtOAc (2x 20 ml). Combined organic layer was dried over Na₂SO₄ and concentrated to obtain 1-[5-(trifluoromethyl)-3-pyridyl]ethanone (0.2 g, 47 %) as brown liquid. The product was used in next step without purification.

### Step 2: 1-[5-(Trifluoromethyl)-3-pyridyl]ethanone oxime

To a solution of 1-[5-(trifluoromethyl)-3-pyridyl]ethanone (3 g, 15.86 mmol, 1 eq.) in MeOH (50 ml), hydroxylamine hydrochloride (2.736 g, 39.654 mmol, 2.5 eq) and pyridine (3.13 g, 0.040 mol, 2.5 eq) were added under N₂. The mixture was stirred for 2 h at 65 °C under N₂. TLC (20% EtOAc in heptane) showed that the reaction was completed. The mixture was concentrated and then dissolved in EtOAc (100 ml) and H₂O (100 ml). The aqueous phase was extracted with EtOAc (3x 50 ml). Combined organic layer was washed with brine (100 ml), dried over Na₂SO₄, concentrated and purified by silica gel column (0-20 % EtOAc in heptane) to give E-isomer 1-[5-(trifluoromethyl)-3-pyridyl]ethanone oxime (1.8 g, 54 %) as white solid. ¹H NMR (500 MHz, DMSO-d₆) δ = 11.8 (s, 1H), 9.12 (s, 1H), 8.96 (s, 1H), 8.31 (s, 1H), 2.23 (s, 3H). MS: [M + H] ⁺ 205.

### Step 3: Methyl (2E)-2-methoxyimino-2-[3-methyl-2-[[(E)-1-[5-(trifluoromethyl)-3-pyridyl]ethyl-ideneamino]oxymethyl]phenyl]acetate

To a solution of 1-[5-(trifluoromethyl)-3-pyridyl]ethanone oxime (0.500 g, 2.44 mmol, 1 eq) in DMF (10 ml), Cs₂CO₃ (1.59g, 4.89 mmol, 2 eq) was added. The mixture was stirred for 10 min at 25 °C. Methyl (2*E*)-2-[2-(bromomethyl)-3-methyl-phenyl]-2-methoxyimino-acetate (0.978 g, 2.69 mmol, 1.1 eq) in DMF ( 5ml) was added and the reaction mixture was stirred for 12 h at 25 °C under N₂. TLC (10 % EtOAc in heptane) showed that the reaction was completed. The mixture was quenched with cold H₂O (100 ml), extracted with EtOAc (3x 50 ml). The organic phase was washed with brine (100 ml), dried over Na₂SO₄, concentrated and purified by silica gel column 0-10 % EtOAc in heptane to give the title compound (0.800 g, 76.5 %) as off-white solid. ¹H NMR (500 MHz, DMSO-d₆) δ = 9.01 (d, 2H), 8.28 (s, 1H), 7.22(d, 2H), 7.01 (s, 1H), 4.98 (br s, 2H), 4.13 (s, 3H), 3.90 (s, 3H), 2.49-2.43 (d, 3H), 2.18 (s, 3H). MS: [M + H] ⁺ 424.

### Example 85: (2E)-2-methoxyimino-N-methyl-2-[3-methyl-2-[[(E)-1-[5-(trifluoromethyl)-3-pyridyl]-ethylideneamino]oxymethyl]phenyl]acetamide:

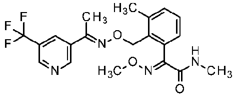

To a solution of methyl (2*E*)-2-methoxyimino-2-[3-methyl-2-[[(*E*)-1-[5-(trifluoromethyl)-3-pyridyl]ethylideneamino]oxymethyl]phenyl]acetate (0.500 g, 1 eq) in THF (10 ml), methyl amine hydrochloride was added (4 ml, 40% in H₂O) and was stirred for 2 h at 25 °C. TLC (30 % EtOAc in heptane) showed that the reaction was completed. Solvent was evaporated. Crude mass obtained was diluted with H₂O (30 ml), extracted with EtOAc (3x 20 ml). The organic phase was washed with brine (20 ml), dried over Na₂SO₄ and concentrated. Crude compound was washed with n-pentane (4x 20 ml) to give the title compound (0.300 g, 60%) as off-white solid. ¹H NMR (500 MHz, DMSO-d₆) δ = 9.10 (s, H), 9.0 (s,1H), 8.3 (d, 1H), 8.2 (br s, 1H), 7.31 (d, 2H), 6.9 (s, 1H), 5.12 (br s, 2H), 3.9(s, 3H), 2.70 (m, 3H), 2.55 (m, 3H), 2.20 (s, 3H).

### Example 89: (2E)-2-Methoxyimino-N-methyl-2-[3-methyl-2-[[(E)-1-[6-(trifluoromethyl)pyrazin-2-yl]ethylideneamino]oxymethyl]phenyl]acetamide

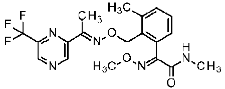

### Step 1: 1-[6-(Trifluoromethyl)pyrazin-2-yl]ethanone

To a solution of 1-(6-bromopyrazin-2-yl)ethanone (1.2 g, 5.97 mmol) and methyl 2,2-difluoro-2-fluorosulfonyl-acetate (3.4 g, 18 mmol) in DMF (12 ml), Cul (1.36 g, 7.16 mmol) was added under N₂. The mixture was stirred for 2 h at 90 °C. The colour of the mixture changed from pale brown to dark brown over time. The reaction was quenched with water and extracted with EtOAc. The emulsion formed was filtered through celite and washed with EtOAc. The layers were separated, and aqueous layer was extracted with EtOAc (10 ml). The organic layers were combined, washed with cold water, brine, dried over Na2SO4 and concentrated to dryness. The crude product was purified by column chromatography (20-25 % EtOAc in heptane) to give 1-[6-(trifluoromethyl)pyrazin-2-yl]ethenone. Yield 0.25g, (22 %). ¹H NMR (DMSO-d₆, 500MHz): δ 9.45-9.40 (d,1H), 9.13-9.07 (m,1H), 2.58(s, 3H).

### Step 2: 1-[6-(Trifluoromethyl)pyrazin-2-yl]ethanone oxime

To a solution of 1-[6-(trifluoromethyl)pyrazin-2-yl]ethenone (0.25g, 1 mmol) in methanol (4 ml) was added hydroxylamine hydrochloride (0.1g, 1.64 mmol), pyridine (0.2g, 3 mmol) under N₂. The mixture was stirred for 4 h at 66 °C. The reaction mixture was concentrated, dissolved in ethylacetate (10 ml) and washed with water (10 ml). The aqueous phase was extracted with ethylacetate and the organic layers were combined. The organic layer was concentrated and purified by column chromatography using 20% ethylacetate in heptane as eluant to give [6-(trifluoromethyl)pyrazin-2-yl]ethanone oxime. Yield 0.2g (74.1%). ¹H NMR (DMSO-d₆, 500 MHz): δ 12.2 (s,1H), 12.1 (s,1H) 9.4 (s,1H), 9.1 (s,1H), 9.0 (s,1H), 8.8 (s,1H) 2.2 (s, 6H).

### Step 3: Methyl (2E)-2-methoxyimino-2-[3-methyl-2-[[(E)-1-[6-(trifluoromethyl)pyrazin-2-yl]ethylideneamino]oxymethyl]phenyl]acetate

To a solution of 1-[6-(trifluoromethyl)pyrazin-2-yl]ethanone oxime (0.24 g, 1.17 mmol) in DMF (10 ml), methyl (2*E*)-2-[2-(bromomethyl)-3-methyl-phenyl]-2-methoxyimino-acetate (0.57 g, 1.93 mmol) and Cs₂CO₃ (1.14 g, 3.5 mmol) were added. The mixture was stirred for 1 h at 25 °C and then heated to 80 °C for 2 h. The mixture was quenched with water (40 ml) and extracted with EtOAc (40 ml). The organic phase was washed with brine (30 ml), dried over Na2SO4 and concentrated. The crude product was purified by column chromatography (20% EtOAc in heptane) to give methyl (2*E*)-2-methoxyimino-2-[3-methyl-2-[[(*E*)-1-[6-(trifluoromethyl)pyrazin-2-yl]-ethylideneamino]oxymethyl] phenyl] acetate. Yield 0.07 g (15%). ¹H NMR (DMSO-d₆, 500 MHz): δ 9.2 (s,1H), 9.2 (s,1H) 7.3 (m,2H), 7.1-7.0 (m,1H), 5.23 (br s, 2 H), 3.9 (s, 3 H), 3.7 (s, 3 H), 2.51 (s, 3 H), 2.1 (s, 3 H).

### Step 4: (2E)-2-methoxyimino-N-methyl-2-[3-methyl-2-[[(E)-1-[6-(trifluoromethyl)pyrazin-2-yl]-ethylideneamino]oxymethyl]phenyl]acetamide

To a solution of methyl (2*E*)-2-methoxyimino-2-[3-methyl-2-[[(*E*)-1-[6-(trifluoromethyl)pyrazin-2-yl]ethylideneamino]oxymethyl]phenyl]acetate (0.06g, 0.14 mmol) in THF (1 ml), methyl amine (~33% in water, 1 ml) was added. The reaction mixture was stirred for 3 h at 25 °C. The mixture was quenched with water and diluted with EtOAc (5 ml). The organic layer was separated, washed with water, brine, dried over sodium sufate and concentrated. The crude mass was purified by column chromatography (50% EtOAc in heptane) to give the title compound. Yield 0.06 g, (97.5%). ¹H NMR (DMSO-d₆, 500 MHz): δ 9.3 (s,1H), 9.2 (s,1H), 8.3 (m,1H), 7.3 (m,2H), 7.0 (m,1H), 5.23 (br s, 2 H), 3.9 (s, 3 H), 2.7(s, 3 H), 2.5 (s, 3H), 2.0(s, 3 H).

### Example 96: Methyl (2E)-2-methoxyimino-2-[3-methyl-2-[[(E)-1-[5-(trifluoromethyl)pyrazin-2-yl]ethylideneaminoloxymethyl]phenyl]acetate

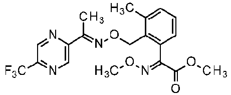

### Step 1: 1-[5-(Trifluoromethyl)pyrazin-2-yl]ethenone

To a solution of 2-chloro-5-(trifluoromethyl)pyrazine (0.5 g, 27 mmol) in dioxane (5 ml), PdCl₂(PPh₃)₂ (0.2 g, 0.27 mmol) and tributyl(1-ethoxyvinyl)stannane (1.08 g, 3 mmol) were added. The reaction was heated to reflux and stirred for 1 h. 1 N aqueous HCI (2.5 ml) was added. The reaction was stirred at reflux for 1 h. The reaction was monitored by TLC (10% EtOAc in heptane). The mixture was cooled to RT and partitioned with EtOAc. The EtOAc layer was separated, washed with brine, dried over Na₂SO₄ and concentrated. Crude mass was purified by column chromatography (5-10 % EtOAc in heptane) to give 1-[5-(trifluoromethyl)pyrazin-2-yl]-ethenone. Yield 0.3g (57.6%). ¹H NMR (CDCl₃, 500 MHz): δ 9.3 (s,1H), 9.0 (s,1H), 2.8 (s, 3H).

### Step 2: 1-[5-(Trifluoromethyl)pyrazin-2-yl]ethanone oxime

To a solution of 1-[5-(trifluoromethyl)pyrazin-2-yl]ethanone (0.3 g, 2mmol) in methanol (4 ml), hydroxylamine hydrochloride (0.13 g, 2 mmol) and pyridine (0.25 g, 2.36 mmol) were added under N₂. The mixture was stirred for 2 h under reflux, concentrated, dissolved in EtOAc (10 ml) and washed with water (10 ml). The aqueous phase was extracted with EtOAc (10 ml). The combined organic layer was washed with brine (20 ml), dried over Na₂SO₄ and concentrated. The crude mass was purified using column chromatography (20% EtOAc in heptane) to give 1-[5-(trifluoromethyl)pyrazin-2-yl]ethanone oxime. Yield 0.2g (61.8%). ¹H NMR (DMSO-d₆, 500 MHz): δ 12.3 (s,1H), 9.2 (s,2H), 2.2 (s, 3H).

### Step 3: Methyl (2E)-2-methoxyimino-2-[3-methyl-2-[[(E)-1-[5-(trifluoromethyl)pyrazin-2-yl]ethyl-ideneamino]oxymethyl]phenyl]acetate

To a solution of 1-[5-(trifluoromethyl)pyrazin-2-yl]ethanone oxime (0.2 g, 0.97 mmol) in AcN (3 ml), methyl (2*E*)-2-[2-(bromomethyl)-3-methyl-phenyl]-2-methoxyimino-acetate (0.29 g, 0.97 mmol) and Cs₂CO₃ (0.63 g, 1.95 mmol) were added. The mixture was stirred for 2 h at 25°C, quenched with water (40 ml) and extracted with EtOAc (40 ml). The organic layer was separated, washed with brine (30 ml), dried over Na2SO4 and concentrated. The crude mixture was purified by column chromatography (15% EtOAc in heptane) to give the title compound. Yield 0.4g (97.4%). ¹H NMR (DMSO-d₆, 500MHz): δ 9.2 (s,1H), 9.1 (s,1H) 7.3 (m,2H), 7.1-7.0 (m,1H), 5.23 (br s, 2 H), 3.9 (s, 3 H), 3.7(s, 3 H), 2.51 (s, 3 H), 2.2 (s, 3 H).

### Example 97: (2E)-2-methoxyimino-N-methyl-2-[3-methyl-2-[[(E)-1-[5-(trifluoromethyl)pyrazin-2-yl]ethylideneaminoloxymethyl]phenyl]acetamide

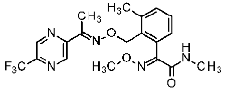

To a solution of methyl (2*E*)-2-methoxyimino-2-[3-methyl-2-[[(*E*)-1-[5-(trifluoromethyl)pyrazin-2-yl]ethylideneamino]oxymethyl]phenyl]acetate (0.2 g, 0.58 mmol) in THF (2.5 ml), methyl amine (~33% in water, 1.2 ml) was added. The reaction was stirred for 3 h at 25 °C and quenched with water. EtOAc (5 ml) was added. The organic layer was separated, washed with brine, dried over Na₂SO₄ and concentrated. The crude product was purified by column chromatography (20% EtOAc in heptane) to give the title compound. Yield 0.19 g (71.6%). ¹H NMR (DMSO-d₆, 500 MHz): δ 9.17 (s,1H), 9.12(s,1H); 8.3 (m,1H), 7.3 (m,2H), 7.0 (m,1H), 5.23 (br s, 2 H), 3.86 (s, 3 H), 2.69 (s, 3 H), 2.5 (s, 3 H), 2.18(s, 3 H).

### Example 113: Methyl (2E)-2-methoxyimino-2-[3-methyl-2-[[(E)-1-[6-(trifluoromethyl)pyrimidin-4-yl]ethylideneamino]oxymethyl]phenyl]acetate

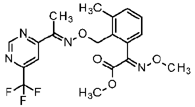

### Step 1: 1-[6-(Trifluoromethyl)pyrimidin-4-yl]ethanone

To a solution of 4-chloro-6-(trifluoromethyl)pyrimidine (1.0 g, 5.48 mmol) in dioxane (10ml), PdCl₂(PPh₃)₂ (0.4g, 0.54 mmol)and tributyl(1-ethoxyvinyl)stannane (2.96g, 8 mmol) were added. The reaction mixture was refluxed for 3 h, cooled to RT followed by addition of 1 N aqueous HCI (2.5 ml) and stirring for further 1 h. The reaction was diluted with EtOAc (30 ml). The organic layer was separated, washed using brine (40 ml), dried over Na₂SO₄ and concentrated under vacuum. The crude material was purified by column chromatography (0-30 % EtOAc in heptane) to give 1-[6-(trifluoromethyl)pyrimidin-4-yl]ethenone. Yield 0.52g (49.9%). ¹H NMR (DMSO-d₆, 500 MHz): δ 9.7 (s,1H), 8.3 (s,1H), 2.7 (s,3H).

### Step 2: 1-[6-(Trifluoromethyl)pyrimidin-4-yl]ethanone oxime

To a solution of 1-[6-(trifluoromethyl)pyrimidin-4-yl]ethanone (0.19 g, 0.99 mmol) in MeOH (10 ml), hydroxylamine hydrochloride (0.066 g, 2 mmol) and pyridine (0.15g, 2 mmol) were added. The mixture was refluxed for 1 h, concentrated and dissolved in EtOAc (10 ml) and washed with H₂O (10 ml). The aqueous phase was extracted with EtOAc (10 ml). The combined organic layer was washed with brine (10ml), dried over Na₂SO₄ and concentrated. The crude mass was purified by column chromatography (40% EtOAc in heptane) to give 1-[6-(trifluoromethyl)pyrimidin-4-yl]ethanone oxime. Yield 0.15g (73%). ¹H NMR (DMSO-d₆, 500 MHz): δ 12.8 (s,1H), 9.46 (s,1H), 8.17-8.16 (d,1H), 2.15 (s, 3H).

### Step 3: Methyl (2E)-2-methoxyimino-2-[3-methyl-2-[[(E)-1-[6-(trifluoromethyl)pyrimidin-4-yl]ethyl-ideneamino]oxymethyl]phenyl]acetate

To a solution of cyclopropyl[4-(trifluoromethyl)-2-pyridyl]methanone oxime (0.5 g, 2.4 mmol) in DMF (10 ml), methyl (2*E*)-2-[2-(bromomethyl)-3-methyl-phenyl]-2-methoxyimino-acetate (0.73 g, 2.4 mmol) and Cs₂CO₃ (1.58 g, 4.88 mmol) were added. The reaction mixture was stirred for 12 h at 25 °C. The reaction was quenched with water (30 ml), extracted with EtOAc (20 ml). The organic layer was separated, washed with brine (30 ml), dried over Na₂SO₄ and concentrated in vacuum. The crude mass was purified by column chromatography (0-30 % EtOAc in heptane) to give the title compound as a white solid. Yield 0.38g (36.4%). ¹H NMR (DMSO-d₆, 500 MHz): δ 9.47 (s,1H), 8.10 (s,1H), 7.33-7.31 (m,2H), 7.05-7.02 (m,1H), 5.17 (br s, 2 H), 3.90 (s, 3 H), 3.69 (s, 3 H) 2.50 (s, 3 H), 2.09 (s, 3 H).

### Example 114: (2E)-2-methoxyimino-N-methyl-2-[3-methyl-2-[[(E)-1-[4-(trifluoromethyl)-6-vinyl-2-pyridyl]ethylideneamino]oxymethyl]phenyl]acetamide

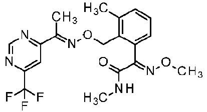

To a solution of methyl (2*E*)-2-methoxyimino-2-[3-methyl-2-[[(*E*)-1-[6-(trifluoromethyl)pyrimi-din-4-yl]ethylideneamino]oxymethyl]phenyl]acetate (0.2g, 0.47 mmol) in THF (10ml), methyl amine (~33% in water, 2 ml) was added. The mixture was stirred for 5 h at 25 °C. The reaction was diluted with EtOAc (10 ml) and water (10 ml). The organic layer was separated and washed with brine, dried over Na₂SO₄ and concentrated under vacuum. The crude product was purified by column chromatography (40% EtOAc in heptane) to give the title compound. Yield 0.18 g (90%). ¹H NMR (DMSO-d₆, 500 MHz): δ 9.5 (s,1H), 8.2 (m,1H), 8.2 (s,1H), 7.32-7.30 (m,2H), 7.0-6.9 (m,1H), 5.2 (br s, 2 H), 3.9 (s, 3 H), 2.7 (s, 3 H), 2.50 (s, 3 H), 2.09 (s, 3 H).

### Example 116: Methyl (2E)-2-methoxyimino-2-[3-methyl-2-[[(E)-1-[4-(trifluoromethyl)-6-vinyl-2-pyridyl]ethylideneaminoloxymethyl]phenyl]acetate

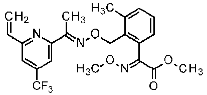

### Step 1: 1-[6-Chloro-4-(trifluoromethyl)-2-pyridyl]ethanone

To a solution of 2,6-dichloro-4-(trifluoromethyl)pyridine (1.0 g, 5 mmol) in dioxane (5 ml), PdCl₂(dppf) (0.17g, 0.5 mmol) and tributyl(1-ethoxyvinyl)stannane (1.17g, 3 mmol) were added. The reaction was stirred at reflux for 1 h under N₂. 1 N aqueous HCI (2.5 ml) was added and the reaction was continued for further 1 h at reflux. The reaction was monitored by TLC (10 % EtOAc in heptane). The mixture was brought to room temperature and diluted with water and extracted with EtOAc (30 ml). The organic layer was separated, washed with brine, dried over Na₂SO₄ and evaporated to dryness. The crude mass was purified using column chromatography (5-10 % EtOAc in heptane) to give 1-[6-chloro-4-(trifluoromethyl)-2-pyridyl]ethanone. Yield 0.43g (60%). ¹H NMR (DMSO-d₆, 500 MHz): δ 8.4 (s,1H), 8.1 (s,1H), 2.7 (s,3H).

### Step 2: 1-[6-Chloro-4-(trifluoromethyl)-2-pyridyl]ethanone oxime

To a solution of 1-[6-chloro-4-(trifluoromethyl)-2-pyridyl]ethanone (0.6 g, 2.68 mmol) in MeOH (6 ml), hydroxylamine hydrochloride (0.2 g, 4 mmol) and pyridine (0.42 g, 5 mmol) were added. The reaction was stirred for 2 h under reflux. The mixture was concentrated and partitioned between EtOAc (10 ml) and water (10 ml). Organic phase was separated, washed with brine (20 ml), dried over Na₂SO₄ and concentrated. Crude mass was purified by column chromatography (30% EtOAc in heptane) to give 1-[6-chloro-4-(trifluoromethyl)-2-pyridyl]ethanone oxime. Yield 0.32g (50.4%). ¹H NMR (DMSO-d₆, 500 MHz): δ 12.1 (s,1H), 8.0 (s,2H), 2.2 (s,3H).

### Step 3: Methyl (2E)-2-[2-[[(E)-1-[6-chloro-4-(trifluoromethyl)-2-pyridyl]ethylideneamino]oxymethyl]-3-methyl-phenyl]-2-methoxyimino-acetate

To a solution of 1-[6-chloro-4-(trifluoromethyl)-2-pyridyl]ethanone oxime (0.32 g, 1.34 mmol) in DMF (4ml), methyl (2*E*)-2-[2-(bromomethyl)-3-methyl-phenyl]-2-methoxyimino-acetate (0.4 g, 1 mmol) and cesium carbonate (0.87g, 3 mmol) were added. The mixture was stirred for 16 h at 25 °C, quenched with water (40 ml) and extracted with EtOAc (40 ml). The organic layer was separated, washed with brine (30 ml), dried over Na₂SO₄, concentrated and purified by column chromatography (0 - 20% EtOAc in heptane) to give methyl (2E)-2-[2-[[(E)-1-[6-chloro-4-(trifluoromethyl)-2-pyridyl]ethylideneamino]oxymethyl]-3-methyl-phenyl]-2-methoxyimino-acetate as a white solid. Yield 0.33g (54.7%). ¹H NMR (DMSO-d₆, 500 MHz): δ 8.01 (s,1H), 7.4 (m,1H), 7.35-7.26 (m,2H), 7.03-7.0 (m,1H), 5.23 (br s, 2 H), 4.02 (s, 3 H), 3.83 (s, 3 H), 2.49 (s, 3 H), 2.23 (s, 3 H).

### Step 4: Methyl (2E)-2-methoxyimino-2-[3-methyl-2-[[(E)-1-[4-(trifluoromethyl)-6-vinyl-2-pyridyl]-ethylideneamino]oxymethyl]phenyl]acetate

To a solution of methyl (2*E*)-2-[2-[[(*E*)-1-[6-chloro-4-(trifluoromethyl)-2-pyridyl]ethylidene-amino]oxymethyl]-3-methyl-phenyl]-2-methoxyimino-acetate (0.11 g, 0.24 mmol) in dioxane (1.4 ml) and water (0.6 ml), (dppf)PdCl₂ (0.009 g, 0.012 mmol), potassium carbonate (0.066 g, 0.48 mmol) and potassium trifluoro(vinyl)borane (0.064 g, 0.48 mmol) were added. The reaction mixture was refluxed for 1.5 h and monitored by TLC (30% EtOAc in heptane). The reaction was brought to RT and quenched with water (50 ml). The mixture was extracted with EtOAc (50 ml). The organic layer was washed with brine, dried over Na₂SO₄ and concentrated in vacuum. The crude mass was purified by column chromatography (0-30% EtOAc in heptane) to give the title compound. Yield 0.085g (76.2%). ¹H NMR (DMSO-d₆, 500 MHz): δ 8.01 (s,1H), 7.47 (m,1H), 7.35-7.26 (m,2H), 7.03-7.0 (m,1H), 6.86-6.80 (m,1H), 6.38-6.35 (d,1H), 5.58-5.56 (d,1H), 5.18 (br s, 2 H), 4.02 (s, 3 H), 3.82(s, 3 H), 2.50 (s, 3 H), 2.30 (s, 3 H).

### Example 138: (2E)-2-methoxyimino-N-methyl-2-[3-methyl-2-[[(E)-1-[4-(trifluoromethyl)-6-vinyl-2-pyridyl]ethylideneamino]oxymethyl]phenyl]acetamide

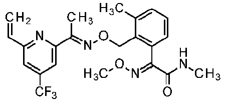

To a solution of methyl (2*E*)-2-methoxyimino-2-[3-methyl-2-[[(*E*)-1-[4-(trifluoromethyl)-6-vinyl-2-pyridyl]ethylideneamino]oxymethyl]phenyl]acetate (0.28 g, 0.62 mmol) in THF (8 ml), methyl amine (~33% in water, 1.2ml) was added. The mixture was stirred for 3 h at 25 °C. The reaction was diluted with EtOAc and washed with water. The organic layer was separated and washed with brine, dried over Na₂SO₄ and concentrated under vacuum. The crude mass was purified via column chromatography (30% EtOAc in heptane) to give the title compound. Yield 0.25g (99.3%). ¹H NMR (DMSO-d₆, 500 MHz): δ 8.23-8.22 (s,1H), 7.92-7.88 (m,2H), 7.31-7.27 (m,2H), 6.98-6.92 (m,2H), 6.48-6.45 (d,1H), 5.66-5.63 (d,1H), 5.12 (br s, 2 H), 3.86 (s, 3 H), 2.67(s, 3 H), 2.50 (s, 3 H), 2.22 (s, 3 H).

### Example 117: Methyl (2E)-2-methoxyimino-2-[3-methyl-2-[[(E)-1-[2-methyl-5-(trifluoromethyl) pyrazol-3-yl]ethylideneamino]oxymethyl]phenyl]acetate

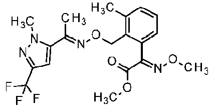

### Step 1: 2-Methyl-5-(trifluoromethyl)-1H-pyrazol-3-one

To a solution of ethyl 4,4,4-trifluoro-3-oxo-butanoate (1.5 g, 8.1 mmol) in ethanol (20 ml), methylhydrazine (0.45 g, 10 mmol), pyridine (1.41 g, 18 mmol) and H₂SO₄ (1 ml) were added. The reaction mixture was stirred for 2 h at RT. The reaction was diluted with EtOAc (10 ml), washed with water, then with brine, dried over Na₂SO₄ and concentrated under vacuum. The crude mass was purified by column chromatography (15-20% EtOAc in heptane) to give 2-methyl-5-(trifluoromethyl)-1H-pyrazol-3-one. Yield 0.4 g (29.4%). ¹H NMR (DMSO-d₆, 500 MHz): δ 11.69 (s,1H), 5.72 (s,1H), 3.59 (s, 3H).

### Step 2: [2-Methyl-5-(trifluoromethyl)-1,3-dihydropyrazol-3-yl]trifluoromethanesulfonate

To a solution of 2-methyl-5-(trifluoromethyl)-1H-pyrazol-3-one (0.8 g, 5 mmol) in DCM (10 ml), triethylamine (0.13g, 5.7 mmol) was added at 0 °C and the mixture was stirred for 10 minutes. The reaction was brought to RT, bis(trifluoromethyl)sulfonic anhydride (1.63 g, 5.7 mmol) was added and stirred for 30 minutes. The reaction was diluted with EtOAc (10 ml) and washed with water, brine and the organic layer was separated. The organic layer was dried over Na₂SO₄ and concentrated under vacuum. The crude mass was purified by column chromatography (15-20% EtOAc in heptane) as eluant to give [2-methyl-5-(trifluoromethyl)-1,3-dihydropyrazol-3-yl]trifluoromethanesulfonate. Yield 53.0 g (83%). ¹H NMR (DMSO-d₆, 500 MHz): δ 7.11 (s,1H), 3.91(s, 3H).

### Step 3: 1-[2-Methyl-5-(trifluoromethyl)pyrazol-3-yl]ethanone

To a solution of [2-methyl-5-(trifluoromethyl)-1,3-dihydropyrazol-3-yltrifluoromethanesulfonate (0.3 g, 1 mmol) in dioxane (10 ml), PdCl₂(dppf) (0.002 g, 0.1 mmol) and tributyl(1-ethoxyvinyl)-stannane (0.54g, 2 mmol) were added and the reaction was refluxed. After 1 h, the reaction was brought to RT and 1 N aqueous HCI (2.5 ml) was added and the reaction was continued for 1 h. The reaction mixture was diluted with EtOAc (30 ml). The organic layer was separated, washed with brine (20 ml), dried over Na₂SO₄ and concentrated in vacuum. The crude mass was purified by column chromatography (5-30 % EtOAc in heptane) to give 1-[2-methyl-5-(trifluoromethyl)pyrazol-3-yl]ethenone. Yield 0.43 g, (60 %).

### Step 4: 1-[2-Methyl-5-(trifluoromethyl)pyrazol-3-yl]ethanone oxime

To a solution of 1-[6-(trifluoromethyl)pyrimidin-4-yl]ethanone (1.2 g, 6mmol) in MeOH (20 ml), hydroxylamine hydrochloride (0.19 g, 1.2 mmol) and pyridine (0.98 g, 1.2 mmol) were added and stirred under reflux for 3 h. The mixture was concentrated and dissolved in EtOAc (10 ml). The EtOAc extract was washed with water, brine, dried over sodium sufate and concentrated under vacuum. The crude mass was purified by column chromatography (30% EtOAc in heptane) to give 1-[2-methyl-5-(trifluoromethyl)pyrazol-3-yl]ethanone oxime. Yield 0.45 g (34.8 %). ¹H NMR (DMSO-d₆, 500 MHz): δ 11.68 (s,1H), 7.01 (s,1H), 3.96 (s, 3H), 2.09 (s, 3H).

### Step 5: Methyl (2E)-2-methoxyimino-2-[3-methyl-2-[[(E)-1-[2-methyl-5-(trifluoromethyl) pyrazol-3-yl]ethylideneamino]oxymethyl]phenyl]acetate

To a solution of 1-[2-methyl-5-(trifluoromethyl)pyrazol-3-yl]ethanone oxime (0.52 g, 2.5 mmol) in DMF (10 ml), methyl (2*E*)-2-[2-(bromomethyl)-3-methyl-phenyl]-2-methoxyimino-acetate (0.8 g, 2.7 mmol) and Cs₂CO₃ (1.63 g, 5.02 mmol) were added. The reaction mixture was stirred for 6 h at 25 °C. The reaction was diluted with water (30 ml) and extracted with EtOAc (40 ml). The organic layer was separated and washed with brine (30 ml), dried over Na₂SO₄ and concentrated under vacuum. The crude mass was purified by column chromatography (30 % EtOAc in heptane) to give the title compound as a white solid. Yield 0.52 g (48.1%). ¹H NMR (DMSO-d₆, 500 MHz): δ 7.32-7.28 (m,2H), 7.04-7.02 (m,1H), 5.07 (br s, 2 H), 3.90 (s, 3 H), 3.86 (s, 3 H), 3.67 (s, 3 H), 2.42 (s, 3 H), 2.1 (s, 3 H).

### Example 118: (2E)-2-methoxyimino-N-methyl-2-[3-methyl-2-[[(E)-1-[2-methyl-5-(trifluoromethyl)pyrazol-3-yl]ethylideneamino]oxymethyl]phenyl]acetamide

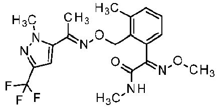

To a solution of methyl (2*E*)-2-methoxyimino-2-[3-methyl-2-[[(*E*)-1-[2-methyl-5-(trifluoromethyl)pyrazol-3-yl]ethylideneamino]oxymethyl]phenyl]acetate (0.41 g, 0.96 mmol) in THF (10 ml), methyl amine (~33% in water, 2 ml) was added. The mixture was stirred for 5 h at 25 °C. The reaction was diluted with H₂O (10 ml) and EtOAc (25 ml). Organic layer was separated and washed with brine, dried over Na₂SO₄ and concentrated under vacuum. Crude mass was purified by column chromatography (30% EtOAc in heptane) to give title compound. Yield 0.4 g (99.8%). ¹H NMR (DMSO-d₆, 500 MHz): δ 8.23-8.22 (d,1H), 7.28-7.27 (m,2H), 7.12 (s,1H), 6.96 -6.95 (m,1H), 5.07 (br s, 2H), 3.86 (s, 3H), 2.66 (s, 3H), 2.51 (s, 3H), 2.41 (s, 3H), 2.10 (s, 3H).

The following examples in Table S were synthesized as described above and characterized by LCMS as described in Table L using Method A for compound examples 1 to 159 while using Method B for compound examples 160 to 164.

**Table L: LCMS Methods**

| **Method details** | **Device details** |
|---|---|
| **Method A** | |
| Column: Agilent Eclipse Plus C18 (50 mm | LCMS2020 (Shimadzu) |
| × 4.6 mm × 3µm) | lonization source: ESI |
| Mobile Phase: | Mass range: 100 - 800 amu |
| A: 10 mM Ammonium formate in water | Polarity: Dual (positive and negative simultaneous scan) |
| B: 0.1 % Formic acid in ACN | |
| Gradient: 10 % B to 100 % B in 1.5 min. | Mode: Scan |
| Hold 1 min 100 % B. 1 min 10 % B. Run time: 3.50 or 3.75 min. | LC System: Nexera High pressure gradient system, Binary pump |
| Flow: 1.2 ml/min; | Detector: PDA |
| Column oven: 30°C/40°C | Scanning wavelength: 220 nm / max plot |

| **Method B** | |
|---|---|
| Column: Luna-C18 (30 mm × 2.0 mm × 3 µm) | LCMS DELIVER-220 (Shimadzu) |
| | lonization source: ESI |
| Mobile Phase: | Mass range: 100 - 1000 amu |
| A: 0.037% Trifluoroacetic acid in water | Polarity: Positive |
| B: 0.018% Trifluoroacetic acid in ACN | Mode: Scan |
| Gradient: 5% B in 0.01 min, 5-95% B (0.01-1.60 min), 95-100% B (1.6-2.5 min), 100-5% B (2.50-2.52 min) with a hold at 5% B for 0.48 min. | LC System: Nexera High pressure gradient system, Binary pump |
| | Detector: DAD |
| | Scanning wavelength: 220 nm / max plot |
| Flow: 0.8 mL/min; Column oven: 40°C | |

**Table S:**

| **No.** | **Structure** | **Rₜ [min]** | **Mass** |
|---|---|---|---|
| 1 | 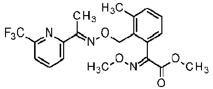 | 2.14 | 424.2 |
| 2 | 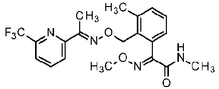 | 2.04-2.05 | 423.3 |
| 3 | 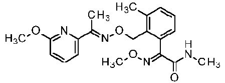 | 1.98-1.99 | 385.2-385.4 |
| 4 | 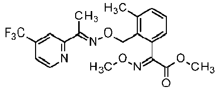 | 2.13-2.2 | 424 |
| 5 | 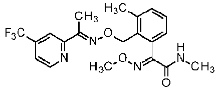 | 2.003 | 432.5 |
| 6 | 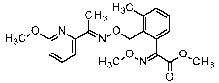 | 2.14 | 386.4 |
| 7 | 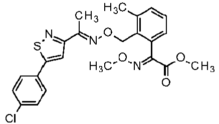 | 2.26 | 472.1 |
| 8 | 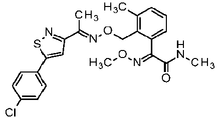 | 2.18 | 471.2 |
| 9 | 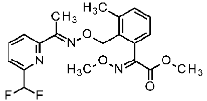 | 2.1 | 406 |
| 10 | 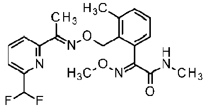 | 1.92 | 405 |
| 11 | 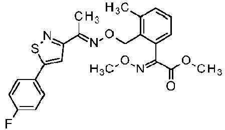 | 2.2 | 456 |
| 12 | 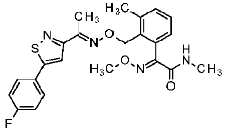 | 2.19 | 455 |
| 13 | 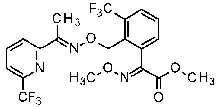 | 2.19 | 478.2 |
| 14 | 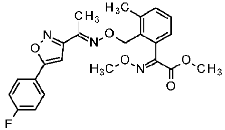 | 2.18 | 440.1 |
| 15 | 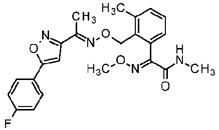 | 2.09 | 439.1 |
| 16 | 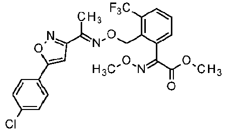 | 2.31 | 510 |
| 17 | 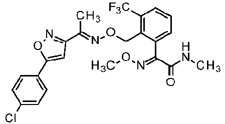 | 2.23 | 509.1 |
| 18 | 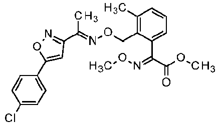 | 2.27 | 456.1 |
| 19 | 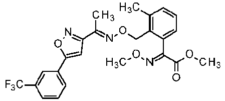 | 2.26 | 490.2 |
| 20 | 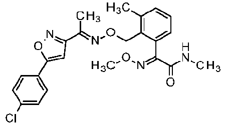 | 2.19 | 455.2 |
| 21 | 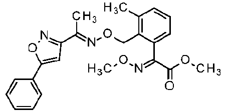 | 2.16 | 422.3 5 |
| 22 | 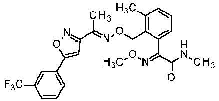 | 2.17 | 489.2 |
| 23 | 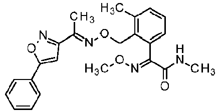 | 2.05 | 421.4 |
| 24 | 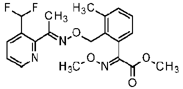 | 2.00 | 406 |
| 25 | 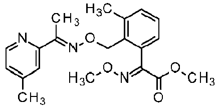 | 1.97 | 370.1 5 |
| 26 | 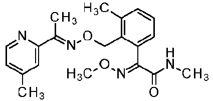 | 1.82 | 369.2 |
| 27 | 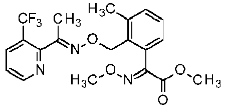 | 1.98 | 424 |
| 28 | 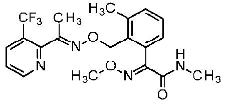 | 1.85-1.87 | 423 |
| 29 | 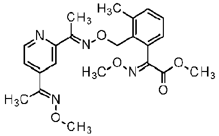 | 2.16 | 427 |
| 30 | 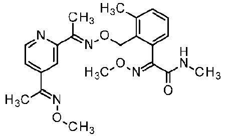 | 1.95 | 426 |
| 31 | 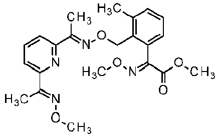 | 2.38 | 427.1 |
| 32 | 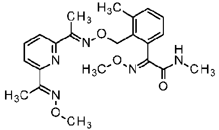 | 2.13 | 426.1 |
| 33 | 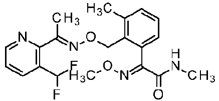 | 1.84 | 405 |
| 34 | 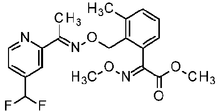 | 2.00-2.07 | 406 |
| 35 | 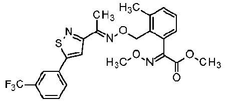 | 2.29 | 506 |
| 36 | 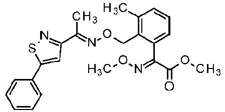 | 2.21 | 438 |
| 37 | 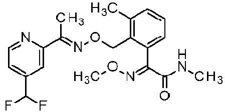 | 1.86 | 405 |
| 38 | 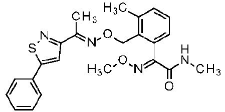 | 2.12 | 437 |
| 39 | 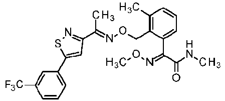 | 2.2 | 505 |
| 40 | 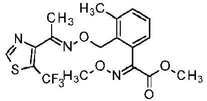 | 2.03 | 429.9 |
| 41 | 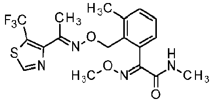 | 1.90 | 428.9 |
| 42 | 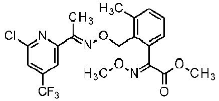 | 2.24 | 457.9 |
| 43 | 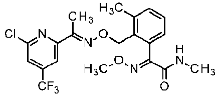 | 2.16 | 456.9 |
| 44 | 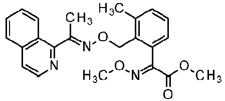 | 2.05 | 405.9 5 |
| 45 | 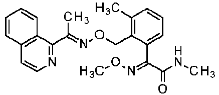 | 1.87 | 405 |
| 46 | 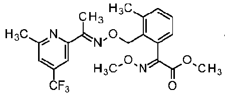 | 2.19 | 438 |
| 47 | 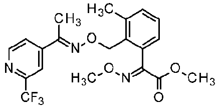 | 2.04 | 424.8 |
| 48 | 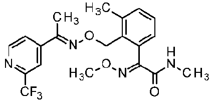 | 1.92 | 423.8 |
| 49 | 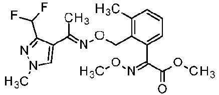 | 1.84 | 409 |
| 50 | 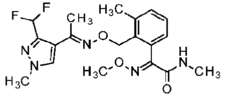 | 1.72 | 408.8 |
| 51 | 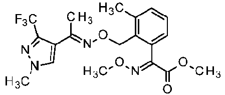 | 1.95 | 427 |
| 52 | 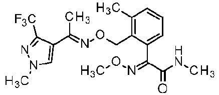 | 1.82 | 426.8 |
| 53 | 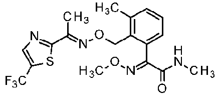 | 2.1 | 429 |
| 54 | 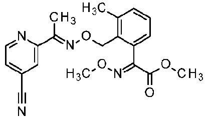 | 1.96-2.04 | 381 |
| 55 | 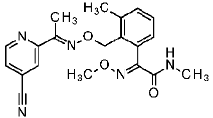 | 1.85 | 380 |
| 56 | 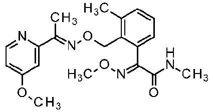 | 1.77 | 385.1 |
| 57 | 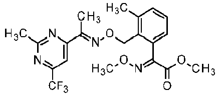 | 2.17 | 439 |
| 58 | 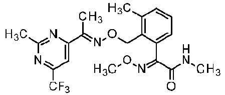 | 2.05 | 438 |
| 59 | 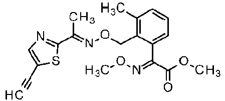 | 2.04 | 386.7 |
| 60 | 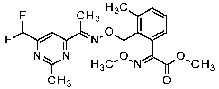 | 2.05 | 421 |
| 61 | 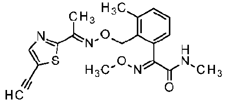 | 1.88 | 385 |
| 62 | 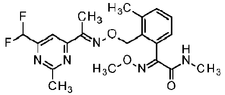 | 1.9 | 420 |
| 63 | 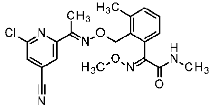 | 2.02 | 414 |
| 64 | 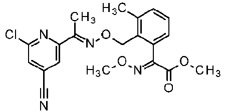 | 2.11 | 415 |
| 65 | 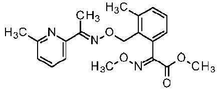 | 2.06 | 370 |
| 66 | 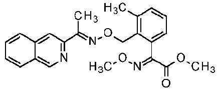 | 2.09 | 407 |
| 67 | 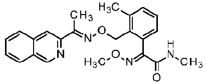 | 1.93 | 405 |
| 68 | 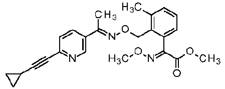 | 2.06 | 420.1 5 |
| 69 | 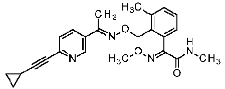 | 1.92 | 419.1 |
| 70 | 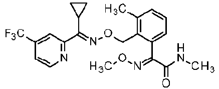 | 2.06-2.14 | 449 |
| 71 | 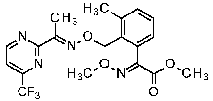 | 1.96 | 425 |
| 72 | 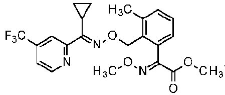 | 2.14 | 450 |
| 73 | 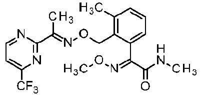 | 1.83 | 424 |
| 74 | 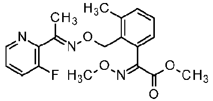 | 1.91 | 374 |
| 75 | 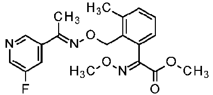 | 1.92 | 374 |
| 76 | 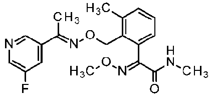 | 1.77 | 373 |
| 77 | 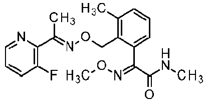 | 1.75 | 373 |
| 78 | 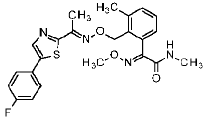 | 2.08 | 455.8 |
| 79 | 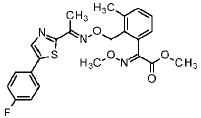 | 2.13 | 424.3 |
| 80 | 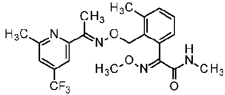 | 2.1 | 437 |
| 81 | 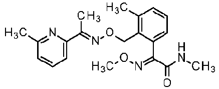 | 1.90 | 369 |
| 82 | 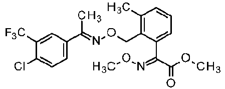 | 2.26 | 457 |
| 83 | 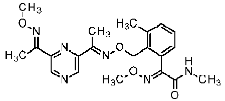 | 2.05 | 427 |
| 84 | 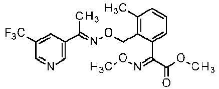 | 2.03 | 424 |
| 85 | 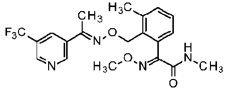 | 1.92 | 423 |
| 86 | 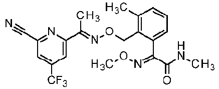 | 2.01 | 448 |
| 87 | 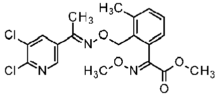 | 2.14 | 424 |
| 88 | 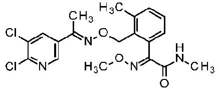 | 2.03 | 423 |
| 89 | 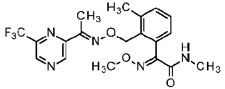 | 1.98 | 424 |
| 90 | 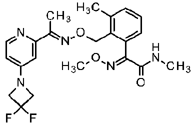 | 1.77 | 445.8 |
| 91 | 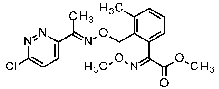 | 1.93 | 391 |
| 92 | 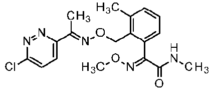 | 1.80 | 390 |
| 93 | 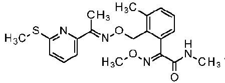 | 1.90 | 401 |
| 94 | 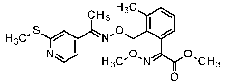 | 2.04 | 402 |
| 95 | 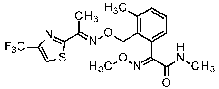 | 1.99 | 429 |
| 96 | 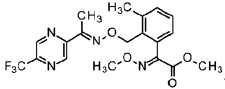 | 2.11 | 425 |
| 97 | 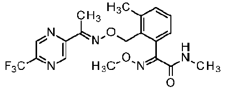 | 2.08-2.16 | 424 |
| 98 | 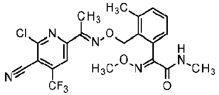 | 2.08 | 482 |
| 99 | 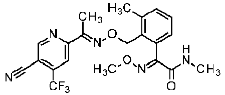 | 1.98 | 448.2 5 |
| 100 | 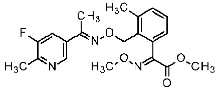 | 1.96 | 388 |
| 101 | 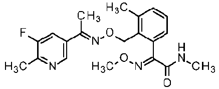 | 1.83 | 387 |
| 102 | 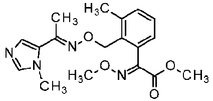 | 1.74 | 359 |
| 103 | 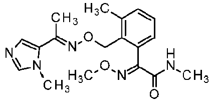 | 1.98 | 358 |
| 104 | 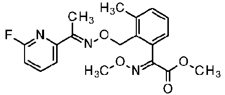 | 2.01 | 374 |
| 105 | 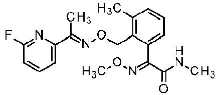 | 1.88 | 373 |
| 106 | 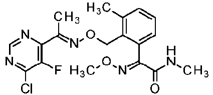 | 1.86 | 408 |
| 107 | 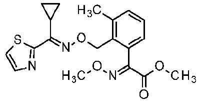 | 2.02 | 388 |
| 108 | 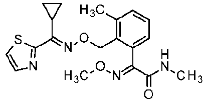 | 1.84 | 387 |
| 109 | 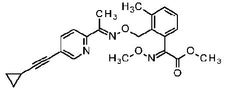 | 2.19 | 420 |
| 110 | 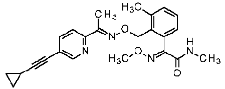 | 2.08 | 419 |
| 111 | 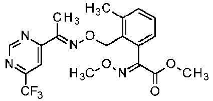 | 2.08 | 425 |
| 112 | 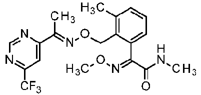 | 1.99 | 424 |
| 113 | 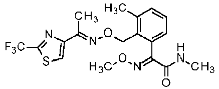 | 1.97 | 429 |
| 114 | 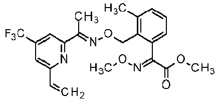 | 2.32 | 449.6 5 |
| 115 | 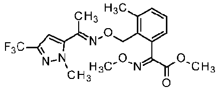 | 2.01 | 427 |
| 116 | 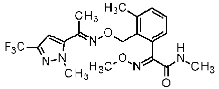 | 1.95 | 426 |
| 117 | 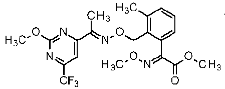 | 2.15 | 455 |
| 118 | 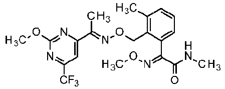 | 2.04 | 454 |
| 119 | 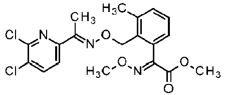 | 2.18 | 425 |
| 120 | 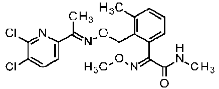 | 2.10 | 424 |
| 121 | 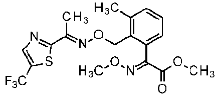 | 2.22 | 430 |
| 122 | 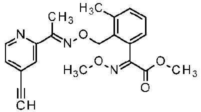 | 2.11 | 380 |
| 123 | 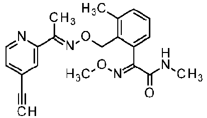 | 1.98 | 379 |
| 124 | 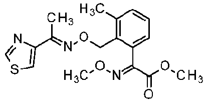 | 1.85 | 362 |
| 125 | 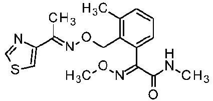 | 1.69 | 361 |
| 126 | 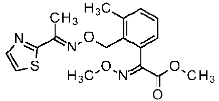 | 1.89 | 362 |
| 127 | 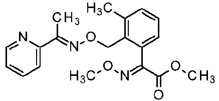 | 1.83 | 356 |
| 128 | 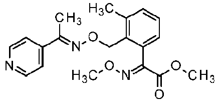 | 1.82 | 356 |
| 129 | 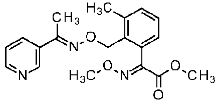 | 1.95 | 356 |
| 130 | 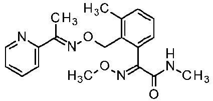 | 1.66 | 355 |
| 131 | 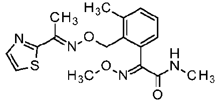 | 1.73 | 361 |
| 132 | 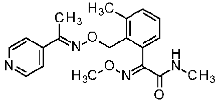 | 1.66 | 355 |
| 133 | 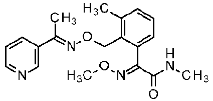 | 1.77 | 355 |
| 134 | 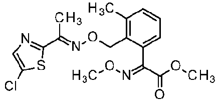 | 2.09 | 396 |
| 135 | 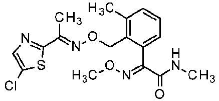 | 1.95 | 395 |
| 136 | 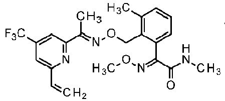 | 2.26 | 449 |
| 137 | 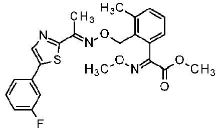 | 2.72 | 456 |
| 138 | 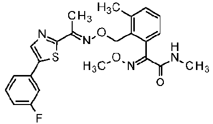 | 2.17 | 455 |
| 139 | 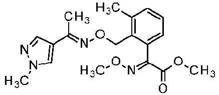 | 1.76 | 359 |
| 140 | 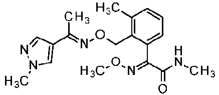 | 158 | 358 |
| 141 | 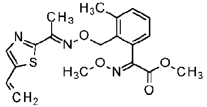 | 2.11 | 388 |
| 142 | 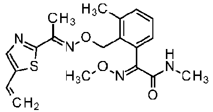 | 1.93 | 388 |
| 143 | 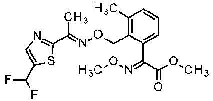 | 2.07 | 412 |
| 144 | 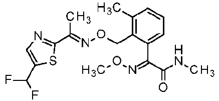 | 1.92 | 411 |
| 145 | 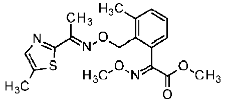 | 1.99 | 376 |
| 146 | 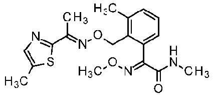 | 1.82 | 375 |
| 147 | 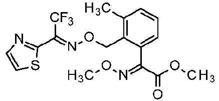 | 1.88 | 415 |
| 148 | 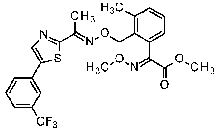 | 2.30 | 506 |
| 149 | 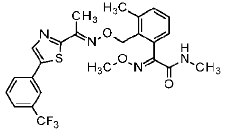 | 2.19 | 505 |
| 150 | 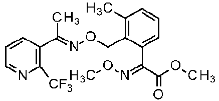 | 1.97 | 424 |
| 151 | 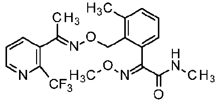 | 1.85 | 423 |
| 152 | 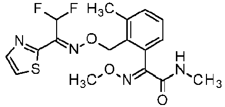 | 1.79 | 397 |
| 153 | 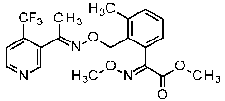 | 1.99 | 424 |
| 154 | 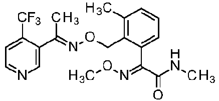 | 1.84 | 423 |
| 155 | 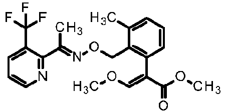 | 1.92 | 423 |
| 156 | 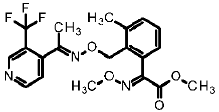 | 2.01 | 424 |
| 157 | 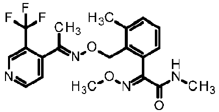 | 1.87 | 423 |
| 158 | 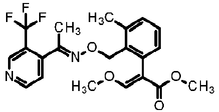 | 1.94 | 423 |
| 159 | 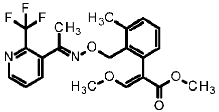 | 2.02 | 423 |
| 160 | 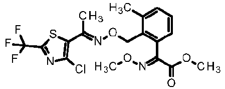 | 1.96 | 464 |
| 161 | 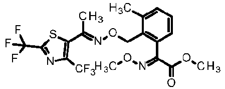 | 1.95 | 498 |
| 162 | 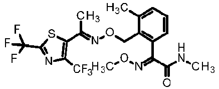 | 1.87 | 497 |
| 163 | 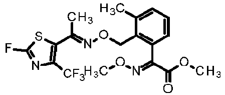 | 1.83 | 448 |
| 164 | 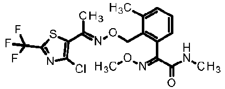 | 1.86 | 463 |

### Biological studies

### A. Green House

The compound was dissolved in a mixture of acetone and/or dimethylsulfoxide and the wetting agent/emulsifier Wettol, which is based on ethoxylated alkylphenoles, in a ratio (volume) solvent-emulsifier of 99 to 1 to give a total volume of 5 ml. Subsequently, water was added to total volume of 100 ml. This stock solution was then diluted with the described solvent-emulsifier-water mixture to the final concentration given in the table below.

### Use example 1. Protective control of soybean rust on soybeans caused by Phakopsora pachyrhizi (PHAKPA P2)

Leaves of potted soybean seedlings were sprayed to run-off with the previously described spray solution, containing the concentration of active ingredient or their mixture as described below. The plants were allowed to air-dry. The trial plants were cultivated for 2 days in a greenhouse chamber at 23-27 °C and a relative humidity between 60 and 80 %. Then the plants were inoculated with spores of *Phakopsora pachyrhizi.* The strain used contains the amino acid substitution F129L in the mitochondrial cytochrome b protein conferring resistance to Qo inhibitors. To ensure the success the artificial inoculation, the plants were transferred to a humid chamber with a relative humidity of about 95 % and 20 to 24 °C for 24 hr. The trial plants were cultivated for up to 14 days in a greenhouse chamber at 23 to 27 °C and a relative humidity between 60 and 80 %. The extent of fungal attack on the leaves was visually assessed as % diseased leaf area, the disease level of untreated controls was usually higher than 85 %.

### Use example 2. Protective control of soybean rust on soybeans caused by Phakopsora pachyrhizi (PHAKPA P6)

Leaves of potted soybean seedlings were sprayed to run-off with the previously described spray solution, containing the concentration of active ingredient as described below. The plants were allowed to air-dry. The trial plants were cultivated for six days in a greenhouse chamber at 23-27 °C and a relative humidity between 60 and 80 %. Then the plants were inoculated with spores of *Phakopsora pachyrhizi.* The strain used contains the amino acid substitution F129L in the mitochondrial cytochrome b protein conferring resistance to Qo inhibitors. To ensure the success the artificial inoculation, the plants were transferred to a humid chamber with a relative humidity of about 95 % and 23 to 27 °C for 24 hr. The trial plants were cultivated for up to 14 days in a greenhouse chamber at 23 to 27 °C and a relative humidity between 60 and 80 %. The extent of fungal attack on the leaves was visually assessed as % diseased leaf area, the disease level of untreated controls was usually higher than 85 %.

The results of the abovementioned use examples are given in Table 1. All test results below are given for the control of phytopathogenic fungi containing the amino acid substitution F129L in the mitochondrial cytochrome b protein conferring resistance to Qo inhibitors.

**Table 1:**

| **Treatment** | | **PHAKPA (F129L) Disease level (%)** | | | |
|---|---|---|---|---|---|
| **No.** | **Structure** | **P2 at 16 ppm** | **P6 at 16 ppm** | **P2 at 4 ppm** | **P6 at 4 ppm** |
| 1 | 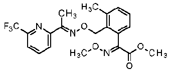 | 57 | 73 | 80 | 70 |
| 2 | 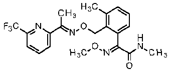 | 17 | 27 | 67 | 59 |
| 3 | 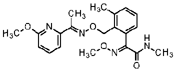 | 8 | 28 | 50 | 60 |
| 4 | 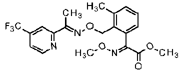 | 0 | 0 | 3 | 4 |
| 5 | 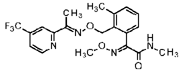 | 0 | 0 | 1 | 2 |
| 6 | 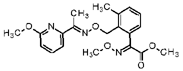 | 32 | 67 | 60 | 73 |
| 7 | 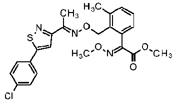 | 22 | 26 | 31 | 42 |
| 8 | 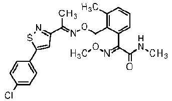 | 27 | 40 | 73 | 53 |
| 9 | 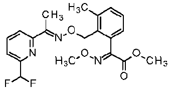 | 50 | 47 | 100 | 90 |
| 10 | 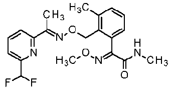 | 7 | 11 | 73 | 87 |
| 11 | 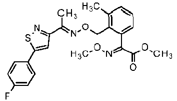 | 8 | 7 | 50 | 37 |
| 12 | 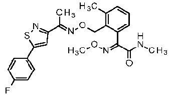 | 12 | 19 | 73 | 50 |
| 13 | 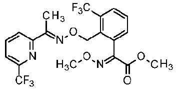 | 90 | 83 | 93 | 87 |
| 14 | 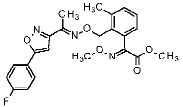 | 77 | 65 | 97 | 87 |
| 15 | 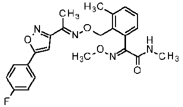 | 4 | 13 | 53 | 63 |
| 16 | 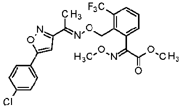 | 100 | 97 | 100 | 100 |
| 17 | 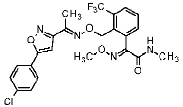 | 100 | 93 | 100 | 90 |
| 18 | 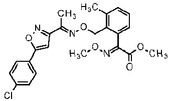 | 73 | 83 | 93 | 90 |
| 19 | 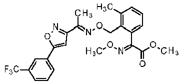 | 94 | 88 | 100 | 97 |
| 20 | 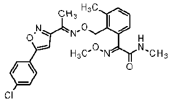 | 40 | 40 | 63 | 60 |
| 21 | 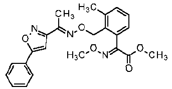 | 15 | 17 | 87 | 90 |
| 22 | 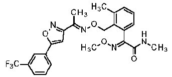 | 33 | 26 | 73 | 67 |
| 23 | 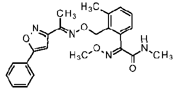 | 5 | 7 | 47 | 80 |
| 24 | 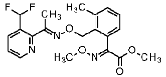 | 33 | 53 | 87 | 93 |
| 25 | 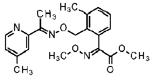 | 57 | 73 | 93 | 97 |
| 26 | 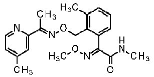 | 11 | 73 | 63 | 90 |
| 27 | 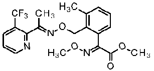 | 24 | 32 | 94 | 96 |
| 28 | 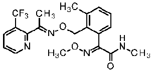 | 1 | 9 | 27 | 44 |
| 29 | 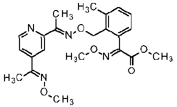 | 7 | 21 | 36 | 58 |
| 30 | 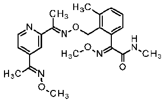 | 2 | 13 | 19 | 45 |
| 31 | 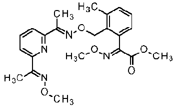 | 67 | 33 | 90 | 83 |
| 32 | 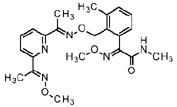 | 13 | 20 | 70 | 70 |
| 33 | 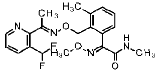 | 4.0 | 14 | 57 | 78 |
| 34 | 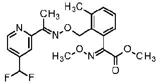 | 7 | 10 | 28 | 26 |
| 35 | 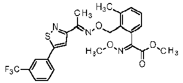 | 40 | 25 | 37 | 50 |
| 36 | 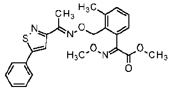 | 32 | 28 | 30 | 28 |
| 37 | 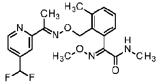 | 4 | 11 | 20 | 52 |
| 38 | 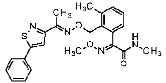 | 12 | 5 | 20 | 13 |
| 39 | 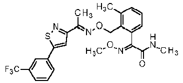 | 15 | 4 | 40 | 40 |
| 40 | 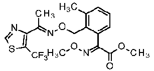 | 18 | 25 | 100 | 83 |
| 41 | 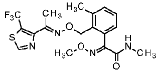 | 6 | 47 | 63 | 83 |
| 42 | 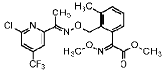 | 28 | 30 | 60 | 57 |
| 43 | 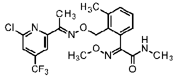 | 1 | 3 | 13 | 32 |
| 44 | 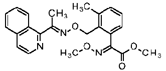 | 87 | 63 | 100 | 83 |
| 45 | 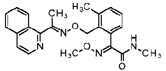 | 25 | 63 | 83 | 87 |
| 46 | 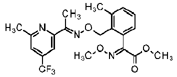 | 21 | 9 | 48 | 48 |
| 47 | 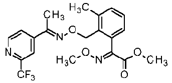 | 87 | 90 | 97 | 83 |
| 48 | 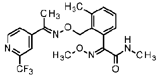 | 4 | 33 | 57 | 83 |
| 49 | 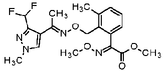 | 100 | 83 | 100 | 90 |
| 50 | 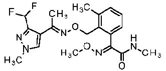 | 47 | 67 | 97 | 83 |
| 51 | 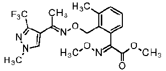 | 87 | 60 | 100 | 97 |
| 52 | 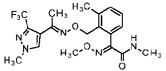 | 17 | 37 | 77 | 73 |
| 53 | 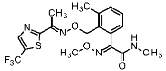 | 1 | 1 | 11 | 12 |
| 54 | 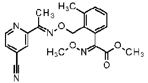 | 6 | 14 | 95 | 95 |
| 55 | 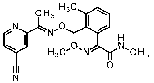 | 5 | 33 | 25 | 75 |
| 56 | 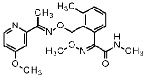 | 6 | 1 | 22 | 57 |
| 57 | 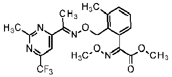 | 12 | 12 | 100 | 93 |
| 58 | 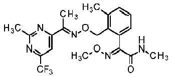 | 6 | 1 | 22 | 57 |
| 59 | 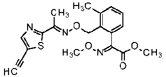 | 30 | 37 | 87 | 77 |
| 60 | 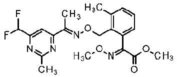 | 23 | 20 | 70 | 70 |
| 61 | 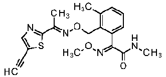 | 33 | 100 | 90 | 100 |
| 62 | 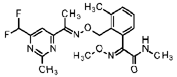 | 7 | 12 | 48 | 67 |
| 63 | 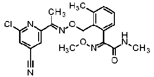 | 11 | 5 | 43 | 70 |
| 64 | 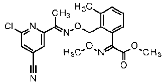 | 30 | 12 | 60 | 63 |
| 65 | 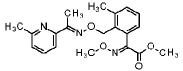 | 35 | 37 | 100 | 100 |
| 66 | 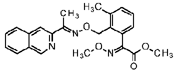 | 38 | 23 | 60 | 57 |
| 67 | 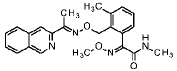 | 22 | 50 | 80 | 97 |
| 68 | 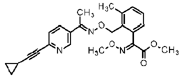 | 80 | 87 | 90 | 87 |
| 69 | 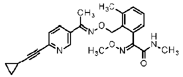 | 50 | 57 | 77 | 87 |
| 70 | 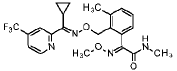 | 0 | 1 | 5 | 14 |
| 71 | 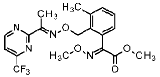 | 5 | 9 | 56 | 54 |
| 72 | 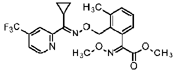 | 22 | 18 | 70 | 77 |
| 73 | 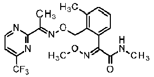 | 4 | 15 | 53 | 47 |
| 74 | 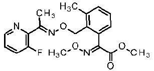 | 28 | 67 | 97 | 80 |
| 75 | 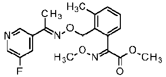 | 97 | 97 | 100 | 100 |
| 76 | 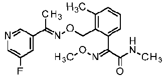 | 40 | 70 | 93 | 97 |
| 77 | 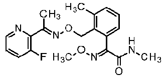 | 4 | 10 | 26 | 53 |
| 78 | 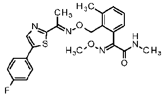 | 90 | 90 | 87 | 88 |
| 79 | 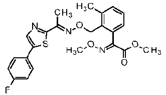 | 92 | 85 | 95 | 92 |
| 80 | 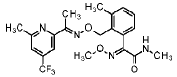 | 0 | 0 | 4 | 15 |
| 81 | 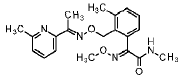 | 63 | 87 | 97 | 97 |
| 82 | 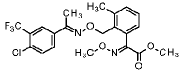 | 20 | 11 | 67 | 63 |
| 83 | 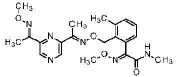 | 90 | 47 | 97 | 93 |
| 84 | 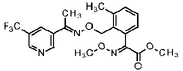 | 100 | 83 | 100 | 90 |
| 85 | 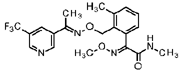 | 43 | 77 | 100 | 100 |
| 86 | 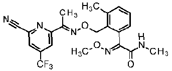 | 90 | 77 | 100 | 97 |
| 87 | 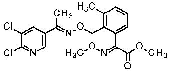 | 100 | 97 | 100 | 100 |
| 88 | 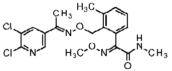 | 20 | 28 | 73 | 93 |
| 89 | 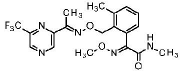 | 83 | 100 | 100 | 100 |
| 90 | 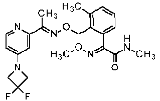 | 95 | 67 | 95 | 70 |
| 91 | 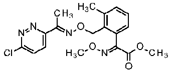 | 88 | 90 | 98 | 53 |
| 92 | 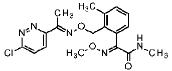 | 90 | 53 | 92 | 53 |
| 93 | 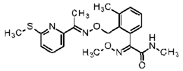 | 16 | 37 | 57 | 80 |
| 94 | 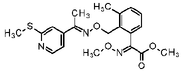 | 97 | 80 | 100 | 87 |
| 95 | 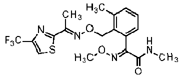 | 53 | 35 | 80 | 87 |
| 96 | 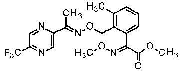 | 7 | 10 | 73 | 77 |
| 97 | 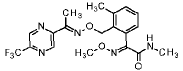 | 0 | 1 | 3 | 14 |
| 98 | 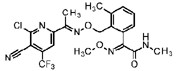 | 32 | 28 | 57 | 57 |
| 99 | 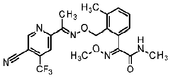 | 1 | 2 | 23 | 9 |
| 100 | 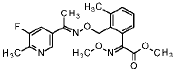 | 90 | 73 | 97 | 100 |
| 101 | 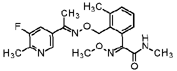 | 32 | 77 | 67 | 97 |
| 102 | 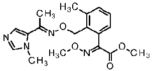 | 100 | 100 | 100 | 100 |
| 103 | 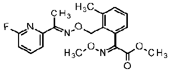 | 70 | 77 | 93 | 93 |
| 104 | 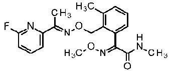 | 0 | 6 | 18 | 53 |
| 105 | 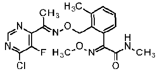 | 53 | 87 | 73 | 97 |
| 106 | 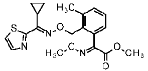 | 100 | 90 | 100 | 87 |
| 107 | 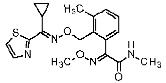 | 6 | 25 | 77 | 67 |
| 108 | 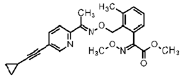 | 20 | 17 | 67 | 67 |
| 109 | 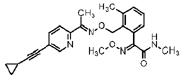 | 2 | 3 | 18 | 40 |
| 110 | 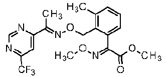 | 7 | 4 | 28 | 20 |
| 111 | 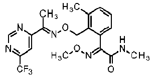 | 1 | 8 | 20 | 43 |
| 112 | 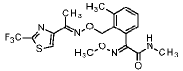 | 6 | 25 | 77 | 67 |
| 113 | 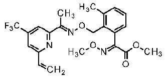 | 28 | 28 | 83 | 70 |
| 114 | 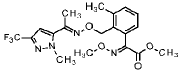 | 57 | 83 | 100 | 100 |
| 115 | 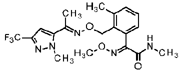 | 22 | 50 | 77 | 87 |
| 116 | 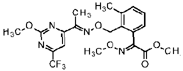 | 1 | 3 | 25 | 60 |
| 117 | 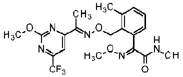 | 5 | 6 | 12 | 25 |
| 118 | 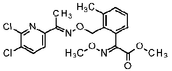 | 41 | 42 | 62 | 67 |
| 119 | 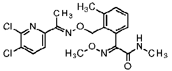 | 3 | 10 | 30 | 49 |
| 120 | 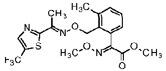 | 23 | 14 | 60 | 50 |
| 121 | 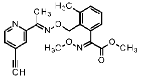 | 47 | 70 | 85 | 95 |
| 122 | 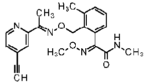 | 33 | 73 | 90 | 88 |
| 123 | 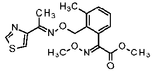 | 87 | 93 | 100 | 100 |
| 124 | 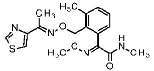 | 33 | 73 | 97 | 90 |
| 125 | 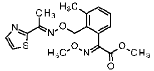 | 100 | 93 | 100 | 100 |
| 126 | 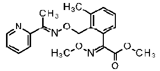 | 76 | 78 | 90 | 78 |
| 127 | 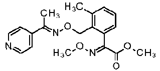 | 87 | 70 | 90 | 78 |
| 128 | 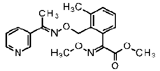 | 60 | 40 | 90 | 82 |
| 129 | 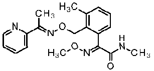 | 80 | 87 | 90 | 87 |
| 130 | 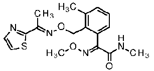 | 42 | 60 | 75 | 85 |
| 131 | 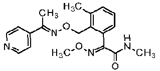 | 70 | 80 | 85 | 82 |
| 132 | 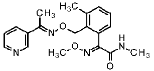 | 0.8 | 11 | 19 | 43 |
| 133 | 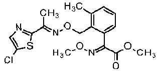 | 52 | 24 | 95 | 85 |
| 134 | 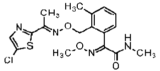 | 9 | 15 | 64 | 69 |
| 135 | 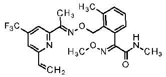 | 4 | 9 | 22 | 27 |
| 136 | 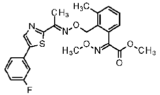 | 90 | 87 | 100 | 90 |
| 137 | 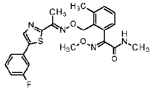 | 100 | 83 | 100 | 93 |
| 138 | 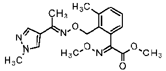 | 100 | 100 | 100 | 100 |
| 139 | 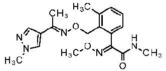 | 100 | 100 | 100 | 100 |
| 140 | 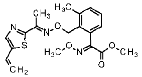 | 100 | 100 | 100 | 100 |
| 141 | 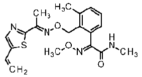 | 63 | 83 | 83 | 97 |
| 142 | 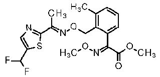 | 90 | 77 | 100 | 100 |
| 143 | 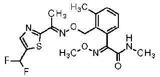 | 19 | 11 | 68 | 72 |
| 144 | 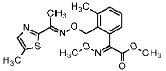 | 87 | 100 | 100 | 100 |
| 145 | 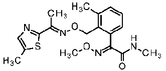 | 20 | 60 | 67 | 90 |
| 146 | 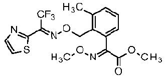 | 4 | 67 | 70 | 100 |
| 147 | 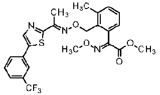 | 83 | 73 | 100 | 100 |
| 148 | 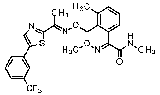 | 67 | 43 | 100 | 100 |
| 149 | 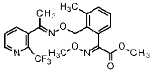 | 100 | 100 | 100 | 100 |
| 150 | 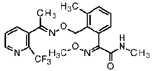 | 13 | 53 | 90 | 90 |
| 151 | 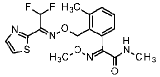 | 33 | 90 | 100 | 100 |
| 152 | 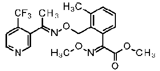 | 100 | 100 | 100 | 100 |
| 153 | 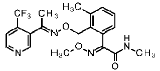 | 83 | 100 | 100 | 100 |
| 154 | 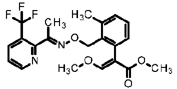 | 0 | 0 | 0 | 16 |
| 155 | 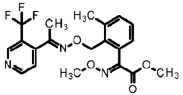 | 100 | 73 | 100 | 83 |
| 156 | 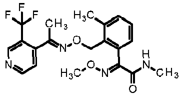 | 67 | 60 | 100 | 70 |
| 157 | 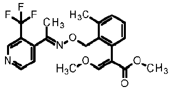 | 20 | 15 | 100 | 73 |
| 158 | 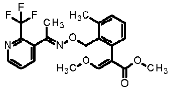 | 0 | 7 | 8 | 23 |
| 159 | 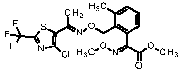 | 100 | 100 | 100 | 100 |
| 160 | 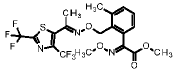 | 0 | 0 | 6 | 14 |
| 161 | 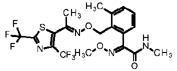 | 100 | 57 | 100 | 100 |
| 162 | 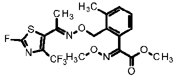 | 100 | 100 | 100 | 100 |
| 163 | 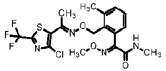 | 53 | 50 | 87 | 97 |

### B. Microtests

The active compounds were formulated separately as a stock solution having a concentration of 10000 ppm in dimethyl sulfoxide. The stock solutions were mixed according to the ratio, pipetted onto a micro titer plate (MTP) and diluted with water to the stated concentrations.

After the addition of the respective fungal spore suspensions as described below, the plates were placed in a water vapor-saturated chamber at a temperature of 18 °C. Using an absorption photometer, the MTPs were measured at 405 nm 7 days after the inoculation.

### Use example 3. Activity against the grey mold Botrytis cinerea (BOTRCI)

A spore suspension of *Botriytis cinerea* in an aqueous biomalt or yeast-bactopeptone-sodiumacetate solution was used.

### Use example 4. Activity against wheat leaf blotch pathogen Septoria tritici (SEPTTR)

A spore suspension of *Septoria tritici* in an aqueous biomalt or yeast-bactopeptone-glycerine or DOB solution was used.

### Use example 5. Activity against the late blight pathogen Phytophthora infestans (PHYTIN)

A spore suspension of *Phytophtora infestans* containing a pea juice-based aqueous nutrient medium or DDC medium was used.

### Use example 6. Activity against Fusarium culmorum (FUSACU)

A spore suspension of Fusarium culmorum in an aqueous biomalt or yeast-bactopeptone-glycerine or DOB solution was used.

The measured parameters were compared to the growth of the active compound-free control variant (100%) and the fungus-free blank value to determine the relative growth in % of the pathogens in the respective active compounds.

**Table 2:**

| **Treatment** | | **Fungal growth at 31 ppm (%)** | | | |
|---|---|---|---|---|---|
| **No.** | **Structure** | **BOTRCI** | **SEPTTR** | **PHYTIN** | **FUSACU** |
| 1 | 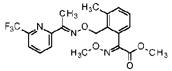 | 20 | 10 | | |
| 2 | 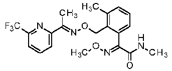 | | 12 | 18 | 7 |
| 3 | 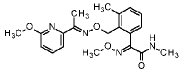 | 8 | 4 | 0 | 6 |
| 4 | 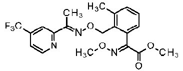 | | 11 | | 10 |
| 5 | 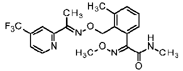 | | 19 | 20 | 8 |
| 6 | 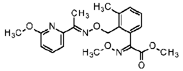 | | 7 | | |
| 7 | 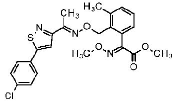 | | | | 11 |
| 8 | 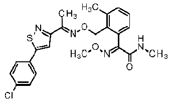 | | | 6 | |
| 9 | 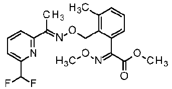 | | 4 | 5 | |
| 10 | 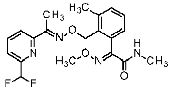 | | 2 | 0 | 9 |
| 11 | 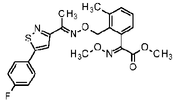 | | 10 | | |
| 12 | 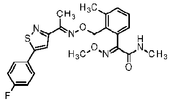 | | | 2 | 11 |
| 14 | 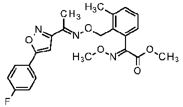 | | | | 20 |
| 15 | 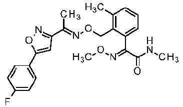 | | | 0 | 20 |
| 16 | 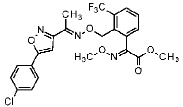 | | | | 19 |
| 17 | 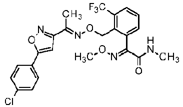 | | 1 | 0 | 14 |
| 18 | 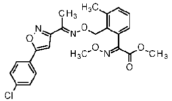 | | | | 16 |
| 19 | 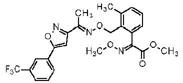 | | | | 20 |
| 20 | 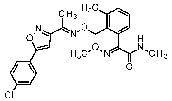 | | | 3 | 17 |
| 21 | 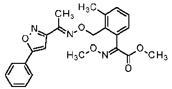 | | | 5 | |
| 22 | 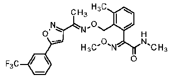 | | | 17 | |
| 23 | 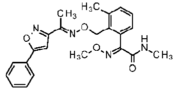 | | | 1 | 5 |
| 24 | 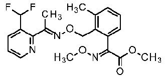 | | 0 | 18 | |
| 25 | 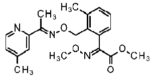 | | 0 | 0 | |
| 26 | 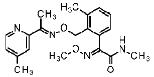 | | 7 | 0 | 12 |
| 27 | 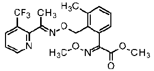 | | 5 | | |
| 29 | 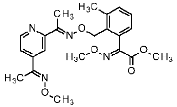 | 14 | 0 | 1 | 12 |
| 30 | 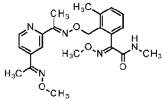 | 18 | 2 | 0 | 10 |
| 31 | 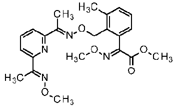 | | 13 | | |
| 33 | 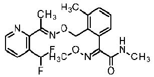 | | 4 | 14 | 11 |
| 34 | 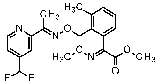 | | 0 | 1 | 10 |
| 35 | 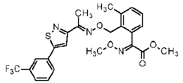 | | | | 20 |
| 36 | 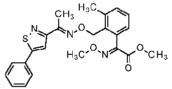 | | | 10 | |
| 37 | 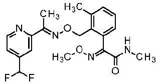 | | 3 | 0 | 12 |
| 38 | 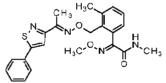 | | | 6 | 16 |
| 39 | 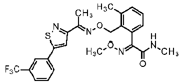 | | | 8 | 14 |
| 40 | 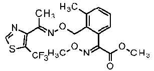 | | 0 | 8 | |
| 41 | 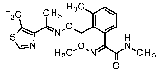 | | 2 | 0 | 9 |
| 42 | 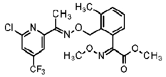 | | | | 10 |
| 43 | 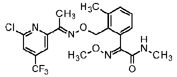 | | 13 | 9 | 14 |
| 44 | 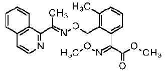 | | 2 | 15 | |
| 45 | 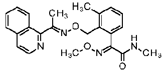 | | 6 | 0 | 4 |
| 46 | 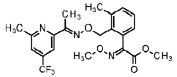 | 0 | 0 | 0 | 1 |
| 47 | 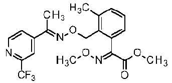 | | 0 | 0 | 8 |
| 48 | 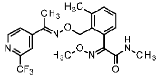 | | | 1 | 17 |
| 49 | 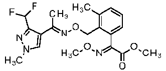 | | | 0 | 17 |
| 50 | 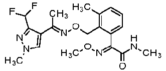 | | | 0 | 18 |
| 51 | 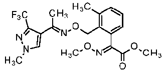 | | 3 | | 8 |
| 52 | 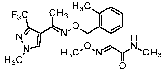 | | | | 12 |
| 53 | 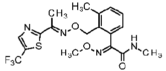 | | | | 21 |
| 54 | 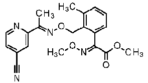 | | 4 | 2 | 14 |
| 55 | 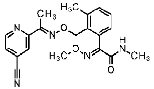 | | 8 | 0 | 12 |
| 56 | 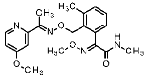 | | | 0 | 11 |
| 57 | 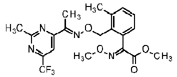 | | 14 | 2 | 12 |
| 58 | 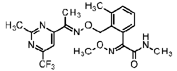 | | 21 | 11 | 17 |
| 61 | 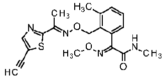 | | | 19 | 11 |
| 62 | 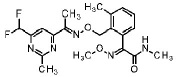 | | | 0 | 11 |
| 63 | 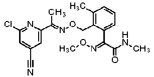 | | | 0 | 18 |
| 64 | 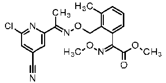 | | 17 | 21 | |
| 65 | 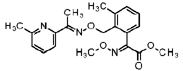 | | 0 | 1 | |
| 67 | 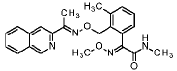 | | 5 | 0 | 9 |
| 68 | 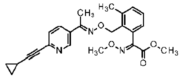 | | | 3 | 13 |
| 69 | 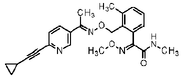 | | | 0 | 11 |
| 70 | 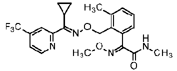 | | 0 | 0 | 12 |
| 71 | 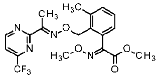 | | 0 | 0 | |
| 72 | 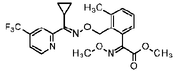 | | 4 | 6 | 14 |
| 73 | 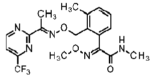 | | 21 | 1 | 13 |
| 74 | 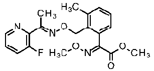 | | 21 | 0 | |
| 75 | 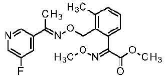 | | | 0 | |
| 76 | 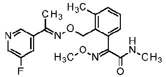 | | | 0 | 16 |
| 77 | 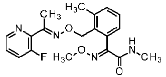 | | | 0 | 11 |
| 78 | 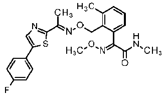 | | | 3 | 16 |
| 79 | 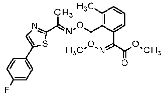 | | | 17 | 13 |
| 80 | 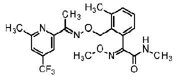 | | | 0 | 16 |
| 81 | 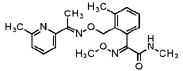 | | | 0 | 9 |
| 82 | 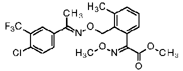 | | | 3 | 17 |
| 83 | 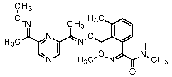 | | | | 21 |
| 84 | 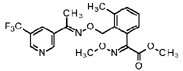 | | 1 | 0 | 12 |
| 85 | 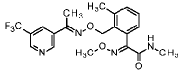 | | | 0 | 16 |
| 86 | 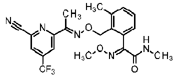 | | | 0 | 16 |
| 87 | 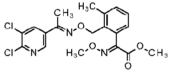 | | 1 | 0 | 17 |
| 88 | 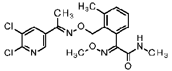 | | 9 | 0 | 14 |
| 89 | 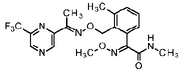 | | | 0 | 13 |
| 90 | 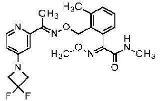 | | | 0 | |
| 91 | 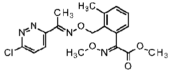 | | | 0 | 16 |
| 92 | 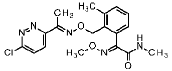 | | | 0 | 17 |
| 93 | 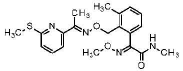 | | 13 | 0 | 17 |
| 94 | 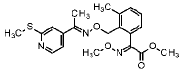 | | 1 | 1 | 15 |
| 95 | 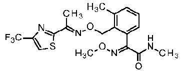 | | | | 17 |
| 96 | 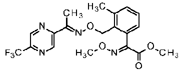 | | 18 | 0 | 13 |
| 97 | 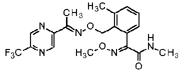 | | | 0 | 17 |
| 99 | 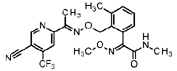 | | | 20 | |
| 100 | 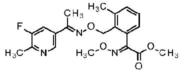 | | 19 | 0 | 12 |
| 101 | 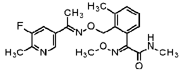 | | | 0 | 15 |
| 102 | 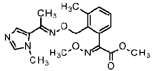 | | | 0 | |
| 103 | 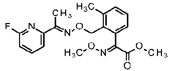 | | 13 | 0 | |
| 104 | 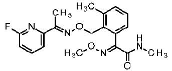 | | | 0 | 16 |
| 105 | 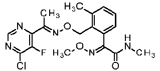 | | 0 | 0 | 8 |
| 108 | 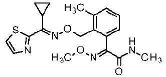 | | | 0 | |
| 109 | 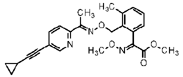 | | | 15 | |
| 110 | 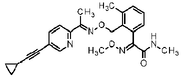 | | | 2 | 21 |
| 111 | 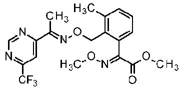 | 0 | 0 | 0 | 1 |
| 112 | 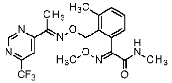 | 0 | 0 | 0 | 1 |
| 113 | 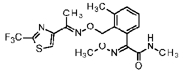 | | | 0 | 13 |
| 115 | 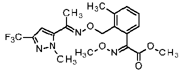 | 18 | | | 18 |
| 116 | 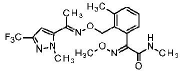 | | 0 | 18 | |
| 117 | 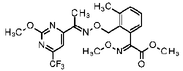 | 0 | 0 | 9 | 0 |
| 119 | 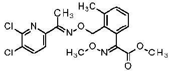 | 6 | 0 | 3 | |
| 120 | 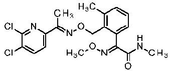 | 0 | 0 | 0 | 1 |
| 121 | 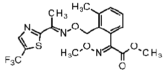 | | | | 17 |
| 122 | 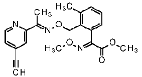 | | 12 | 0 | 10 |
| 123 | 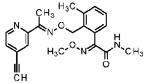 | | 6 | 0 | 11 |
| 124 | 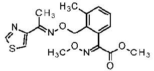 | | | 0 | |
| 125 | 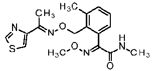 | | | 0 | |
| 127 | 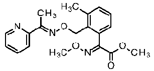 | | | 0 | |
| 128 | 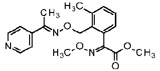 | | | 0 | 17 |
| 129 | 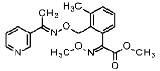 | | 17 | 0 | |
| 130 | 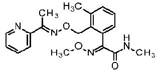 | | | 0 | 16 |
| 132 | 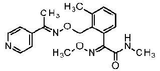 | | | 0 | 20 |
| 133 | 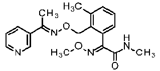 | | | 0 | 19 |
| 134 | 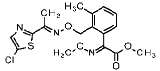 | | | | 13 |
| 135 | 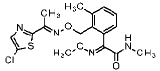 | | | | 15 |
| 139 | 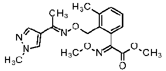 | | 0 | 17 | 5 |
| 142 | 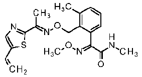 | | | | 14 |
| 144 | 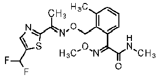 | | | | 20 |
| 152 | 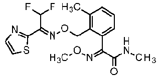 | | | | 14 |
| 153 | 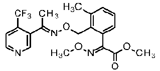 | | | | 18 |

### Comparative trials

**Table C1:**

| | | **PHAKPA (F129L) Disease level (%)** | | | |
|---|---|---|---|---|---|
| **Compound** | **Structure** | **P2 at 4 ppm** | **P2 at 16 ppm** | **P6 at 4 ppm** | **P6 at 16 ppm** |
| Trifloxystrobin as comparative example | 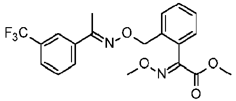 | 97 | 37 | 100 | 53 |
| Ex. 4 | 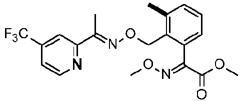 | **9** | **0** | **12** | **0** |

The results in Table C1 show that the specific substituent at position R³ together with the terminal heteroaryl ring improves the fungicidal activity against phytopathogenic fungi containing the amino acid substitution F129L in the mitochondrial cytochrome b protein conferring resistance to Qo inhibitors compared to trifloxystrobin where the position R³ is unsubstituted and the terminal ring is phenyl.

## Claims

1. Non-therapeutic use of compounds of formula I 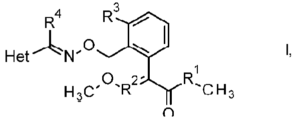 wherein
R¹ is selected from O and NH;
R² is selected from CH and N;
R³ is selected from halogen, CN, C₁-C₄-alkyl, C₂-C₄-alkenyl, C₂-C₄-alkynyl, C₁-C₄-haloalkyl, C₂-C₄-haloalkenyl, C₂-C₄-haloalkynyl, C₃-C₆-cycloalkyl, -O-C₁-C₄-alkyl, -O-C₁-C₄-haloalkyl, -O-C₃-C₆-cycloalkyl, -C₁-C₂-alkyl-C₃-C₆-cycloalkyl, phenyl, 3- to 6-membered heterocycloalkyl and 5- or 6-membered heteroaryl,
wherein said heterocycloalkyl and heteroaryl besides carbon atoms contain 1, 2 or 3 heteroatoms selected from N, O and S, provided that such heterocycloalkyl and heteroaryl cannot contain 2 contiguous atoms selected from O and S;
wherein said phenyl, heterocycloalkyl and heteroaryl are bound directly or via an oxygen atom or via a C₁-C₂-alkylene linker, and wherein said phenyl and heteroaryl are unsubstituted or substituted by 1, 2 or 3 identical or different substituents selected from halogen, CN, NH₂, NO₂, C₁-C₄-alkyl, C₁-C₄-haloalkyl, -O-C₁-C₄-alkyl and -O-C₁-C₄-haloalkyl;
R⁴ is selected from C₁-C₆-alkyl, C₂-C₄-alkenyl, C₂-C₄-alkynyl, C₁-C₆-haloalkyl, C₂-C₄-haloalkenyl, C₂-C₄-haloalkynyl, O-C₁-C₄-alkyl, -C(=O)-C₁-C₄-alkyl, -(C₁-C₂-alkyl)-O-(C₁-C₂-alkyl), -(C₁-C₂-alkyl)-O-(C₁-C₂-haloalkyl), C₃-C₆-cycloalkyl, C₃-C₆-halocycloalkyl and -C₁-C₄-alkyl-C₃-C₆-cycloalkyl;
Het is 5- or 6-membered heteroaryl, wherein said heteroaryl besides carbon atoms contains 1, 2 or 3 heteroatoms selected from N, O and S, provided that such heteroaryl cannot contain 2 contiguous atoms selected from O and S;
wherein said heteroaryl is unsubstituted or carries 1, 2, 3 or up to the maximum number of identical or different groups R^{a}:
R^{a} is selected from halogen, CN, -NR⁵R⁶, C₁-C₄-alkyl, C₂-C₄-alkenyl, C₂-C₄-alkynyl, -O-C₁-C₄-alkyl, -C(=N-O-C₁-C₄-alkyl)-C₁-C₄-alkyl, -C(=O)-C₁-C₄-alkyl, -O-CH₂-C(=N-O-C₁-C₄-alkyl)-C₁-C₄-alkyl, C₃-C₆-cycloalkyl, C₃-C₆-cycloalkenyl, -C₁-C₂-alkyl-C₃-C₆-cycloalkyl, -O-C₃-C₆-cycloalkyl, phenyl, 3- to 6-membered heterocycloalkyl, 3- to 6-membered heterocycloalkenyl and 5- or 6-membered heteroaryl,
wherein said heterocycloalkyl, heterocycloalkenyl and heteroaryl besides carbon atoms contain 1, 2 or 3 heteroatoms selected from N. O and S, provided that such heterocycloalkyl, heterocycloalkenyl and heteroaryl cannot contain 2 contiguous atoms selected from O and S;
and/or
2 R^{a} substituents bound to neighboring carbon ring atoms, together with the two interjacent carbon ring atoms, form a partially unsaturated or aromatic 5- to 6-membered fused carbo- or heterocycle,
wherein the heterocycle includes beside carbon atoms 1 or 2 heteroatoms independently selected from N, O and S as ring member atoms, provided that such heterocycle cannot contain 2 contiguous atoms selected from O and S;
and wherein the aliphatic and cyclic moieties of R^{a} and the abovementioned fused carbo- or heterocycle are unsubstituted or carry 1, 2, 3, 4 or up to the maximum number of identical or different groups R^{b}:
R^{b} is selected from halogen, CN, NH₂, NO₂, C₁-C₄-alkyl, C₁-C₄-haloalkyl, -O-C₁-C₄-alkyl, -O-C₁-C₄-haloalkyl and C₃-C₆-cycloalkyl;
R⁵, R⁶ are independently of each other selected from the group consisting of H, C₁-C₆-alkyl, C₁-C₆-haloalkyl and C₂-C₄-alkynyl;
and in form of stereoisomers and tautomers thereof, and the N-oxides and the agriculturally acceptable salts thereof, for combating phytopathogenic fungi containing an amino acid substitution F129L in the mitochondrial cytochrome b protein conferring resistance to Qo inhibitors.

2. The use according to claim 1, wherein in formula I R¹ is selected from O and NH; and R² is selected from CH and N, provided that R² is N in case R' is NH.

3. The use according to claim 1 or claim 2, wherein in formula I R³ is selected from C₁-C₂-alkyl, C₁-C₂-haloalkyl, C₃-C₄-cycloalkyl, -O-C₁-C₂-alkyl and -O-C₁-C₂-haloalkyl.

4. The use according to any of claims 1 to 3, wherein in formula I R⁴ is selected from C₁-C₄-alkyl, C₂-C₄-alkenyl, -C(=O)-C₁-C₂-alkyl, C₁-C₄-haloalkyl, C₂-C₄-haloalkenyl and -(C₁-C₂-alkyl)-O-(C₁-C₂-alkyl).

5. The use according to any one of claims 1 to 4, wherein in formula I Het is pyridyl or thiazolyl, wherein said pyridyl or thiazolyl is unsubstituted or carries 1, 2 or 3 identical or different groups R^{a} as defined in claim 1.

6. The use according to any one of claims 1 to 5, wherein in formula I R^{a} is selected from is selected from C₁-C₃-alkyl, C₂-C₃-alkenyl, C₂-C₃-alkynyl, -O-C₁-C₃-alkyl,
-C(=N-O-C₁-C₂-alkyl)-C₁-C₂-alkyl, -O-CH₂-C(=N-O-C₁-C₂-alkyl)-C₁-C₂-alkyl, C₃-C₄-cycloalkyl, -C₁-C₂-alkyl-C₃-C₄-cycloalkyl, -O-C₃-C₄-cycloalkyl, phenyl, 3- to 5-membered heterocycloalkyl and 5- or 6-membered heteroaryl, wherein said heterocycloalkyl and heteroaryl besides carbon atoms contain 1 or 2 heteroatoms selected from N, O and S, provided that such heterocycloalkyl and heteroaryl cannot contain 2 contiguous atoms selected from O and S; and/or 2 R^{a} substituents bound to neighboring carbon ring atoms, together with the two interjacent carbon ring atoms, form a fused phenyl ring, and wherein the aliphatic and cyclic moieties of R^{a} and the abovementioned fused phenyl ring are unsubstituted or carry 1, 2, 3, 4 or up to the maximum number of identical or different groups R^{b} which independently of one another are selected from halogen, CN, methyl and C₁-haloalkyl.

7. A non-therapeutic method for combating phytopathogenic fungi containing an amino acid substitution F129L in the mitochondrial cytochrome b protein conferring resistance to Qo inhibitors, comprising:
treating curatively and/or preventively the plants or the plant propagation material of said plants that are at risk of being diseased from the said phytopathogenic fungi, and/or applying to the said phytopathogenic fungi with an effective amount of at least one compound of formula I as defined in any of claims 1 to 6 or a composition comprising it thereof.

8. Compounds of formula I 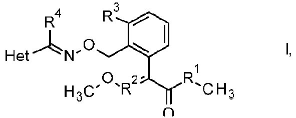 wherein
R¹ is selected from O and NH;
R² is selected from CH and N;
R³ is selected from halogen, CN, C₁-C₄-alkyl, C₂-C₄-alkenyl, C₂-C₄-alkynyl, C₁-C₄-haloalkyl, C₂-C₄-haloalkenyl, C₂-C₄-haloalkynyl, C₃-C₆-cycloalkyl, -O-C₁-C₄-alkyl, -O-C₁-C₄-haloalkyl, -O-C₃-C₆-cycloalkyl, -C₁-C₂-alkyl-C₃-C₆-cycloalkyl, phenyl, 3- to 6-membered heterocycloalkyl and 5- or 6-membered heteroaryl,
wherein said heterocycloalkyl and heteroaryl besides carbon atoms contain 1, 2 or 3 heteroatoms selected from N, O and S, provided that such heterocycloalkyl and heteroaryl cannot contain 2 contiguous atoms selected from O and S;
wherein said phenyl, heterocycloalkyl and heteroaryl are bound directly or via an oxygen atom or via a C₁-C₂-alkylene linker, and wherein said phenyl and heteroaryl are unsubstituted or substituted by 1, 2 or 3 identical or different substituents selected from halogen, CN, NH₂, NO₂, C₁-C₄-alkyl, C₁-C₄-haloalkyl, -O-C₁-C₄-alkyl and -O-C₁-C₄-haloalkyl;
R⁴ is selected from C₁-C₆-alkyl, C₂-C₄-alkenyl, C₂-C₄-alkynyl, C₁-C₆-haloalkyl, C₂-C₄-haloalkenyl, C₂-C₄-haloalkynyl, O-C₁-C₄-alkyl, -C(=O)-C₁-C₄-alkyl, -(C₁-C₂-alkyl)-O-(C₁-C₂-alkyl), -(C₁-C₂-alkyl)-O-(C₁-C₂-haloalkyl), C₃-C₆-cycloalkyl, C₃-C₆-halocycloalkyl and -C₁-C₄-alkyl-C₃-C₆-cycloalkyl;
Het is 5- or 6-membered heteroaryl, wherein said heteroaryl besides carbon atoms contain 1, 2 or 3 heteroatoms selected from N, O and S, provided that such heteroaryl cannot contain 2 contiguous atoms selected from O and S;
wherein said heteroaryl is unsubstituted or carries 1, 2, 3 or up to the maximum number of identical or different groups R^{a}:
R^{a} is selected from halogen, CN, -NR⁵R⁶, C₁-C₄-alkyl, C₂-C₄-alkenyl, C₂-C₄-alkynyl,
-O-C₁-C₄-alkyl, -C(=N-O-C₁-C₄-alkyl)-C₁-C₄-alkyl, -C(=O)-C₁-C₄-alkyl, -O-CH₂-C(=N-O-C₁-C₄-alkyl)-C₁-C₄-alkyl, C₃-C₆-cycloalkyl, C₃-C₆-cycloalkenyl, -C₁-C₂-alkyl-C₃-C₆-cycloalkyl, -O-C₃-C₆-cycloalkyl, phenyl, 3- to 6-membered heterocycloalkyl, 3- to 6-membered heterocycloalkenyl and 5- or 6-membered heteroaryl,
wherein said heterocycloalkyl, heterocycloalkenyl and heteroaryl besides carbon atoms contain 1, 2 or 3 heteroatoms selected from N, O and S, provided that such heterocycloalkyl, heterocycloalkenyl and heteroaryl cannot contain 2 contiguous atoms selected from O and S;
and/or
2 R^{a} substituents bound to neighboring carbon ring atoms, together with the two interjacent carbon ring atoms, form a partially unsaturated or aromatic 5- to 6-membered fused carbo- or heterocycle,
wherein the heterocycle includes beside carbon atoms 1 or 2 heteroatoms independently selected from N, O and S as ring member atoms, provided that such heterocycle cannot contain 2 contiguous atoms selected from O and S; and wherein the aliphatic and cyclic moieties of R^{a} and the abovementioned fused carbo- or heterocycle are unsubstituted or carry 1, 2, 3, 4 or up to the maximum number of identical or different groups R^{b}:
R^{b} is selected from halogen, CN, NH₂, NO₂, C₁-C₄-alkyl, C₁-C₄-haloalkyl, -O-C₁-C₄-alkyl and -O-C₁-C₄-haloalkyl;
R⁵, R⁶ are independently of each other selected from the group consisting of H, C₁-C₆-alkyl, C₁-C₆-haloalkyl and C₂-C₄-alkynyl;
and in form of stereoisomers and tautomers thereof, and the N-oxides and the agriculturally acceptable salts thereof.

9. The compounds according to claim 8, wherein R¹ is selected from O and NH; and R² is selected from CH and N, provided that R² is N in case R¹ is NH.

10. The compounds according to any one of the claims 8 to 9, wherein R³ is selected from CN, C₁-C₂-alkyl, C₁-C₂-haloalkyl, C₃-C₄-cycloalkyl, -O-C₁-C₂-alkyl and -O-C₁-C₂-haloalkyl.

11. The compounds according to any one of the claims 8 to 10, wherein R⁴ is selected from C₁-C₆-alkyl, C₂-C₄-alkenyl, C₁-C₆-haloalkyl, C₂-C₄-haloalkenyl, -(C₁-C₂-alkyl)-O-(C₁-C₂-alkyl) and -(C₁-C₂-alkyl)-O-(C₁-C₂-haloalkyl).

12. The compounds according to any one of claims 8 to 11, wherein Het is pyridyl or thiazolyl, wherein said pyridyl or thiazolyl is unsubstituted or carries 1, 2 or 3 identical or different groups R^{a} as defined in claim 8

13. Agrochemical compositions comprising an auxiliary and at least one compound of formula I, as defined in any of claims 8 to 12 or in the form of a stereoisomer or an agriculturally acceptable salt or a tautomer or N-oxide thereof.

14. The non-therapeutic use of at least one compound of formula I as defined in any of the claims 8 to 12 or of an agrochemical composition as defined in claim 13 for combating phytopathogenic fungi.

15. A non-therapeutic method for combating phytopathogenic fungi comprising: treating curatively and/or preventively the plants or the plant propagation material of said plants that are at risk of being diseased from the said phytopathogenic fungi, and/or applying to the said phytopathogenic fungi, at least one compound of formula I as defined in any of the claims 8 to 12 or an agrochemcial composition as defined in claim 13.

## Patentansprüche

1. Nichttherapeutische Verwendung von Verbindungen der Formel I 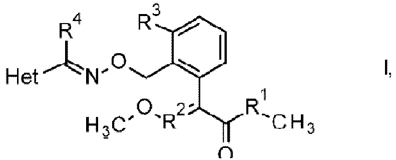 wobei
R¹ ausgewählt ist aus O und NH;
R² ausgewählt ist aus CH und N;
R³ ausgewählt ist aus Halogen, CN, C₁-C₄-Alkyl, C₂-C₄-Alkenyl, C₂-C₄-Alkinyl, C₁-C₄-Halogenalkyl, C₂-C₄-Halogenalkenyl, C₂-C₄-Halogenalkinyl, C₃-C₆-Cycloalkyl, - O-C₁-C₄-Alkyl, -O-C₁-C₄-Halogenalkyl, -O-C₃-C₆-Cycloalkyl, -C₁-C₂-Alkyl-C₃-C₆-cycloalkyl, Phenyl, 3- bis 6-gliedrigem Heterocycloalkyl und 5- oder 6-gliedrigem Heteroaryl;
wobei das Heterocycloalkyl und Heteroaryl neben Kohlenstoffatomen 1, 2 oder 3 Heteroatome ausgewählt aus N, O und S enthalten, mit der Maßgabe, dass ein solches Heterocycloalkyl und Heteroaryl nicht 2 benachbarte Atome ausgewählt aus O und S enthalten kann;
wobei das Phenyl, Heterocycloalkyl und Heteroaryl direkt oder über ein Sauerstoffatom oder über einen C₁-C₂-Alkylen-Linker gebunden sind, und wobei das Phenyl und Heteroaryl unsubstituiert oder durch 1, 2 oder 3 gleiche oder verschiedene Substituenten ausgewählt aus Halogen, CN, NH₂, NO₂, C₁-C₄-Alkyl, C₁-C₄-Halogenalkyl, -O-C₁-C₄-Alkyl und -O-C₁-C₄-Halogenalkyl substituiert sind;
R⁴ ausgewählt ist aus C₁-C₆-Alkyl, C₂-C₄-Alkenyl, C₂-C₄-Alkinyl, C₁-C₆-Halogenalkyl, C₂-C₄-Halogenalkenyl, C₂-C₄-Halogenalkinyl, O-C₁-C₄-Alkyl, -C(=O)-C₁-C₄-Alkyl, -(C₁-C₂-Alkyl)-O-(C₁-C₂-alkyl), -(C₁-C₂-Alkyl)-O-(C₁-C₂-halogenalkyl), C₃-C₆-Cycloalkyl, C₃-C₆-Halogencycloalkyl und -C₁-C₄-Alkyl-C₃-C₆-cycloalkyl;
Het 5- oder 6-gliedriges Heteroaryl ist, wobei das Heteroaryl neben Kohlenstoffatomen 1, 2 oder 3 Heteroatome ausgewählt aus N, O und S enthält, mit der Maßgabe, dass ein solches Heteroaryl nicht 2 benachbarte Atome ausgewählt aus O und S enthalten kann;
wobei das Heteroaryl unsubstituiert ist oder 1, 2, 3 oder bis zur maximalen Anzahl gleiche oder verschiedene R^{a}-Gruppen trägt:
R^{a} ausgewählt ist aus Halogen, CN, -NR⁵R⁶, C₁-C₄-Alkyl, C₂-C₄-Alkenyl, C₂-C₄-Alkinyl, -O-C₁-C₄-Alkyl, -C(=N-O-C₁-C₄-Alkyl)-C₁-C₄-alkyl, -C(=O)-C₁-C₄-Alkyl, -O-CH₂-C(=N-O-C₁-C₄-Alkyl)-C₁-C₄-alkyl, C₃-C₆-Cycloalkyl, C₃-C₆-Cycloalkenyl, -C₁-C₂-Alkyl-C₃-C₆-cycloalkyl, -O-C₃-C₆-Cycloalkyl, Phenyl, 3- bis 6-gliedrigem Heterocycloalkyl, 3- bis 6-gliedrigem Heterocycloalkenyl und 5- oder 6-gliedrigem Heteroaryl;
wobei das Heterocycloalkyl, Heterocycloalkenyl und Heteroaryl neben Kohlenstoffatomen 1, 2 oder 3 Heteroatome ausgewählt aus N, O und S enthalten, mit der Maßgabe, dass ein solches Heterocycloalkyl, Heterocycloalkenyl oder Heteroaryl nicht 2 benachbarte Atome ausgewählt aus O und S enthalten kann;
und/oder
2 an benachbarte Kohlenstoffringatome gebundene R^{a}-Substituenten zusammen mit den beiden dazwischenliegenden Kohlenstoffringatomen einen teilweise ungesättigten oder aromatischen 5- bis 6-gliedrigen kondensierten Carbo- oder Heterocyclus bilden;
wobei der Heterocyclus neben Kohlenstoffatomen 1 oder 2 Heteroatome unabhängig voneinander ausgewählt aus N, O und S enthält, mit der Maßgabe, dass ein solcher Heterocyclus nicht 2 benachbarte Atome ausgewählt aus O und S enthalten kann;
und wobei die aliphatischen und cyclischen Anteile von R^{a} und der genannte kondensierte Carbo- oder Heterocyclus unsubstituiert sind oder 1, 2, 3, 4 oder bis zur maximalen Anzahl gleiche oder verschiedene R^{b}-Gruppen tragen:
R^{b} ausgewählt ist aus Halogen, CN, NH₂, NO₂, C₁-C₄-Alkyl, C₁-C₄-Halogenalkyl, -O-C₁-C₄-Alkyl, -O-C₁-C₄-Halogenalkyl und C₃-C₆-Cycloalkyl;
R⁵, R⁶ unabhängig voneinander ausgewählt sind aus der Gruppe bestehend aus H, C₁-C₆-Alkyl, C₁-C₆-Halogenalkyl und C₂-C₄-Alkinyl;
und in Form von Stereoisomeren und Tautomeren davon und den N-Oxiden und den landwirtschaftlich unbedenklichen Salzen davon zur Bekämpfung phytopathogener Pilze, die eine Aminosäuresubstitution F129L im mitochondrialen Cytochrom-b-Protein enthalten, die Resistenz gegen Qo-Inhibitoren verleiht.

2. Verwendung nach Anspruch 1, wobei in Formel I R¹ ausgewählt ist aus O und NH; und R² ausgewählt ist aus CH und N, mit der Maßgabe, dass R² N ist, wenn R^{l} NH ist.

3. Verwendung nach Anspruch 1 oder Anspruch 2, wobei in Formel I R³ ausgewählt ist aus C₁-C₂-Alkyl, C₁-C₂-Halogenalkyl, C₃-C₄-Cycloalkyl, -O-C₁-C₂-Alkyl und -O-C₁-C₂-Halogenalkyl.

4. Verwendung nach einem der Ansprüche 1 bis 3, wobei in Formel I R⁴ ausgewählt ist aus C₁-C₄-Alkyl, C₂-C₄-Alkenyl, -C(=O)-C₁-C₂-Alkyl, C₁-C₄-Halogenalkyl, C₂-C₄-Halogenalkenyl und -(C₁-C₂-Alkyl)-O-(C₁-C₂-alkyl).

5. Verwendung nach einem der Ansprüche 1 bis 4, wobei in Formel I Het Pyridyl oder Thiazolyl ist, wobei das Pyridyl oder Thiazolyl unsubstituiert ist oder 1, 2 oder 3 gleiche oder verschiedene Gruppen R^{a} trägt, wie in Anspruch 1 definiert.

6. Verwendung nach einem der Ansprüche 1 bis 5, wobei in Formel I R^{a} ausgewählt ist aus C₁-C₃-Alkyl, C₂-C₃-Alkenyl, C₂-C₃-Alkinyl, -O-C₁-C₃-Alkyl, -C (=N-O-C₁-C₂-Alkyl) -C₁-C₂-alkyl, -O-CH₂-C(=N-O-C₁-C₂-Alkyl)-C₁-C₂-alkyl, C₃-C₄-Cycloalkyl, -C₁-C₂-Alkyl-C₃-C₄-cycloalkyl, - O-C₃-C₄-Cycloalkyl, Phenyl, 3- bis 5-gliedrigem Heterocycloalkyl und 5- oder 6-gliedrigem Heteroaryl, wobei das Heterocycloalkyl und das Heteroaryl neben Kohlenstoffatomen 1 oder 2 Heteroatome ausgewählt aus N, O und S enthalten, mit der Maßgabe, dass solches Heterocycloalkyl und Heteroaryl nicht 2 benachbarte Atome ausgewählt aus O und S enthält; und/oder 2 an benachbarte Kohlenstoffringatome gebundene R^{a}-Substituenten zusammen mit den beiden dazwischenliegenden Kohlenstoffringatomen einen kondensierten Phenylring bilden, und wobei die aliphatischen und cyclischen Anteile von R^{a} und der genannte kondensierte Phenylring unsubstituiert sind oder 1, 2, 3, 4 oder bis zur maximalen Anzahl von gleichen oder verschiedenen Gruppen R^{b} tragen, die unabhängig voneinander ausgewählt sind aus Halogen, CN, Methyl und C₁-Halogenalkyl.

7. Nichttherapeutisches Verfahren zur Bekämpfung phytopathogener Pilze, die eine Aminosäuresubstitution F129L in dem mitochondrialen Cytochrom-b-Protein enthalten, das Resistenz gegen Qo-Inhibitoren verleiht, umfassend:
kuratives und/oder präventives Behandeln der Pflanzen oder des Pflanzenfortpflanzungsmaterials der Pflanzen, bei denen ein Risiko einer Erkrankung durch die phytopathogenen Pilze besteht, mit einer effektiven Menge von mindestens einer Verbindung der Formel I wie in einem der Ansprüche 1 bis 6 definiert oder einer Zusammensetzung, die diese umfasst, und/oder Aufbringen derselben auf die phytopathogenen Pilze.

8. Verbindungen der Formel I 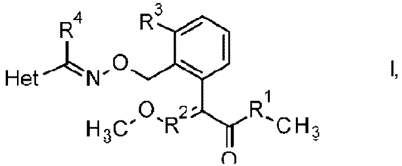 wobei
R¹ ausgewählt ist aus O und NH;
R² ausgewählt ist aus CH und N;
R³ ausgewählt ist aus Halogen, CN, C₁-C₄-Alkyl, C₂-C₄-Alkenyl, C₂-C₄-Alkinyl, C₁-C₄-Halogenalkyl, C₂-C₄-Halogenalkenyl, C₂-C₄-Halogenalkinyl, C₃-C₆-Cycloalkyl, - O-C₁-C₄-Alkyl, -O-C₁-C₄-Halogenalkyl, -O-C₃-C₆-Cycloalkyl, -C₁-C₂-Alkyl-C₃-C₆-cycloalkyl, Phenyl, 3- bis 6-gliedrigem Heterocycloalkyl und 5- oder 6-gliedrigem Heteroaryl;
wobei das Heterocycloalkyl und Heteroaryl neben Kohlenstoffatomen 1, 2 oder 3 Heteroatome ausgewählt aus N, O und S enthalten, mit der Maßgabe, dass ein solches Heterocycloalkyl und Heteroaryl nicht 2 benachbarte Atome ausgewählt aus O und S enthalten kann;
wobei das Phenyl, Heterocycloalkyl und Heteroaryl direkt oder über ein Sauerstoffatom oder über einen C₁-C₂-Alkylen-Linker gebunden sind, und wobei das Phenyl und Heteroaryl unsubstituiert oder durch 1, 2 oder 3 gleiche oder verschiedene Substituenten ausgewählt aus Halogen, CN, NH₂, NO₂, C₁-C₄-Alkyl, C₁-C₄Halogenalkyl, -O-C₁-C₄-Alkyl und -O-C₁-C₄-Halogenalkyl substituiert sind;
R⁴ ausgewählt ist aus C₁-C₆-Alkyl, C₂-C₄-Alkenyl, C₂-C₄-Alkinyl, C₁-C₆-Halogenalkyl, C₂-C₄-Halogenalkenyl, C₂-C₄-Halogenalkinyl, O-C₁-C₄-Alkyl, -C(=O)-C₁-C₄-Alkyl, -(C₁-C₂-Alkyl)-O-(C₁-C₂-alkyl), -(C₁-C₂-Alkyl)-O-(C₁-C₂-halogenalkyl), C₃-C₆-Cycloalkyl, C₃-C₆-Halogencycloalkyl und -C₁-C₄-Alkyl-C₃-C₆-cycloalkyl;
Het 5- oder 6-gliedriges Heteroaryl ist, wobei das Heteroaryl neben Kohlenstoffatomen 1, 2 oder 3 Heteroatome ausgewählt aus N, O und S enthält, mit der Maßgabe, dass ein solches Heteroaryl nicht 2 benachbarte Atome ausgewählt aus O und S enthalten kann;
wobei das Heteroaryl unsubstituiert ist oder 1, 2, 3 oder bis zur maximalen Anzahl gleiche oder verschiedene R^{a}-Gruppen trägt:
R^{a} ausgewählt ist aus Halogen, CN, -NR⁵R⁶, C₁-C₄-Alkyl, C₂-C₄-Alkenyl, C₂-C₄-Alkinyl,
-O-C₁-C₄-Alkyl, -C(=N-O-C₁-C₄-Alkyl)-C₁-C₄-alkyl, -C(=O)-C₁-C₄-Alkyl, -O-CH₂-C(=N-O-C₁-C₄-Alkyl)-C₁-C₄-alkyl, C₃-C₆-Cycloalkyl, C₃-C₆-Cycloalkenyl, -C₁-C₂-Alkyl-C₃-C₆-cycloalkyl, -O-C₃-C₆-Cycloalkyl, Phenyl, 3- bis 6-gliedrigem Heterocycloalkyl, 3- bis 6-gliedrigem Heterocycloalkenyl und 5- oder 6-gliedrigem Heteroaryl;
wobei das Heterocycloalkyl, Heterocycloalkenyl und Heteroaryl neben Kohlenstoffatomen 1, 2 oder 3 Heteroatome ausgewählt aus N, O und S enthalten, mit der Maßgabe, dass ein solches Heterocycloalkyl, Heterocycloalkenyl oder Heteroaryl nicht 2 benachbarte Atome ausgewählt aus O und S enthalten kann;
und/oder
2 an benachbarte Kohlenstoffringatome gebundene R^{a}-Substituenten zusammen mit den beiden dazwischenliegenden Kohlenstoffringatomen einen teilweise ungesättigten oder aromatischen 5- bis 6-gliedrigen kondensierten Carbo- oder Heterocyclus bilden;
wobei der Heterocyclus neben Kohlenstoffatomen 1 oder 2 Heteroatome unabhängig ausgewählt aus N, O und S als Ringgliedatome einschließt, mit der Maßgabe, dass ein solcher Heterocyclus nicht 2 benachbarte Atome ausgewählt aus O und S enthalten kann; und wobei die aliphatischen und cyclischen Anteile von R^{a} und der genannte kondensierte Carbo- oder Heterocyclus unsubstituiert sind oder 1, 2, 3, 4 oder bis zu der maximalen Anzahl identischer oder verschiedener Gruppen R^{b} tragen:
R^{b} ausgewählt ist aus Halogen, CN, NH₂, NO₂, C₁-C₄-Alkyl, C₁-C₄-Halogenalkyl, -O-C₁-C₄-Alkyl und -O-C₁-C₄-Halogenalkyl;
R⁵, R⁶ unabhängig voneinander ausgewählt sind aus der Gruppe bestehend aus H, C₁-C₆-Alkyl, C₁-C₆-Halogenalkyl und C₂-C₄-Alkinyl;
und in Form von Stereoisomeren und Tautomeren davon, und die N-Oxide und die landwirtschaftlich unbedenklichen Salze davon.

9. Verbindungen nach Anspruch 8, wobei R¹ ausgewählt ist aus O und NH; und R² ausgewählt ist aus CH und N, mit der Maßgabe, dass R² N ist, wenn R¹ NH ist.

10. Verbindungen nach einem der Ansprüche 8 bis 9, wobei R³ ausgewählt ist aus CN, C₁-C₂-Alkyl, C₁-C₂-Halogenalkyl, C₃-C₄-Cycloalkyl, -O-C₁-C₂-Alkyl und -O-C₁-C₂-Halogenalkyl.

11. Verbindungen nach einem der Ansprüche 8 bis 10, wobei R⁴ ausgewählt ist aus C₁-C₆-Alkyl, C₂-C₄-Alkenyl, C₁-C₆-Halogenalkyl, C₂-C₄-Halogenalkenyl, -(C₁-C₂-Alkyl)-O-(C₁-C₂-alkyl) und -(C₁-C₂-Alkyl)-O-(C₁-C₂-halogenalkyl).

12. Verbindungen nach einem der Ansprüche 8 bis 11, wobei Het Pyridyl oder Thiazolyl ist, wobei das Pyridyl oder Thiazolyl unsubstituiert ist oder 1, 2 oder 3 gleiche oder verschiedene Gruppen R^{a} trägt, wie in Anspruch 8 definiert.

13. Agrochemische Zusammensetzungen, umfassend einen Hilfsstoff und mindestens eine Verbindung der Formel I, wie in einem der Ansprüche 8 bis 12 definiert, oder in Form eines Stereoisomers davon oder ein landwirtschaftlich unbedenkliches Salz oder N-Oxid davon.

14. Nichttherapeutische Verwendung von mindestens einer Verbindung der Formel I, wie in einem der Ansprüche 8 bis 12 definiert, oder einer agrochemischen Zusammensetzung, wie in Anspruch 13 definiert, zur Bekämpfung phytopathogener Pilze.

15. Nichttherapeutisches Verfahren zur Bekämpfung phytopathogener Pilze, umfassend:
kuratives und/oder präventives Behandeln der Pflanzen oder des Pflanzenfortpflanzungsmaterials der Pflanzen, bei denen ein Risiko einer Erkrankung durch die phytopathogenen Pilze besteht, mit mindestens einer Verbindung der Formel I, wie in einem der Ansprüche 8 bis 12 definiert, oder einer agrochemischen Zusammensetzung, wie in Anspruch 13 definiert, und/oder Aufbringen derselben auf die phytopathogenen Pilze.

## Revendications

1. Utilisation non thérapeutique de composés de formule I 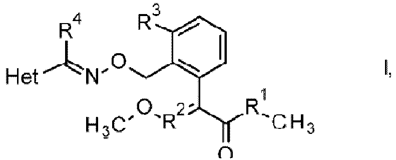
R¹ étant choisi parmi O et NH ;
R² étant choisi parmi CH et N ;
R³ étant choisi parmi halogène, CN, C₁-C₄-alkyle, C₂-C₄-alcényle, C₂-C₄-alcynyle, C₁-C₄-halogénoalkyle, C₂-C₄-halogénoalcényle, C₂-C₄-halogénoalcynyle, C₃-C₆-cycloalkyle, -O-C₁-C₄-alkyle, -O-C₁-C₄-halogénoalkyle, - O-C₃-C₆-cycloalkyle, -C₁-C₂-alkyl-C₃-C₆-cycloalkyle, phényle, hétérocycloalkyle à 3 à 6 chaînons et hétéroaryle à 5 ou 6 chaînons,
lesdits hétérocycloalkyle et hétéroaryle contenant outre des atomes de carbone 1, 2 ou 3 hétéroatomes choisis parmi N, O et S, à condition qu'un tel hétérocycloalkyle et hétéroaryle ne puisse pas contenir 2 atomes contigus choisis parmi O et S ;
lesdits phényle, hétérocycloalkyle et hétéroaryle étant liés directement ou via un atome d'oxygène ou via un lieur C₁-C₂-alkylène, et lesdits phényle et hétéroaryle étant non substitués ou substitués par 1, 2 ou 3 substituants identiques ou différents choisis parmi halogène, CN, NH₂, NO₂, C₁-C₄-alkyle, C₁-C₄-halogénoalkyle, -O-C₁-C₄-alkyle et -O-C₁-C₄-halogénoalkyle ;
R⁴ étant choisi parmi C₁-C₆-alkyle, C₂-C₄-alcényle, C₂-C₄-alcynyle, C₁-C₆-halogénoalkyle, C₂-C₄-halogénoalcényle, C₂-C₄-halogénoalcynyle, O-C₁-C₄-alkyle, -C(=O)-C₁-C₄-alkyle, -(C₁-C₂-alkyl)-O-(C₁-C₂-alkyle), - (C₁-C₂-alkyl)-O-(C₁-C₂-halogénoalkyle), C₃-C₆-cycloalkyle, C₃-C₆-halogénocycloalkyle et -C₁-C₄-alkyl-C₃-C₆-cycloalkyle ;
Het étant hétéroaryle à 5 ou 6 chaînons, ledit hétéroaryle contenant outre des atomes de carbones 1, 2 ou 3 hétéroatomes choisis parmi N, O et S, à condition qu'un tel hétéroaryle ne puisse pas contenir 2 atomes contigus choisis parmi O et S ;
ledit hétéroaryle étant non substitué ou portant 1, 2, 3 ou jusqu'au nombre maximal de groupes identiques ou différents R^{a} :
R^{a} étant choisi parmi halogène, CN, -NR⁵R⁶, C₁-C₄-alkyle, C₂-C₄-alcényle, C₂-C₄-alcynyle, -O-C₁-C₄-alkyle, - C(=N-O-C₁-C₄-alkyl)-C₁-C₄-alkyle, -C(=O)-C₁-C₄-alkyle, -O-CH₂-C(=N-O-C₁-C₄-alkyl)-C₁-C₄-alkyle, C₃-C₆-cycloalkyle, C₃-C₆-cycloalcényle, -C₁-C₂-alkyl-C₃-C₆-cycloalkyle, -OC₃-C₆-cycloalkyle, phényle, hétérocycloalkyle à 3 à 6 chaînons, hétérocycloalcényle à 3 à 6 chaînons et hétéroaryle à 5 ou 6 chaînons,
lesdits hétérocycloalkyle, hétérocycloalcényle et hétéroaryle contenant outre des atomes de carbone 1, 2 ou 3 hétéroatomes choisis parmi N, O et S, à condition que de tels hétérocycloalkyle, hétérocycloalcényle et hétéroaryle ne puissent pas contenir 2 atomes contigus choisis parmi O et S ;
et/ou
2 substituants R^{a} liés à des atomes de cycle de carbone adjacents, conjointement avec les deux atomes de cycle de carbone interjacents, formant un carbocycle ou hétérocycle condensé à 5 ou 6 chaînons partiellement insaturé ou aromatique,
l'hétérocycle comprenant outre des atomes de carbone 1 ou 2 hétéroatomes indépendamment choisi(s) parmi N, O et S en tant qu'atomes d'éléments de cycle, à condition qu'un tel hétérocycle ne puisse pas contenir 2 atomes contigus choisis parmi O et S ;
et les groupements aliphatiques et cycliques de R^{a} et le carbocycle ou hétérocycle condensé susmentionné étant non substitué ou portant 1, 2, 3, 4 ou jusqu'au nombre maximal de groupes identiques ou différents R^{b} :
R^{b} étant choisi parmi halogène, CN, NH₂, NO₂, C₁-C₄-alkyle, C₁-C₄-halogénoalkyle, -O-C₁-C₄-alkyle, -O-C₁-C₄-halogénoalkyle et C₃-C₆-cycloalkyle ;
R⁵, R⁶ étant indépendamment l'un de l'autre choisis dans le groupe constitué par H, C₁-C₆-alkyle, C₁-C₆-halogénoalkyle et C₂-C₄-alcynyle ;
et sous forme de stéréoisomères et formes tautomères correspondant (e)s, et les sels acceptables sur le plan agricole et les N-oxydes correspondants, pour la lutte contre des champignons phytopathogènes contenant une substitution d'acide aminé F129L dans la protéine de cytochrome mitochondrial b conférant une résistance aux inhibiteurs de Qo.

2. Utilisation selon la revendication 1, dans la formule I R¹ étant choisi parmi O et NH ; et R² étant choisi parmi CH et N, à condition que R² soit N dans le cas où R¹ est NH.

3. Utilisation selon la revendication 1 ou la revendication 2, dans la formule I R³ étant choisi parmi C₁-C₂-alkyle, C₁-C₂-halogénoalkyle, C₃-C₄-cycloalkyle, -OC₁-C₂-alkyle et -O-C₁-C₂-halogénoalkyle.

4. Utilisation selon l'une quelconque des revendications 1 à 3, dans la formule I R⁴ étant choisi parmi C₁-C₄-alkyle, C₂-C₄-alcényle, -C(=O)-C₁-C₂-alkyle, C₁-C₄-halogénoalkyle, C₂-C₄-halogénoalcényle et -(C₁-C₂-alkyl)-O-(C₁-C₂-alkyle).

5. Utilisation selon l'une quelconque des revendications 1 à 4, dans la formule I Het étant pyridinyle ou thiazolyle, ledit pyridinyle ou thiazolyle étant non substitué ou portant 1, 2 ou 3 groupes R^{a} identiques ou différents tels que définis dans la revendication 1.

6. Utilisation selon l'une quelconque des revendications 1 à 5, dans la formule I R^{a} étant choisi parmi C₁-C₃-alkyle, C₂-C₃-alcényle, C₂-C₃-alcynyle, -O-C₁-C₃-alkyle, -C(=N-O-C₁-C₂-alkyl)-C₁-C₂-alkyle, -O-CH₂-C(=N-O-C₁-C₂-alkyl)-C₁-C₂-alkyle, C₃-C₄-cycloalkyle, -C₁-C₂-alkyl-C₃-C₄-cycloalkyle, -O-C₃-C₄-cycloalkyle, phényle, hétérocycloalkyle à 3 à 5 chaînons et hétéroaryle à 5 ou 6 chaînons, lesdits hétérocycloalkyle et hétéroaryle contenant outre des atomes de carbone 1 ou 2 hétéroatomes choisis parmi N, O et S, à condition que de tels hétérocycloalkyle et hétéroaryle ne puissent pas contenir 2 atomes contigus choisis parmi O et S ; et/ou 2 substituants R^{a} liés à des atomes de cycle de carbone adjacents, conjointement avec les deux atomes de cycle de carbone interjacents, formant un cycle phényle condensé, et les groupements aliphatiques et cycliques de R^{a} et le cycle phényle condensé susmentionné étant non substitués ou portant 1, 2, 3, 4 ou jusqu'au nombre maximal de groupes identiques ou différents R^{b} qui indépendamment les uns des autres sont choisis parmi halogène, CN, méthyle et C₁-halogénoalkyle.

7. Procédé non thérapeutique pour la lutte contre des champignons phytopathogènes contenant une substitution d'acide aminé F129L dans la protéine de cytochrome mitochondrial b conférant une résistance aux inhibiteurs de Qo, comprenant :
le traitement de manière curative et/ou préventive des végétaux ou du matériel de propagation végétale desdits végétaux qui sont à risque d'être malades par lesdits champignons phytopathogènes, et/ou l'application sur lesdits champignons phytopathogènes, avec une quantité efficace d'au moins un composé de formule I tel que défini dans l'une quelconque des revendications 1 à 6 ou d'une composition le comprenant.

8. Composés de formule I 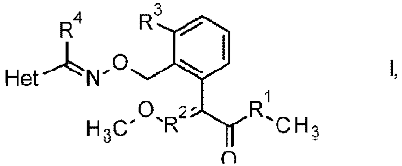
R¹ étant choisi parmi O et NH ;
R² étant choisi parmi CH et N ;
R³ étant choisi parmi halogène, CN, C₁-C₄-alkyle, C₂-C₄-alcényle, C₂-C₄-alcynyle, C₁-C₄-halogénoalkyle, C₂-C₄-halogénoalcényle, C₂-C₄-halogénoalcynyle, C₃-C₆-cycloalkyle, -O-C₁-C₄-alkyle, -O-C₁-C₄-halogénoalkyle, - O-C₃-C₆-cycloalkyle, -C₁-C₂-alkyl-C₃-C₆-cycloalkyle, phényle, hétérocycloalkyle à 3 à 6 chaînons et hétéroaryle à 5 ou 6 chaînons,
lesdits hétérocycloalkyle et hétéroaryle contenant outre des atomes de carbone 1, 2 ou 3 hétéroatomes choisis parmi N, O et S, à condition qu'un tel hétérocycloalkyle et hétéroaryle ne puisse pas contenir 2 atomes contigus choisis parmi O et S ;
lesdits phényle, hétérocycloalkyle et hétéroaryle étant liés directement ou via un atome d'oxygène ou via un lieur C₁-C₂-alkylène, et lesdits phényle et hétéroaryle étant non substitués ou substitués par 1, 2 ou 3 substituants identiques ou différents choisis parmi halogène, CN, NH₂, NO₂, C₁-C₄-alkyle, C₁-C₄-halogénoalkyle, -O-C₁-C₄-alkyle et -O-C₁-C₄-halogénoalkyle ;
R⁴ étant choisi parmi C₁-C₆-alkyle, C₂-C₄-alcényle, C₂-C₄-alcynyle, C₁-C₆-halogénoalkyle, C₂-C₄-halogénoalcényle, C₂-C₄-halogénoalcynyle, O-C₁-C₄-alkyle, -C(=O)-C₁-C₄-alkyle, -(C₁-C₂-alkyl)-O-(C₁-C₂-alkyle), - (C₁-C₂-alkyl)-O- (C₁-C₂-halogénoalkyle), C₃-C₆-cycloalkyle, C₃-C₆-halogénocycloalkyle et -C₁-C₄-alkyl-C₃-C₆-cycloalkyle ;
Het étant hétéroaryle à 5 ou 6 chaînons, ledit hétéroaryle contenant outre des atomes de carbone 1, 2 ou 3 hétéroatomes choisis parmi N, O et S, à condition qu'un tel hétéroaryle ne puisse pas contenir 2 atomes contigus choisis parmi O et S ;
ledit hétéroaryle étant non substitué ou portant 1, 2, 3 ou jusqu'au nombre maximal de groupes identiques ou différents R^{a} :
R^{a} étant choisi parmi halogène, CN, -NR⁵R⁶, C₁-C₄-alkyle, C₂-C₄-alcényle, C₂-C₄-alcynyle,
-O-C₁-C₄-alkyle, -C(=N-O-C₁-C₄-alkyl)-C₁-C₄-alkyle, - C(=O)-C₁-C₄-alkyle, -O-CH₂-C(=N-O-C₁-C₄-alkyl)-C₁-C₄-alkyle, C₃-C₆-cycloalkyle, C₃-C₆-cycloalcényle, -C₁-C₂-alkyl-C₃-C₆-cycloalkyle, -O-C₃-C₆-cycloalkyle, phényle, hétérocycloalkyle à 3 à 6 chaînons, hétérocycloalcényle à 3 à 6 chaînons et hétéroaryle à 5 ou 6 chaînons,
lesdits hétérocycloalkyle, hétérocycloalcényle et hétéroaryle contenant outre des atomes de carbone 1, 2 ou 3 hétéroatomes choisis parmi N, O et S, à condition que de tels hétérocycloalkyle, hétérocycloalcényle et hétéroaryle ne puissent pas contenir 2 atomes contigus choisis parmi O et S ;
et/ou
2 substituants R^{a} liés à des atomes de cycle de carbone adjacents, conjointement avec les deux atomes de cycle de carbone interjacents, formant un carbocycle ou hétérocycle condensé à 5 ou 6 chaînons partiellement insaturé ou aromatique,
l'hétérocycle comprenant outre des atomes de carbone 1 ou 2 hétéroatomes indépendamment choisis parmi N, O et S en tant qu'atomes d'éléments de cycle, à condition qu'untel hétérocycle ne puisse pas contenir 2 atomes contigus choisis parmi O et S ; et les groupements aliphatiques et cycliques de R^{a} et le carbocycle ou hétérocyclyle condensé susmentionné étant non substitués ou portant 1, 2, 3, 4 ou jusqu'au nombre maximal de groupes identiques ou différents R^{b} :
R^{b} étant choisi parmi halogène, CN, NH₂, NO₂, C₁-C₄-alkyle, C₁-C₄-halogénoalkyle, -O-C₁-C₄-alkyle et -O-C₁-C₄-halogénoalkyle ;
R⁵, R⁶ étant indépendamment l'un de l'autre choisis dans le groupe constitué par H, C₁-C₆-alkyle, C₁-C₆-halogénoalkyle et C₂-C₄-alcynyle ;
et sous forme de stéréoisomères et formes tautomères correspondant (e) s, et les sels acceptables sur le plan agricole et les N-oxydes correspondants.

9. Composés selon la revendication 8, R¹ étant choisi parmi O et NH ; et R² étant choisi parmi CH et N, à condition que R² soit N dans le cas où R¹ est NH.

10. Composés selon l'une quelconque des revendications 8 à 9, R³ étant choisi parmi CN, C₁-C₂-alkyle, C₁-C₂-halogénoalkyle, C₃-C₄-cycloalkyle, -O-C₁-C₂-alkyle et -O-C₁-C₂-halogénoalkyle.

11. Composés selon l'une quelconque des revendications 8 à 10, R⁴ étant choisi parmi C₁-C₆-alkyle, C₂-C₄-alcényle, C₁-C₆-halogénoalkyle, C₂-C₄-halogénoalcényle, -(C₁-C₂-alkyl)-O-(C₁-C₂-alkyl)et -(C₁-C₂-alkyl)-O-(C₁-C₂-halogénoalkyle).

12. Composés selon l'une quelconque des revendications 8 à 11, Het étant pyridinyle ou thiazolyle, ledit pyridinyle ou thiazolyle étant non substitué ou portant 1, 2 ou 3 groupes R^{a} identiques ou différents tels que définis dans la revendication 8.

13. Compositions agrochimiques comprenant un auxiliaire et au moins un composé de formule I tel que défini dans l'une quelconque des revendications 8 à 12 ou sous la forme d'un stéréoisomère ou d'un sel acceptable sur le plan agricole ou d'une forme tautomère ou d'un N-oxyde correspondant(e).

14. Utilisation non thérapeutique d'au moins un composé de formule I tel que défini dans l'une quelconque des revendications 8 à 12 ou d'une composition agrochimique telle que définie dans la revendication 13 pour la lutte contre des champignons phytopathogènes.

15. Procédé non thérapeutique de lutte contre des champignons phytopathogènes comprenant :
le traitement de manière curative et/ou préventive des végétaux ou du matériel de propagation végétale desdits végétaux qui sont à risque d'être malades par lesdits champignons phytopathogènes, et/ou l'application sur lesdits champignons phytopathogènes, d'au moins un composé de formule I tel que défini dans l'une quelconque des revendications 8 à 12 ou d'une composition agrochimique telle que définie dans la revendication 13.
